# EUROPEAN PATENT SPECIFICATION

(11) **EP 2 968 995 B1**
(45) Date of publication and mention of the grant of the patent: **09.05.2018**
(21) Application number: 14771041.2
(22) Date of filing: 12.03.2014
(51) Int. Cl.: C07D 409/00, C07D 417/12, A61P 25/16

(54) **INHIBITORS OF LRRK2 KINASE ACTIVITY**
INHIBITOREN DER LRRK2-KINASEAKTIVITÄT
INHIBITEURS DE L'ACTIVITÉ KINASE LRRK2

(30) Priority: 15.03.2013 US 201361793053 P
(43) Date of publication of application: 20.01.2016
(73) Proprietor: Elan Pharmaceuticals LLC, Allegan, Michigan 49010 (US)
(72) Inventor: GAROFALO, Albert, South San Francisco, CA 94080 (US); AUBELE, Danielle, San Mateo, CA 94401 (US)
(74) Representative: Kiddle, Simon John
(86) International application number: PCT/US2014/024700
(87) International publication number: WO 2014/150981

(56) References cited:
- WO-A1-2010/106333
- US-A1- 2007 232 586
- US-A1- 2010 151 064
- CEYLAN, S. ET AL.: "Syntheses and biological activities of new hybrid molecules containing different heterocyclic moieties", ARCHIV DER PHARMAZIE., vol. 346, no. 10, 6 September 2013 (2013-09-06), pages 743-756, XP002761310, VCH VERLAGSGESELLSCHAFT MBH, WEINHEIM; DE ISSN: 0365-6233, DOI: 10.1002/ardp.201300161
- DATABASE CAPLUS [Online] CHEMICAL ABSTRACTS SERVICE, COLUMBUS, OHIO, US; 21 May 2013 (2013-05-21), XP002761311, Database accession no. 2013:785506 & BASOGLU, S. ET AL.: "Design, synthesis and antimicrobial activities of some azole derivatives", ACTA POLONIAE PHARMACEUTICA - DRUG RESEARCH, vol. 70, no. 2, 2013, pages 229-236, POLISH PHARMACEUTICAL SOCIETY, WARZSAW; PL ISSN: 0001-6837
- DATABASE CAPLUS [Online] CHEMICAL ABSTRACTS SERVICE, COLUMBUS, OHIO, US; 10 September 2012 (2012-09-10), XP002761312, Database accession no. 2012:1312242 & TATAR, E. ET AL.: "Synthesis, anti-tuberculosis and antiviral activity of novel 2-isonicotinoylhydrazono-5-arylidene-4-thi azolidinones", INTERNATIONAL JOURNAL OF DRUG DESIGN AND DISCOVERY, vol. 1, no. 1, 2010, Pharma Syndicate, Hyderabad; IN ISSN: 0975-8275
- DATABASE CAPLUS [Online] CHEMICAL ABSTRACTS SERVICE, COLUMBUS, OHIO, US; 5 July 2012 (2012-07-05), XP002761313, Database accession no. 2012:957276 & MOKHTAR, H. M. ET AL.: "Synthesis of novel 2,4-disubstituted triazoles with potential biological activity", ALEXANDRIA JOURNAL OF PHARMACEUTICAL SCIENCES., vol. 26, no. 1, 2012, pages 19-26, ALEXANDRIA; EG ISSN: 1110-1792
- LIESEN A. P. ET AL: "Synthesis and evaluation of anti-Toxoplasma gondii and antimicrobial activities of thiosemicarbazides, 4-thiazolidinones and 1,3,4-thiadiazoles", EUROPEAN JOURNAL OF MEDICINAL CHEMISTRY, EDITIONS SCIENTIFIQUE ELSEVIER, PARIS; FR, vol. 45, no. 9, 1 September 2010 (2010-09-01), pages 3685-3691, XP027458791, ISSN: 0223-5234, DOI: 10.1016/J.EJMECH.2010.05.017 [retrieved on 2010-09-01]
- DATABASE PUBCHEM COMPOUND [Online] 05 December 2007 'SCHEMBL8734256', XP055286942 Retrieved from NCBI Database accession no. 19933978

## Description

### BACKGROUND OF THE INVENTION

Parkinson's disease (PD) is the most common form of parkinsonism, a neurodegenerative movement disorder characterized by resting tremors, rigidity, postural instability, impaired speech and bradykinesia (slowed movement). In addition to PD, parkinsonism is exhibited in a range of conditions such as progressive supranuclear palsy, corticobasal degeneration, multiple system atrophy, and dementia with Lewy bodies. PD was first formally described in 1817 by James Parkinson in his monograph titled, "An Essay on the Shaking Palsy" (Parkinson, J.; J. Neuropsychiatry Clin. Neurosci. 2002, 14(2), 223-236 (reprinted)). He noted that the symptoms of this particular disorder include involuntary tremors, decreased muscular strength, bent posture, and difficulty walking. The pathological correlate is the loss of dopaminergic neurons in the substantia nigra of the basal ganglia and the presence of Lewy bodies (LB) in postmortem brain tissues. Aggregated α-synuclein forms the intracellular LB deposits, which are the pathological hallmark of PD and other Lewy body diseases. Most cases of PD appear not to have a genetic component. For that reason the most common form of PD is known as sporadic Parkinson's disease or idiopathic Parkinson's disease (IPD). Other forms of PD include autosomal recessive juvenile parkinsonism (AR-JP) and several rare familial forms. PD affects approximately 1-2% of the population over age 50 (∼1.5 million in the US, over 5 million worldwide) and early onset cases can occur as early as 30 years of age.

Current therapeutic strategies for PD focus primarily on reducing the severity of symptoms by using supplement dopaminergic medications. These drugs may lose efficacy after prolonged treatment and display severe side effects. Levodopa, a precursor of dopamine, is the most commonly prescribed drug for treatment. At present, there is no disease-modifying therapy that addresses the underlying neuropathological cause of the disease, thus constituting a significant unmet medical need. Cumulative evidence over the past decade demonstrate that autosomal mutations of several genes might be responsible for a sizable disease subpopulation. A prime example is the recent discovery that dominant point mutations in Leucine-rich repeat kinase 2 (LRRK2) cause late-onset PD with clinical and pathological features indistinguishable from idiopathic PD. The extensive genetic analyses undertaken so far indicate that LRRK2 mutations are relatively frequent, accounting for 5-10% of PD cases with a positive family history (familial PD) and 1-2% of sporadic PD cases.

Leucine-rich repeat kinase 2 (also known as dardarin) is a product of the PARK8 gene. It is a member of the tyrosine kinase-like branch of the kinome. LRRK2 encodes a large multi-domain protein that consists of N-terminal leucine-rich repeats (LRR), a ROC (Ras-GTPase in complex proteins) domain, a COR (C-terminal of ROC) domain, a protein kinase domain most homologous to the RIP (Receptor Interacting Protein) kinases, and a C-terminal WD40 domain. It is expressed throughout the brain including regions associated with motor neuron dysfunction. It is also found in various other tissues, most notably in the kidneys, where expression is highest. Levels in kidney are reported to be ∼6-fold higher than in brain. Mutations in the PARK8 gene have been associated with familial PD. All the pathogenic mutations can lead to a wide spectrum of cellular toxicity in a kinase-dependent manner. The most prevalent amino acid substitution found in mutant LRRK2 is G2019S, which is located within the highly conserved DF/YG motif in the activation loop and causes significant increase in kinase activity.

Patients with LRRK2 mutations exhibit Lewy body pathology and LRRK2 is considered one of the most relevant targets for treating PD and other LB diseases. The G2019S mutation is believed to be responsible for 3-40% of familial and sporadic PD cases, dependent on study population, with Lewy body pathology most often associated with G2019S. Because the LRRK2 G2019S mutation has increased kinase activity, inhibition of this activity is a therapeutic target for the treatment of PD. Importantly, most of the LRRK2-mediated toxicity and pathology can be prevented by treatment with specific kinase inhibitors, suggesting that kinase inhibitors could be useful therapeutic agents for PD patients with LRRK2 mutations and potentially for sporadic PD as well. Additional LRRK2 mutations, such as I2020T, also in the kinase domain, R1441C and R1441G in the ROC domain, and Y1699C in the COR domain are also associated with PD, and mutations G2385R and R1628P are considered risk factors for sporadic PD in Asian populations. Animal models expressing mutant LRRK2 recapitulate certain cardinal features of human PD.

Although the function of LRRK2 is not well known at this time, the recent demonstration that LRRK2 modulates synuclein-mediated toxicity and neurodegeneration *in vitro* and *in vivo* further highlights an important role of LRRK2 in PD pathogenesis. For example, transgenic studies in mice have shown that LRRK2 may regulate α-synuclein toxicity by modulating the accumulation of α-synuclein. It has also been suggested that α-synuclein neurodegeneration is related to LRRK2 regulation of cytoskeletal dynamics.

Thus with involvement of LRRK2 in relation to α-synuclein toxicity, and the prevalence of LRRK2 mutant G2019S in familial PD, LRRK2 inhibitors would be useful in treating PD, as well as other LBDs, such as Diffuse Lewy body disease, Lewy body variant of Alzheimer's disease, combined Parkinson's disease and Alzheimer's disease, multiple system atrophy, and Dementia with Lewy bodies.

Targeting of LRRK2 kinase may also provide therapeutic benefits in certain cancers, such as melanoma, acute myelogenous leukemia, breast carcinoma, lung adenocarincoma, prostate adenocarcinoma, renal cell carcinoma (e.g. papillary renal cell carcinoma), and papillary thyroid carcinoma, in certain autoimmune diseases, such as Inflammatory Bowel Disease (e.g. Crohn's disease and ulcerative colitis), and in leprosy.

As there are presently limited therapeutic options for treating PD and other disorders associated with aberrant LRRK2 kinase activity, there remains a need for developing LRRK2 inhibitors.

Ceylan et al., Arch. Pharm. Chem. Life Sci. 2013, 346, 743-756 describes synthesis and biological activity of hybrid molecules containing heterocyclic moieties some of which demonstrate antimicrobial, antiurease, and/or antilipase activity.

Further heterocyclic hydrazide compounds are described in Basoglu et al., Acta Poloniae Pharmaceutica, 70(2), 229-236 (2013); Tatar et al., International Journal of Drug Design and Discovery (2010), 1(1), 19-32; and Mokhtar & Farahat, Alexandria Journal of Pharmaceutical Sciences (2012), 26(1), 19-26.

Liesen et al., European Journal of Medicinal Chemistry 45 (2010), 3685-3691 describes synthesis and evaluation of anti-*Toxoplasma gondii* and antimicrobial activity of three new thiosemicarbazides, 4-thiazolidinones, and 1,3,4-thiadiazoles.

US 2010/0151064 describes compounds having a thiazolhydrazide scaffold and pharmaceutically acceptable salts and compositions of these which can be used for prophylaxis and/or treatment of neurodegenerative diseases and conditions.

### SUMMARY OF THE INVENTION

Compounds are provided that are inhibitors of kinase activity of Leucine-rich repeat kinase 2 (LRRK2) and mutations thereof. In one instance, compounds are provided that are inhibitors of LRRK2 mutant kinase activity, including LRRK2 mutant G2019S, a mutation present in familial PD and having increased kinase activity. Inhibitors of LRRK2 kinase activity, including any mutations thereof, in one instance LRRK2 mutant G2019S, are thus useful for the treatment of neurodegenerative diseases, such as Parkinson's disease, and other Lewy body-type diseases, including Diffuse Lewy body disease, Lewy body variant of Alzheimer's disease, combined Parkinson's disease and Alzheimer's disease, multiple system atrophy, and Dementia with Lewy bodies. Such LRRK2 kinase activity inhibitors are also useful in the treatment of cancers, such as melanoma, acute myelogenous leukemia, breast carcinoma, lung adenocarincoma, prostate adenocarcinoma, renal cell carcinoma, and papillary thyroid carcinoma and autoimmune diseases, such as Inflammatory Bowel Disease (e.g. Crohn's disease and ulcerative colitis). Also provided are pharmaceutical compositions comprising inhibitors of LRRK2 kinase activity, including any mutations thereof, and methods of utilizing those compositions in the treatment and prevention of various neurodegenerative disorders associated with LRRK2, such as Parkinson's Disease and other Lewy body-type diseases and in the treatment of various cancers and autoimmune diseases.

In one instance, compounds described herein that are inhibitors of LRRK2 kinase activity, including LRRK2 mutant kinase activity, including LRRK2 mutant G2019S kinase activity, are selective relative to other kinases.

In one aspect, compounds are provided having a structure according to Formula I:
or a salt thereof, wherein: 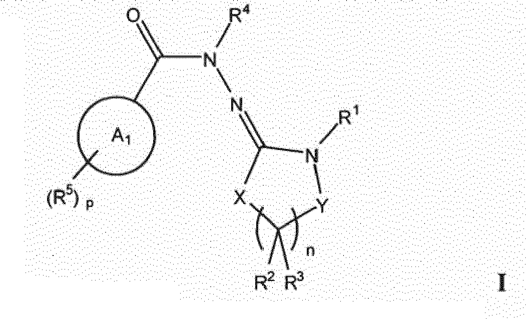 A₁ is selected from the group consisting of pyridine, pyrimidine, pyridazine, pyrazine, triazine, pyrrole, pyrazole, imidazole, triazole, tetrazole, oxazole, isoxazole, oxadiazole, thiazole, isothiazole, thiadiazole, furan, and thiophene;
X is -O-, -S-, -CR⁶R⁷-, or -NR⁸-;
Y is -C(O)- or -CH₂-;
R¹ is alkyl, cycloalkyl, heterocycloalkyl, aryl optionally substituted with one or more R⁹, or heteroaryl optionally substituted with one or more R¹⁰, wherein said alkyl is optionally substituted with one or more R¹¹; and wherein said cycloalkyl and said heterocycloalkyl are optionally substituted with one or more R¹²;
R² and R³ at each occurrence are independently selected from the group consisting of hydrogen, halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₃₋₆ cycloalkyl, and 3-6 membered heterocycloalkyl; or
R² and R³ at each occurrence are independently selected from the group consisting of hydrogen, halogen
or R² and R³ combined form =O or =NR¹⁵;
or one set of R² and R³ attached to the same carbon can combine with the carbon to which they are attached to form a carbocyclic ring or heterocyclic ring, wherein each ring is optionally substituted with one or more substituents selected from the group consisting of hydrogen, halogen, alkyl, -CN, -NO₂, =O, -OR¹³, -SR¹³, -NR¹⁴R¹⁵, =NR¹⁵, -C(O)R¹⁶, -S(O)ₘR¹⁶, cycloalkyl, and heterocycloalkyl, wherein cycloalkyl, and heterocycloalkyl are optionally substituted with one or more R¹² and wherein said alkyl is optionally substituted with one or more substituents selected from the group consisting of halogen, -CN, -NO₂, =O, -OR¹³, -SR¹³, -NR¹⁴R¹⁵, =NR¹⁵, -C(O)R¹⁶, -S(O)ₘR¹⁶, cycloalkyl, and heterocycloalkyl;
R⁴ is hydrogen or alkyl;
R⁵ at each occurrence is independently selected from the group consisting of halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl;
R⁶ and R⁷ are independently selected from the group consisting of hydrogen, halogen, alkyl, -CN, -NO₂, -OR¹³, -SR¹³, -NR¹⁴R¹⁵, -C(O)R¹⁶, -S(O)ₘR¹⁶, cycloalkyl, and heterocycloalkyl, wherein said alkyl is optionally substituted with one or more substituents selected from the group consisting of halogen, -CN, -NO₂, =O, -OR¹³, -SR¹³, -NR¹⁴R¹⁵ , =NR¹⁵, -C(O)R¹⁶, -S(O)ₘR¹⁶, cycloalkyl and heterocycloalkyl, and wherein said cycloalkyl and heterocycloalkyl are optionally substituted with one or more R¹¹;
R⁸ is selected from the group consisting of hydrogen and alkyl, wherein said alkyl is optionally substituted with one or more substituents selected from the group consisting of halogen, -CN, -NO₂, =O, -OR¹³, -SR¹³, -NR¹⁴R¹⁵, =NR¹⁵, -C(O)R¹⁶, -S(O)ₘR¹⁶, and N-linked heterocycloalkyl;
R⁹ and R¹⁰ at each occurrence are independently selected from the group consisting of halogen,
   alkyl, -CN, -NO₂, -OR¹⁷, -SR¹⁷, -NR¹⁸R¹⁹, -C(O)R²⁰, -S(O)ₘ,R²⁰, -C(O)OR¹⁷, -OC( O)R²⁰, -C(O)NR¹⁸R¹⁹, -NR¹⁸C(O)R²⁰, -S(O)₂NR¹⁸R¹⁹, -NR¹⁸S(O)₂R²⁰, cycloalkyl and heterocycloalkyl, wherein said alkyl is optionally substituted with one or more halogen, cycloalkyl, -CN, -NO₂, =O, -OR¹⁷, -SR¹⁷, -NR¹⁸R¹⁹,
   =NR¹⁹, -C(O)R²⁰, -S(O)ₘR²⁰, -C(O)OR¹⁷, -OC(O)R²⁰, -C(O)NR¹⁸R¹⁹, -NR¹⁸C(O) R²⁰, -S(O)₂NR¹⁸R¹⁹, -NR¹⁸S(O)₂R²⁰, cycloalkyl and heterocycloalkyl, and wherein said cycloalkyl and heterocycloalkyl are optionally substituted with one or more R¹²;
R¹¹ at each occurrence is independently selected from the group consisting of halogen, -CN, -NO₂, =O, -OR¹⁷, -SR¹⁷, -NR¹⁸R¹⁹,
   =NR¹⁹, -C(O)R²⁰, -S(O)ₘR²⁰, -C(O)OR¹⁷, -OC(O)R²⁰, -C(O)NR¹⁸R¹⁹, -NR¹⁸C(O) R²⁰, -S(O)₂NR¹⁸R¹⁹, -NR¹⁸S(O)₂R²⁰, cycloalkyl optionally substituted with one or more R¹², heterocycloalkyl optionally substituted with one or more R¹², aryl optionally substituted with one or more R⁹, or heteroaryl optionally substituted with one or more R¹⁰;
R¹² at each occurrence is independently selected from the group consisting of halogen, alkyl, haloalkyl, -OH, =O, alkoxy, haloalkoxy, -SH, =S, -S(O)_{q}- alkyl, -S(O)_{q}-haloalkyl, -NH₂, alkylamino, dialkylamino, and N-linked-heterocycloalkyl;
R¹³, R¹⁴, R¹⁵, R¹⁷, R¹⁸, and R¹⁹ at each occurrence are independently selected from the group consisting of hydrogen and alkyl wherein said alkyl is optionally substituted with one or more substituents selected from the group consisting of halogen, -OH, =O, alkoxy, haloalkoxy, -SH, =S, -S(O)_{q}-alkyl, -S(O)_{q}-haloalkyl, -NH₂, alkylamino, dialkylamino, and N-linked-heterocycloalkyl;
R¹⁶ and R²⁰ at each occurrence are independently alkyl optionally substituted with one or more substituents selected from the group consisting of halogen, -OH, =O, alkoxy, haloalkoxy, -SH, =S, -S(O)_{q}-alkyl, -S(O),-haloalkyl, -NH₂, alkylamino, dialkylamino, and N-linked-heterocycloalkyl;
m is 1 or 2;
n is 1 or 2;
p is 1 or 2; and
q is 0, 1, or 2.

### DETAILED DESCRIPTION OF THE INVENTION

### Definitions

The definitions and explanations below are for the terms as used throughout this entire document including both the specification and the claims. Reference to compounds as described herein (e.g. a compound of Formula I), includes reference to Formula I including any sub-generic embodiments thereof, e.g. Formula Ia (including all sub-generic embodiments thereof). Throughout the specification and the appended claims, a given formula or name shall encompass all isomers thereof, such as stereoisomers (e.g. diastereomers, enantiomers), geometrical isomers, tautomers, and mixtures thereof where such isomers exist, unless the description designates a specific isomer.

It should be noted that, as used in this specification and the appended claims, the singular forms "a", "an", and "the" include plural referents unless the content clearly dictates otherwise. Thus, for example, reference to a composition containing "a compound" includes a composition containing a single compound, as well as a composition containing a mixture of two or more compounds. It should also be noted that the term "or" is generally employed in its sense including "and/or" unless the content clearly dictates otherwise.

Compounds were named using ChemDraw Ultra v. 10.0.4, (available from Cambridgesoft at 100 Cambridge Park Drive, Cambridge, MA 02140). Alternatively, the names were generated based on the IUPAC rules or were derived from names originally generated using the aforementioned nomenclature programs. In any instance where there may be any ambiguity between a name given to a compound structure, or if no name is provided for a given structure, the provided structure is intended to clearly define the compound, and those compounds only described by the given structure can be readily named using the above methods, or other methods known to one skilled in the art.

Where multiple substituents are indicated as being attached to a structure, those substituents are independently selected. For example "C₁₋₆ alkyl optionally substituted with one or more substituents R¹¹" indicates that alkyl may be substituted with one or more R¹¹ groups, wherein each R¹¹ group is independently selected from the Markush group of options (i.e., can be the same or different than another R¹¹ group). Similarly, when different R groups are described as having the same Markush group of options, each R is independently selected from the Markush group of options. For example "R² and R³ are independently selected from the group consisting of hydrogen, halogen, etc.", means that R² is independently selected from hydrogen, halogen etc., and R³ is independently selected from hydrogen, halogen, etc. As such, these R group definitions can be readily narrowed independently in a subsequent dependent claim. In the instance where one set of R² and R³ attached to the same carbon can combine with the carbon to which they are attached to form a C₃₋₇ carbocyclic ring or 3 to 7 membered heterocyclic ring, a spirocyclic ring substituent is formed. When n is 1, there is clearly only one set of R² and R³ that can form such a spirocyclic ring, and when n is 2, there are two sets of R² and R³ that can form such a spirocyclic ring, but clearly only one or the other, and not both, of these sets of R² and R³ may form such a spirocyclic ring. It is understood that for any optionally substituted group, any such substitution results in a stable molecule.

The term "alkyl", e.g. "C₁₋₃ alkyl" or "C₁₋₆ alkyl", by itself or as part of another substituent, means, unless otherwise stated, a straight or branched chain saturated or unsaturated (mono or poly-unsaturated) hydrocarbon radical having from 1 to x carbon atoms, such as from 1 to 6 carbon atoms (i.e. C₁₋₆ alkyl) or from 1 to 3 carbon atoms (i.e. C₁₋₃ alkyl). When unsaturated, alkyl includes C₂₋ₓ alkenyl, e.g. C₂₋₆ alkenyl as well as C₂₋ₓ alkynyl, e.g. C₂₋₆ alkynyl. The term "alkyl" includes di- and multivalent radicals. For example, the term "alkyl" includes "alkylene" wherever appropriate, e.g., when the formula indicates that the alkyl group is divalent or when substituents are joined to form a ring. "Alkylene" is exemplified by, but not limited to, -CH₂CH₂-. Examples of C₁₋₆ alkyl radicals include, but are not limited to methyl, ethyl, *n*-propyl, *iso*-propyl, *n*-butyl, *tert*-butyl, *iso-*butyl, *sec*-butyl, as well as homologs and isomers of, for example, *n*-pentyl, and *n*-hexyl. Examples of C₁₋₃ alkyl radicals include methyl, ethyl, *n*-propyl, and *iso-*propyl. Exemplary alkenyl groups include vinyl, 2-propenyl, 1-but-3-enyl, crotyl, 2-(butadienyl), 2,4-pentadienyl, 3-(1,4-pentadienyl), 2-isopentenyl, 1-pent-3-enyl, 1-hex-5-enyl and the like.. Exemplary alkynyl groups include prop-1-ynyl, prop-2-ynyl (i.e., propargyl), ethynyl and 3-butynyl. Where it is indicated that alkyl is optionally substituted with one or more substituents, one or more is typically 1, 2, 3, 4, or 5, also 1, 2, 3, or 4, also 1, 2, or 3, also 1 or 2, or one, where multiple substituents are independently selected unless indicated otherwise. It is understood that any substitutions of alkyl, or alkyl substituted on another moiety, are attached at any available atom to provide a stable compound.

The terms "alkoxy", "alkylamino", "dialkylamino", and "alkylthio" (or thioalkoxy) refer to substituted or unsubstituted alkyl groups (as defined above) that are attached to the remainder of the molecule via an oxygen atom, an amino group, or a sulfur atom, respectively. Where the group designation has C₁₋ₓ, e.g. C₁₋₆, this indicates the number of carbon atoms in the alkyl portion of the group. For example, "C₁₋₆ alkylamino" refers to an amino group substituted with one C₁₋₆ alkyl group and "C₁₋₆ dialkylamino" refers to an amino group substituted independently with two C₁₋₆ alkyl groups. The alkyl group is as described herein above. Where it is indicated that an alkyl group within alkoxy, alkylamino, dialkylamino, or alkylthio is optionally substituted with one or more substituents, one or more is typically 1, 2, 3, 4, or 5, also 1, 2, 3, or 4, also 1, 2, or 3, also 1 or 2, or one, where multiple substituents are independently selected unless indicated otherwise. It is understood that any substitutions of alkoxy, alkylamino, dialkylamino, or alkylthio, or alkoxy, alkylamino, dialkylamino, or alkylthio substituted on another moiety, are attached at any available atom to provide a stable compound.

The term "C₃₋₆ cycloalkyl" by itself or in combination with other terms, means a saturated or mono- or polycyclic unsaturated, non-aromatic carbocyclic radical having from 3 to 6 carbon atoms. Examples of cycloalkyl include, but are not limited to, cyclopropyl, cyclobutyl, cyclopentyl, cyclohexyl, 1-cyclohexenyl, 3-cyclohexenyl, and the like. Examples of unsaturated cycloalkyl include cyclopentenyl and cyclohexenyl. Where it is indicated that cycloalkyl is optionally substituted with one or more substituents, one or more is typically 1, 2, 3, 4 or 5, also 1, 2, 3, or 4, also 1, 2, or 3, also 1 or 2, or one, where multiple substituents are independently selected unless indicated otherwise. It is understood that any substitutions of cycloalkyl, or cycloalkyl substituted on another moiety, are attached at any available atom to provide a stable compound.

The term "heterocycloalkyl", "heterocyclic", or "heterocycle", by itself or in combination with other terms, means a saturated or unsaturated, non-aromatic ring having, for example, 3- to 10-members or 3- to 8-members, also 4-, 5-, 6- or 7-members, where at least one member and up to 5 members, also up to 3 members, of the ring are heteroatoms selected from, e.g., N, O, S, Si, B and P (in one example N, O and S), remaining ring atoms are carbon atoms, in stable combinations known to those of skill in the art, or a saturated or unsaturated non-aromatic fused ring system having 4- to 8-members (e.g. bicyclic ring system of fused 4- to 8-membered rings), where at least one and up to 5 members, also up to 3 members, are heteroatoms (e.g., from 1 to 5 heteroatoms, also 1 to 3 heteroatoms, selected from N, O and S), and the remaining ring atoms are carbon atoms, in stable combinations known to those of skill in the art. The heterocycloalkyl ring nitrogen, sulfur and phosphorus atoms are optionally oxidized, and the nitrogen atom(s) are optionally quaternized. "4-7 membered heterocycloalkyl" or "5-7 membered heterocycloalkyl" means a monocyclic heterocyclic ring having 4-, 5-, 6-, or 7- members, or 5-, 6-, or 7-members, where 1, 2, or 3, also 1 or 2 members is N, O or S, and the remaining ring atoms are carbon atoms. When the "heterocyclic" group includes a fused aryl, heteroaryl or cycloalkyl ring, then the "heterocyclic" group is attached to the group it is a substituent of via the heterocyclic ring. The point of attachment of heterocycloalkyl from the group to which it is a substituent of can be via a carbon atom or via a heteroatom. Exemplary heterocycloalkyl groups for compounds described herein (e.g. compounds of Formula I) include morpholinyl, thiomorpholinyl, thiomorpholinyl S-oxide, thiomorpholinyl S,S-dioxide, piperazinyl, homopiperazinyl, pyrrolidinyl, pyrrolinyl, imidazolidinyl, tetrahydropyranyl, piperidinyl, tetrahydrofuranyl, tetrahydrothienyl, piperidinyl, homopiperidinyl, homomorpholinyl, homothiomorpholinyl, homothiomorpholinyl S,S-dioxide, oxazolidinonyl, dihydropyrazolyl, dihydropyrrolyl, dihydropyrazolyl, dihydropyridyl, dihydropyrimidinyl, dihydrofuryl, dihydropyranyl, tetrahydrothienyl S-oxide, tetrahydrothienyl S,S-dioxide, homothiomorpholinyl S-oxide, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like. In one example, heterocycloalkyl is cycoalkylamino. Where it is indicated that heterocycloalkyl is optionally substituted with one or more substituents, one or more is typically 1, 2, 3, 4 or 5, also 1, 2, 3, or 4, also 1, 2, or 3, also 1 or 2, or one, where multiple substituents are independently selected unless indicated otherwise. It is understood that any substitutions of heterocycloalkyl, or heterocycloalkyl substituted on another moiety, are attached at any available atom to provide a stable compound.

The term "N-linked-heterocycloalkyl", by itself or as part of another substituent, means the group -NR'R", where R' and R" combine with the nitrogen to form a 5-7 membered heterocycloalkyl, where the heterocycloalkyl may contain an additional heteroatom within the ring, such as O, N, or S, and may also be further substituted with alkyl, e.g. C₁₋₆ alkyl, C₂₋₆alkenyl or C₂₋₆alkynyl. A nitrogen atom in the ring is bound to the remainder of the compound. Examples of N-linked-heterocycloalkyl include, but are not limited to, piperidine, piperazine, 4-methylpiperazine, morpholine, and thiomorpholine. Where it is indicated that N-linked-heterocycloalkyl is optionally substituted with one or more substituents, one or more is typically 1, 2, 3, 4 or 5, also 1, 2, 3, or 4, also 1, 2, or 3, also 1 or 2, or one, where multiple substituents are independently selected unless indicated otherwise. It is understood that any substitutions of N-linked-heterocycloalkyl, or N-linked-heterocycloalkyl substituted on another moiety, are attached at any available atom to provide a stable compound.

The term "aryl", by itself or as part of another substituent means, unless otherwise stated, means an aromatic monocyclic or polycyclic carbocyclic group having 6 to 14 carbon atoms, or 6 to 10 carbon atoms, in one example phenyl. Exemplary aryl groups include a fused cycloalkyl, heterocycloalkyl or heteroaryl ring (e.g., from 1 to 3 other rings). When the "aryl" group includes a fused cycloalkyl, heterocycloalkyl or heteroaryl group, then the "aryl" group is attached to the group it is a substituent of via an aryl ring (e.g., a phenyl ring). An "optionally substituted aryl" group is optionally substituted with one or more substituents as described herein (e.g., with 1 to 5 independent substituents). Non-limiting examples of aryl groups include phenyl, 1-naphthyl, 2-naphthyl, indanyl, indenyl, dihydronaphthyl, fluorenyl, tetralinyl, benzo[d][1,3]dioxolyl, 2,3-dihydrobenzo[b][1,4]dioxinyl, or 6,7,8,9-tetrahydro-5H-benzo[a]cycloheptenyl. In one example, "aryl" groups include phenyl, benzo[d][1,3]dioxolyl and naphthyl. In one example, "aryl" is phenyl. Where it is indicated that aryl is optionally substituted with one or more substituents, one or more is typically 1, 2, 3, 4 or 5, also 1, 2, 3, or 4, also 1, 2, or 3, also 1 or 2, or one, where multiple substituents are independently selected unless indicated otherwise. It is understood that any substitutions of aryl, or aryl substituted on another moiety, are attached at any available atom to provide a stable compound.

The term "heteroaryl", by itself or as part of another substituent means, unless otherwise stated, a polyunsaturated, 5-, 6- or 7-membered aromatic moiety containing at least one heteroatom (e.g., 1 to 5 heteroatoms, also 1-3 heteroatoms) selected from N, O, S, Si and B (in one example N, O and S), and the remaining ring atoms are carbon atoms, in stable combinations known to those of skill in the art. Heteroaryl ring nitrogen and sulfur atoms are optionally oxidized, and the nitrogen atom(s) are optionally quaternized. The "heteroaryl" group can be a single ring or be fused to other aryl, heteroaryl, cycloalkyl or heterocycloalkyl rings (e.g., from 1 to 3 other rings). When the "heteroaryl" group includes a fused aryl, cycloalkyl or heterocycloalkyl ring, then the "heteroaryl" group is attached to the group it is a substituent of via a heteroaryl ring. The point of attachment of heteroaryl to the group it is a substituent of can be via a carbon atom or a heteroatom (e.g. nitrogen). In one example, the heteroaryl group has from 1 to 10 carbon atoms and from 1 to 5 heteroatoms selected from O, S and N. "5 or 6 membered heteroaryl" means a monocyclic heteroaryl ring having 5- or 6-members, where 1, 2, 3, or 4, also 1, 2 or 3, also 1 or 2, also 1 member(s) is N, O or S. Non-limiting examples of heteroaryl groups include pyridyl, pyrimidinyl, quinolinyl, benzothienyl, indolyl, indolinyl, pryidazinyl, pyrazinyl, isoindolyl, isoquinolyl, quinazolinyl, quinoxalinyl, phthalazinyl, imidazolyl, isoxazolyl, pyrazolyl, oxazolyl, thiazolyl, indolizinyl, indazolyl, benzothiazolyl, benzimidazolyl, benzofuranyl, furanyl, thienyl, pyrrolyl, oxadiazolyl, thiadiazolyl, triazolyl, tetrazolyl, isothiazolyl, naphthyridinyl, isochromanyl, chromanyl, tetrahydroisoquinolinyl, isoindolinyl, isobenzothienyl, benzoxazolyl, pyridopyridyl, purinyl, benzodioxolyl, triazinyl, pteridinyl, benzothiazolyl, imidazopyridyl, imidazothiazolyl, dihydrobenzisoxazinyl, benzisoxazinyl, benzoxazinyl, dihydrobenzisothiazinyl, benzopyranyl, benzothiopyranyl, chromonyl, chromanonyl, pyridyl-N-oxide, tetrahydroquinolinyl, dihydroquinolinyl, dihydroquinolinonyl, dihydroisoquinolinonyl, dihydrocoumarinyl, dihydroisocoumarinyl, isoindolinonyl, benzodioxanyl, benzoxazolinonyl, pyrrolyl N-oxide, pyrimidinyl N-oxide, pyridazinyl N-oxide, pyrazinyl N-oxide, quinolinyl N-oxide, indolyl N-oxide, indolinyl N-oxide, isoquinolyl N-oxide, quinazolinyl N-oxide, quinoxalinyl N-oxide, phthalazinyl N-oxide, imidazolyl N-oxide, isoxazolyl N-oxide, oxazolyl N-oxide, thiazolyl N-oxide, indolizinyl N-oxide, indazolyl N-oxide, benzothiazolyl N-oxide, benzimidazolyl N-oxide, pyrrolyl N-oxide, oxadiazolyl N-oxide, thiadiazolyl N-oxide, triazolyl N-oxide, tetrazolyl N-oxide, benzothiopyranyl S-oxide, benzothiopyranyl S,S-dioxide. In one example, heteroaryl groups include imidazolyl, pyrazolyl, thiadiazolyl, triazolyl, isoxazolyl, oxazolyl, isothiazolyl, thiazolyl, oxadiazolyl, pyridazinyl, pyrazinyl, pyrimidinyl, and pyridyl. Where it is indicated that heteroaryl is optionally substituted with one or more substituents, one or more is typically 1, 2, 3, 4 or 5, also 1, 2, 3, or 4, also 1, 2, or 3, also 1 or 2, or one, where multiple substituents are independently selected unless indicated otherwise. It is understood that any substitutions of heteroaryl, or heteroaryl substituted on another moiety, are attached at any available atom to provide a stable compound.

The terms "halo" or "halogen," by themselves or as part of another substituent, mean at least one of fluorine, chlorine, bromine and iodine.

By "haloalkyl" or "haloalkoxy" is meant an alkyl, alkenyl, alkynyl, alkoxy, alkenyloxy or alkynyloxy radical, as defined above, wherein at least one hydrogen atom of the foregoing is replaced by a halogen atom, where typically 1, 2, 3, 4, or 5, also 1, 2, 3, or 4, also 1, 2, or 3, also 1 or 2, or 1 hydrogen atom is replaced by an independently selected halogen. More typically, 1, 2 or 3 hydrogen atoms on the same carbon are replaced with 1, 2 or 3 halogen atoms. In one example the halogen is fluorine or chlorine, in one example fluorine. The term "haloalkyl" or "haloalkoxy" is meant to include monohaloalkyl and polyhaloalkyl. Where the group designation has C₁₋ₓ, e.g. C₁₋₆, this indicates the number of carbon atoms in the alkyl, alkenyl or alkynyl portion of the group. For example, the term "C₁₋₆ haloalkyl" is meant to include, but not limited to, chloromethyl, 1-bromoethyl, fluoromethyl, difluoromethyl, trifluoromethyl, 2,2,2-trifluoroethyl, perfluoroethyl, 4-chlorobutyl, and 3-bromopropyl; and the term "C₁₋₆ haloalkoxy" is meant to include, but not limited to, fluoromethoxy, difluoromethoxy, trifluoromethoxy, 2,2,2-trifluoroethoxy, and perfluoroethoxy.

As used herein, the term "heteroatom" includes oxygen (O), nitrogen (N), sulfur (S), silicon (Si), boron (B) and phosphorus (P). In one example heteroatoms are O, S and N.

By "oxo" is meant the group =O.

As used herein, the term "aromatic ring" or "non-aromatic ring" is consistent with the definition commonly used in the art. For example, aromatic rings include phenyl and pyridyl. Non-aromatic rings include cyclohexanes, cyclohexenes, and the like.

As used herein, the term "fused ring system" means at least two rings, wherein each ring has at least 2 atoms in common with another ring. "Fused ring systems can include aromatic as well as non aromatic rings. Examples of "fused ring systems" are naphthalenes, indoles, quinolines, chromenes and the like. Likewise, the term "fused ring" refers to a ring that has at least two atoms in common with the ring to which it is fused.

As used herein, the term "selective" or "selectivity" as it relates to kinase activity, means that a compound as described herein, e.g. a compound of Formula I, is a more potent inhibitor of a particular kinase, such as LRRK2 kinase, when compared to another kinase. While LRRK2 has other enzymatic activities, it is understood that when inhibitory activity or selectivity of LRRK2, or any mutation thereof, is mentioned, it is the LRRK2 kinase activity that is being referred to, unless clearly stated otherwise. As such, selectivity of LRRK2 relative to another kinase indicates a comparison of the IC₅₀ of a compound on the kinase activity of LRRK2 to the IC₅₀ of the compound on the kinase activity of another kinase. For example, a compound that is 10 fold selective for LRRK2 kinase activity relative to another kinase activity will have a ratio of IC₅₀(other kinase) ÷ IC₅₀(LRRK2) = 10 (or a ratio of IC₅₀(LRRK2) ÷ IC₅₀(other kinase) = 0.1).

The phrase "therapeutically effective amount" as used herein means that amount of a compound, material, or composition as described herein (e.g. compounds of Formula I and compositions thereof), which is effective for producing a desired therapeutic effect, at a reasonable benefit/risk ratio applicable to any medical treatment. For example, a "therapeutically effective amount" is an amount effective to reduce or lessen at least one symptom of the disease or condition being treated or to reduce or delay onset of one or more clinical markers or symptoms associated with the disease or condition, or to modify or reverse the disease process.

The terms "treatment" or "treating" when referring to a disease or condition, means producing a desired therapeutic effect. Exemplary therapeutic effects include delaying onset or reducing at least one symptom associated with the disease, positively affecting (e.g., reducing or delaying onset) of a clinical marker associated with the disease and slowing or reversing disease progression.

The term "pharmaceutically acceptable" refers to those properties and/or substances that are acceptable to a patient (e.g., human patient) from a toxicological and/or safety point of view.

The term "salts" means salts of the compounds as described herein, e.g. compounds of Formula I, which are prepared with relatively nontoxic acids or bases, depending on the particular substituents found on the compounds described herein. A salt includes a "pharmaceutically acceptable salt", meaning a salt that is acceptable to a patient (e.g., human patient) from a toxicological and/or safety point of view. It is understood that where any compound includes a salt, the salt could be selected to be a pharmaceutically acceptable salt. A compound of Formula I may be prepared as a salt. Such salts and their preparation for use as pharmaceuticals are readily known to those of skill in the art. Such salts may provide improved properties, e.g. solubility or pharmacokinetic properties, such that the activity of the compound of Formula I is enhanced upon administration to a subject. It is understood that such salts are effectively equivalent to compounds of Formula I, i.e. when such a salt is administered into a subject, the administration effectively encompasses the use of a compound of Formula I.

The term "pharmaceutically acceptable solvate" means any solvate, including any hydrate, of a compound as described herein, e.g. a compound of Formula I, which is prepared with a relatively nontoxic solvent or solvents. A compound as described herein, e.g. a compound of Formula I, can exist in unsolvated forms as well as solvated forms, including hydrated forms. Such solvates may provide improved properties, e.g. solubility or pharmacokinetic properties, such that the activity of the compound of Formula I is enhanced upon administration to a subject. It is understood that such pharmaceutically acceptable solvated forms are effectively equivalent to compounds of Formula I, i.e. when such a solvated form is administered into a subject, the administration effectively encompasses the use of a compound of Formula I.

The term "pharmaceutically acceptable carrier" means any pharmaceutically acceptable ingredient known to those of skill in the art, which is typically considered a non-active ingredient.

The term "pharmaceutically acceptable derivative" or "prodrug" means any derivative of a compound of Formula I that is suitable for pharmaceutical use. For example, a prodrug of a compound as described herein which, upon administration to a recipient, is capable of providing, either directly or indirectly, a compound as described herein (e.g. a compound of Formula I). In some examples, a prodrug increases the bioavailability of a compound as described herein when such compound is administered to a mammal (e.g., by allowing an orally administered compound to be more readily absorbed into the blood stream) or which enhance delivery of the parent compound to a biological compartment (e.g., the brain) relative to the parent species. It is understood that such a prodrug form is effectively equivalent to a compound of Formula I, i.e. when such a prodrug form is administered into a subject, the administration effectively encompasses the use of a compound of Formula I.

The term "polymorph" refers to a crystal form of a compound as described herein. It is understood that a compound as described herein may occur in many different crystal forms, or polymorphs, or can be made into amorphous form (i.e. solid form without any defined crystal structure). While such varied solid forms may have different pharmaceutical properties, it is understood that any such crystal form comprises a compound as described herein, i.e. it is encompassed by a compound of Formula I. Similarly, a salt or solvate of a compound of Formula I may exist as polymorphs, where any such polymorph is encompassed by a salt or a pharmaceutically acceptable solvate of a compound of Formula I.

The term "metabolite" refers to a derivative of a compound as described herein resulting from administering such a compound to a recipient, wherein the metabolite results from metabolic processes in the body of a recipient. In some examples, a metabolite may be pharmaceutically active. Any metabolites may be identified using routine techniques known in the art, and their biological activity assessed as described herein.

The term "conjugate" refers to a derivative of a compound as described herein resulting in the linking of a suitable adjunct to provide additional features or uses. A compound of Formula I may be further conjugated via a suitably reactive group to link a moiety to the compound of Formula I, such that the linked moiety provides, for example, improved targeting to certain tissues, improved transport across the blood brain barrier, a suitable binding molecule for use as a probe, or the like. The portion of the conjugate that is derived from a compound of Formula I is expected to have similar properties to a compound of Formula I, for example such portion of the conjugate will readily bind to LRRK2 in a similar manner to the non-derivative compound of Formula I. Such conjugates can be used, for example, for targeted drug delivery, improved delivery to the brain or CNS, as a probe for identifying LRRK2 in a biological mixture or for isolating LRRK2 from a biological mixture, or the like.

The term "isotopically enhanced" or "isotopically enhanced form" means that a compound as described herein, e.g. a compound of Formula I, may be modified to contain unnatural proportions of certain atomic isotopes at one or more of the atoms that constitute such a compound. For example, a compound can be radiolabeled with radioactive isotopes, such as for example tritium (³H), iodine-125 (¹²⁵I) or carbon-14 (¹⁴C). Such isotopic variations of a compound as described herein, whether radioactive or not, is effectively encompassed by compounds as described herein. For example, a compound in which one or more of the hydrogen atoms are replaced with another stable isotope of hydrogen (i.e., deuterium) or a radioactive isotope (i.e., tritium), is expected to have similar activity to the compound without isotopic enhancement as it relates to LRRK2 kinase inhibition, and such a compound is effectively equivalent to a compound of Formula I. Such an isotopically enhanced compound may be useful, for example, in detection of the compound in vivo or in biological tissue, such as a radiolabelled compound containing ³H or ¹⁴C to assess tissue distribution, or a positron emitting compound containing ¹¹C, ¹⁵O, ¹³N, ¹⁸F or the like useful in positron emission tomography for in vivo imaging. Similarly, a deuterated compound may provide a compound with greater metabolic stability than the analogous non-deuterated compound, such that the deuterated compound has better pharmacokinetic properties. Any isotopically enhanced compound is expected to have similar inhibitory activity as it relates to LRRK2 kinase, and other kinases. Such a compound is readily prepared by those of skill in the art, for example by the methods as described herein or other methods known in the art, where suitable isotopically enhanced reagents may be used to provide the isotopically enhanced compounds.

As used herein, the term "chiral", "enantiomerically enriched" or "diastereomerically enriched" refers to a compound having an enantiomeric excess (ee) or a diastereomeric excess (de) of greater than about 50%, also greater than about 70% and also greater than about 90%. In one example, enantiomeric or diastereomeric excess is higher than about 90%, e.g., those compositions with greater than about 95%, greater than about 97% and greater than about 99% ee or de. The terms "enantiomeric excess" and "diastereomeric excess" are used in their conventional sense. Compounds with a single stereocenter are referred to as being present in "enantiomeric excess", those with at least two stereocenters are referred to as being present in "diastereomeric excess". The value of ee will be a number from 0 to 100, zero being racemic and 100 being enantiomerically pure. For example, a 90% ee reflects the presence of 95% of one enantiomer and 5% of the other(s) in the material in question. Stereoisomers are detectable if a concentration of such stereoisomer in the analyzed mixture can be determined using common analytical methods, such as chiral HPLC.

Hence, in one embodiment, compositions are provided including a first stereoisomer and at least one additional stereoisomer of a compound as described herein, e.g. a compound of Formula I. The first stereoisomer can be present in a diastereomeric or enantiomeric excess of at least about 80%, also at least about 90% and more also at least about 95%. In one embodiment, the first stereoisomer is present in a diastereomeric or enantiomeric excess of at least about 96%, at least about 97%, at least about 98%, at least about 99% or at least about 99.5%. In another embodiment, the compound of Formula I is enantiomerically or diastereomerically pure (diastereomeric or enantiomeric excess is about 100%). Enantiomeric or diastereomeric excess can be determined relative to exactly one other stereoisomer, or can be determined relative to the sum of at least two other stereoisomers. In an exemplary embodiment, enantiomeric or diastereomeric excess is determined relative to all other detectable stereoisomers, which are present in the mixture. Stereoisomers are detectable if a concentration of such stereoisomer in the analyzed mixture can be determined using common analytical methods, such as chiral HPLC.

The terms "use in combination", "combination use" or the like, means use of a compound as described herein with one or more other therapeutics for the treatment, prevention, or amelioration of symptoms of a disease. Combination use includes use of a compound as described herein at any point before, during or after treatment with one or more other therapeutic treatments, for example a compound as described herein and another therapeutic agent can be administered essentially simultaneously, either in different vehicles, or can be administered in the same vehicle (e.g. can be manufactured into the same pill, tablet, solution, etc.); or a compound as described herein can be administered prior to (e.g. minutes, hours, days, or weeks before) administering another therapeutic agent; or a compound as described herein can be administered subsequently to (e.g. minutes, hours, days, or weeks after) administering another therapeutic agent.

The term "LRRK2-mediated condition", "Leucine-rich repeat kinase 2 mediated disorder" or any other variation thereof, as used herein means any disease or other condition in which LRRK2, including any mutations thereof, is known to play a role, or a disease state that is associated with elevated activity or expression of LRRK2, including any mutations thereof. For example, a "LRRK2-mediated condition" may be relieved by inhibiting LRRK2 kinase activity. Such conditions include certain neurodegenerative diseases, such as Lewy body diseases, including, but not limited to, Parkinson's disease, Lewy body variant of Alzheimer's disease, combined Parkinson's disease and Alzheimer's disease, dementia with Lewy bodies, diffuse Lewy body disease, as well as any syndrome identified as multiple system atrophy; certain cancers, such as melanoma, papillary renal cell carcinoma and papillary thyroid carcinoma; certain autoimmune diseases, such as Inflammatory Bowel Disease (e.g. Crohn's disease and ulcerative colitis); and leprosy.

The term "neurodegenerative diseases" includes any disease or condition characterized by problems with movements, such as ataxia, and conditions affecting cognitive abilities (*e.g.,* memory) as well as conditions generally related to all types of dementia. "Neurodegenerative diseases" may be associated with impairment or loss of cognitive abilities, potential loss of cognitive abilities and/or impairment or loss of brain cells. Exemplary "neurodegenerative diseases" include Alzheimer's disease, Parkinson's disease, amyotrophic lateral sclerosis (ALS), Down syndrome, dementia, multi-infarct dementia, mild cognitive impairment (MCI), epilepsy, seizures, Huntington's disease, neurodegeneration induced by viral infection (*e.g.* AIDS, encephalopathies), traumatic brain injuries, as well as ischemia and stroke. "Neurodegenerative diseases" also includes any undesirable condition associated with the disease. For instance, a method of treating a neurodegenerative disease includes methods of treating or preventing loss of neuronal function characteristic of neurodegenerative disease.

In one aspect, compounds are provided having a structure according to Formula I as defined in claim 1.

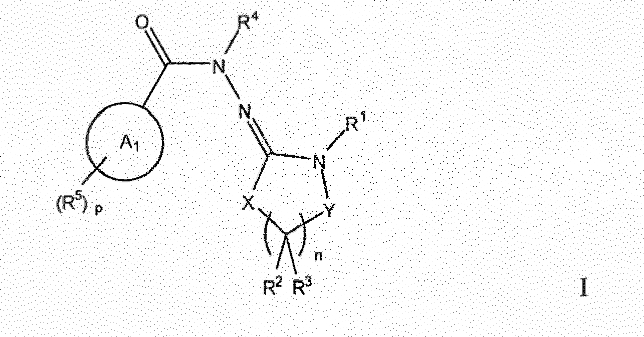

In a particular embodiment, compounds of Formula I are provided, or a salt thereof, wherein, in addition to the definitions above:
R¹ is C₁₋₆ alkyl, C₂₋₆ alkenyl, C₂₋₆ alkynyl, C₃₋₆ cycloalkyl, 3-6 membered heterocycloalkyl, aryl optionally substituted with one or more R⁹, or heteroaryl optionally substituted with one or more R¹⁰, wherein C₁₋₆ alkyl, C₂₋₆ alkenyl, and C₂₋₆ alkynyl are optionally substituted with one or more R¹¹; and wherein C₃₋₆ cycloalkyl and 3-6 membered heterocycloalkyl are optionally substituted with one or more R¹²;
R² and R³ at each occurrence are independently selected from the group consisting of hydrogen, halogen, C₁₋₆ alkyl, C₂₋₆ alkenyl, C₂₋₆
   alkynyl, C₃₋₆ cycloalkyl, and 3-6 membered heterocycloalkyl
or R² and R³ combined form =O or =NR¹⁵;
or one set of R² and R³ attached to the same carbon can combine with the carbon to which they are attached to form a C₃₋₆ carbocyclic ring or 3 to 7 membered heterocyclic ring, wherein each ring is optionally substituted with one or more substituents selected from the group consisting of halogen, C₁-₆ alkyl, C₂₋₆ alkenyl, C₂₋₆ alkynyl, -CN, -NO₂, =O, -OR¹³, -SR¹³, -NR¹⁴R¹⁵,
   =NR¹⁵, -C(O)R¹⁶, -S(O)ₘR¹⁶, C₃₋₆ cycloalkyl, and 3-6 membered heterocycloalkyl, wherein C₃₋₆ cycloalkyl, and 3-6 membered heterocycloalkyl are optionally substituted with one or more R¹² and wherein C₁₋₆ alkyl, C₂₋₆ alkenyl, and C₂₋₆ alkynyl are optionally substituted with one or more substituents selected from the group consisting of halogen, -CN, -NO₂, =O, -OR¹³, -SR¹³, -NR¹⁴R¹⁵, =NR¹⁵, -C(O)R¹⁶, -S(O)ₘR¹⁶, C₃₋₆ cycloalky), and 3-6 membered heterocycloalkyl;
R⁴ is hydrogen or C₁₋₃ alkyl;
R⁵ at each occurrence is independently selected from the group consisting of halogen, C₁₋₆ alkyl, C₂₋₆ alkenyl, C₂₋₆ alkynyl
R⁶ and R⁷ are independently selected from the group consisting of hydrogen, halogen, C₁₋₆ alkyl, C₂₋₆ alkenyl, C₂₋₆
   alkynyl, -CN, -NO₂, -OR¹³, -SR¹³, -NR¹⁴R¹⁵, -C(O)R¹⁶, -S(O)ₘR¹⁶, C₃₋₆ cycloalkyl, and 3-6 membered heterocycloalkyl, wherein C₁₋₆ alkyl, C₂₋₆ alkenyl, and C₂₋₆ alkynyl are optionally substituted with one or more substituents selected from the group consisting of halogen, -CN, -NO₂, =O, -OR¹³, -SR¹³, -NR¹⁴R¹⁵, =NR¹⁵, -C(O)R¹⁶, -S(O)ₘR¹⁶, C₃₋₆ cycloalkyl, and 3-6 membered heterocycloalkyl, and wherein C₃₋₆ cycloalkyl, and 3-6 membered heterocycloalkyl, or said C₁₋₆ alkyl, C₂₋₆ alkenyl, or C₂₋₆ alkynyl, is optionally substituted with one or more R¹¹;
R⁸ is selected from the group consisting of hydrogen, C₁-₆ alkyl, C₂₋₆ alkenyl, and C₂₋₆ alkynyl, wherein C₁-₆ alkyl, C₂₋₆ alkenyl, and C₂₋₆ alkynyl are optionally substituted with one or more substituents selected from the group consisting of halogen, -CN, -NO₂, =O, -OR¹³, -SR¹³, -NR¹⁴R¹⁵, =NR¹⁵, -C(O)R¹⁶, -S(O)ₘR¹⁶, and N-linked heterocycloalkyl;
R⁹ and R¹⁰ at each occurrence are independently selected from the group consisting of halogen, C₁₋₆ alkyl, C₂₋₆ alkenyl, C₂₋₆
   alkynyl, -CN, -NO₂, -OR¹⁷, -SR¹⁷, -NR¹⁸R¹⁹, -C(O)R²⁰, -S(O)ₘR²⁰, -C(O)OR¹⁷, -O C(O)R²⁰, -C(O)NR¹⁸R¹⁹, -NR¹⁸C(O)R²⁰, -S(O)₂NR¹⁸R¹⁹, -NR¹⁸S(O)₂R²⁰, C₃₋₆ cycloalkyl, and 3-6 membered heterocycloalkyl, wherein C₁₋₆ alkyl, C₂₋₆ alkenyl, and C₂₋₆ alkynyl are optionally substituted with one or more halogen, C₃₋₆ cycloalkyl, -CN, -NO₂, =O, -OR¹⁷, -SR¹⁷, -NR¹⁸R¹⁹,
   =NR¹⁹, -C(O)R²⁰, -S(O)ₘR²⁰, -C(O)OR¹⁷, -OC(O)R²⁰, -C(O)NR¹⁸R¹⁹, -NR¹⁸C(O) R²⁰, -S(O)₂NR¹⁸R¹⁹, -NR¹⁸S(O)₂R²⁰, C₃₋₆ cycloalkyl, and 3-6 membered heterocycloalkyl, and wherein C₃₋₆ cycloalkyl, and 3-6 membered heterocycloalkyl or said C₁₋₆ alkyl, C₂₋₆ alkenyl, or C₂₋₆ alkynyl, are optionally substituted with one or more R¹²;
R¹¹ at each occurrence is independently selected from the group consisting of halogen, -CN, -NO₂, =O, -OR¹⁷, -SR¹⁷, -NR¹⁸R¹⁹,
   =NR¹⁹, -C(O)R²⁰, -S(O)ₘR²⁰, -C(O)OR¹⁷, -OC(O)R²⁰, -C(O)NR¹⁸R¹⁹, -NR¹⁸C(O) R²⁰, -S(O)₂NR¹⁸R¹⁹, -NR¹⁸S(O)₂R²⁰, C₃₋₆ cycloalkyl optionally substituted with one or more R¹², 3-6 membered heterocycloalkyl optionally substituted with one or more R¹², aryl optionally substituted with one or more R⁹, or heteroaryl optionally substituted with one or more R¹⁰;
R¹² at each occurrence is independently selected from the group consisting of halogen, C₁-₆ alkyl, C₁₋₆ haloalkyl, C₂₋₆ alkenyl, C₂₋₆ haloalkenyl, C₂₋₆ alkynyl, C₂₋₆ haloalkynyl, -OH, =O, C₁₋₆ alkoxy, C₁₋₆ haloalkoxy, -SH, =S, -S(O)_{q}-C₁₋₆ alkyl, -S(O)_{q}-C₁₋₆ haloalkyl, -NH₂, C₁₋₆ alkylamino, C₁₋₆ dialkylamino, and N-linked-heterocycloalkyl;
R¹³, R¹⁴, R¹⁵, R¹⁷, R¹⁸, and R¹⁹ at each occurrence are independently selected from the group consisting of hydrogen, C₁₋₆ alkyl, C₂₋₆ alkenyl, and C₂₋₆ alkynyl, wherein C₁₋₆ alkyl, C₂₋₆ alkenyl, and C₂₋₆ alkynyl are optionally substituted with one or more substituents selected from the group consisting of halogen, -OH, =O, C₁₋₆ alkoxy, C₁₋₆ haloalkoxy, -SH, =S, -S(O)_{q}-C₁₋₆ alkyl, -S(O)_{q}-C₁₋₆ haloalkyl, -NH₂, C₁₋₆ alkylamino, C₁₋₆ dialkylamino, and N-linked-heterocycloalkyl;
R¹⁶ and R²⁰ at each occurrence are independently selected from the group consisting of C₁₋₆ alkyl, C₂₋₆ alkenyl, and C₂₋₆ alkynyl, wherein C₁₋₆ alkyl, C₂₋₆ alkenyl, and C₂₋₆ alkynyl are optionally substituted with one or more substituents selected from the group consisting of halogen, -OH, =O, C₁₋₆ alkoxy, C₁₋₆ haloalkoxy, -SH, =S, -S(O)_{q}-C₁₋₆ alkyl, -S(O)_{q}-C₁₋₆ haloalkyl, -NH₂, C₁₋₆ alkylamino, C₁₋₆ dialkylamino, and N-linked-heterocycloalkyl;
m is 1 or 2;
n is 1 or 2;
p is 1 or 2; and
q is 0, 1, or 2.
A₁ is selected from the group consisting of pyridine, pyrimidine, pyridazine, pyrazine, triazine, pyrrole, pyrazole, imidazole, triazole, tetrazole, oxazole, isoxazole, oxadiazole, thiazole, isothiazole, thiadiazole, furan, and thiophene; each R⁵ is independently selected from the group consisting of halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl; and p is 1 or 2, also 1.

In some embodiments of a compound of Formula I, A₁ is selected from the group consisting of pyrrole, pyrazole, imidazole, triazole, tetrazole, oxazole, isoxazole, oxadiazole, thiazole, isothiazole, thiadiazole, furan, and thiophene. In some embodiments, A₁ is selected from the group consisting of pyrrole, pyrazole, imidazole, triazole, tetrazole, oxazole, isoxazole, oxadiazole, thiazole, isothiazole, thiadiazole, furan, and thiophene, and each R⁵ is independently selected from the group consisting of halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl. In some embodiments, A₁ is selected from the group consisting of pyrrole, pyrazole, imidazole, triazole, tetrazole, oxazole, isoxazole, oxadiazole, thiazole, isothiazole, thiadiazole, furan, and thiophene; each R⁵ is independently selected from the group consisting of halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl; and p is 1 or 2, also 1.

In some embodiments of a compound of Formula I, A₁ is selected from the group consisting of oxazole, isoxazole, thiazole, isothiazole, furan, and thiophene. In some embodiments, A₁ is selected from the group consisting of oxazole, isoxazole, thiazole, isothiazole, furan, and thiophene, and each R⁵ is independently selected from the group consisting of halogen, C₁₋₆alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl. In some embodiments, A₁ is selected from the group consisting of oxazole, isoxazole, thiazole, isothiazole, furan, and thiophene; each R⁵ is independently selected from the group consisting of halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl; and p is 1 or 2, also 1.

In some embodiments of a compound of Formula I, A₁ is selected from the group consisting of: 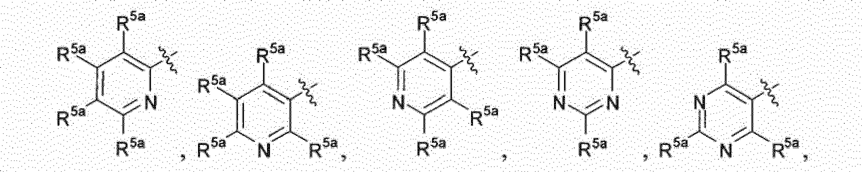 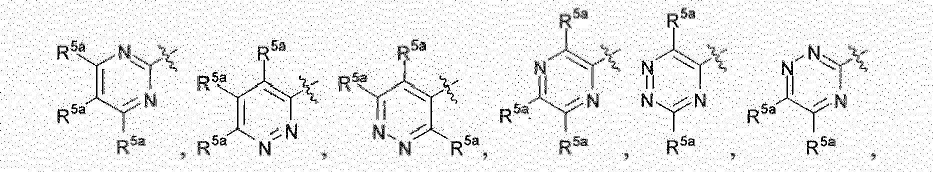 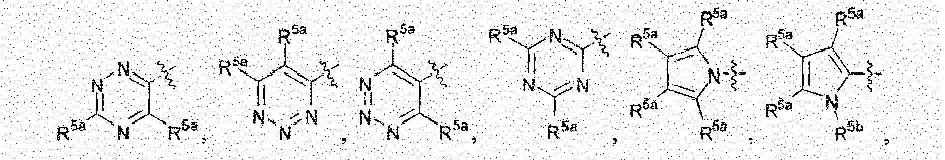 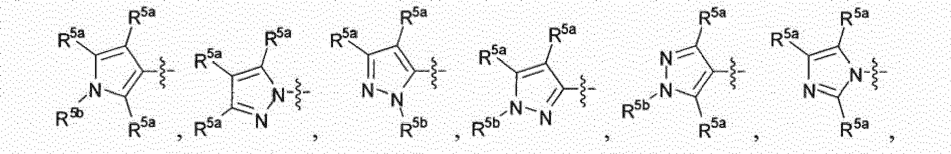 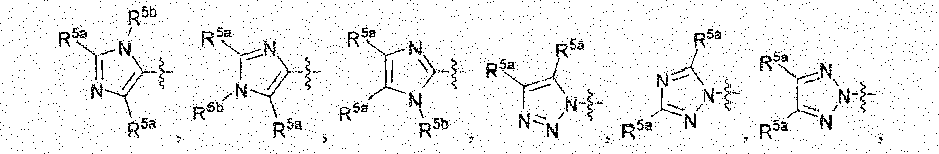 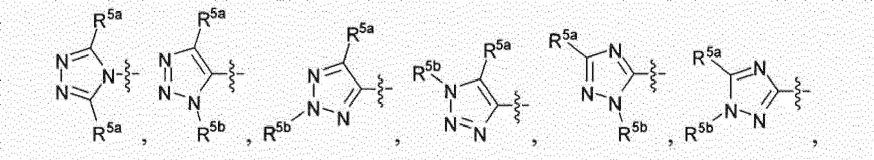 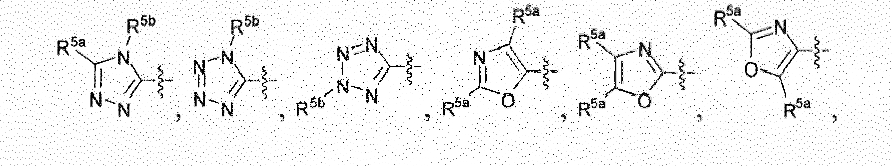 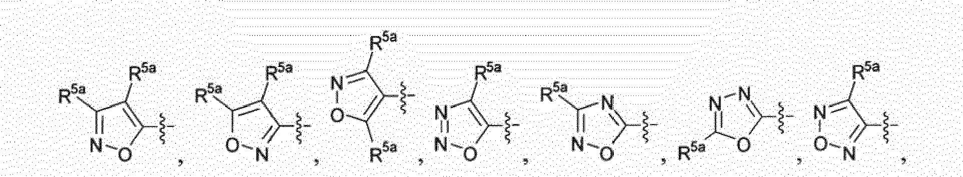 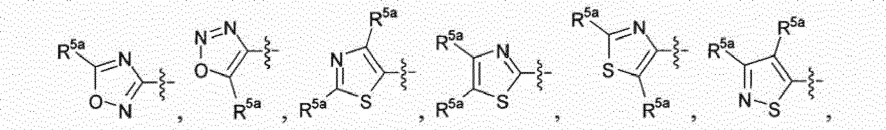 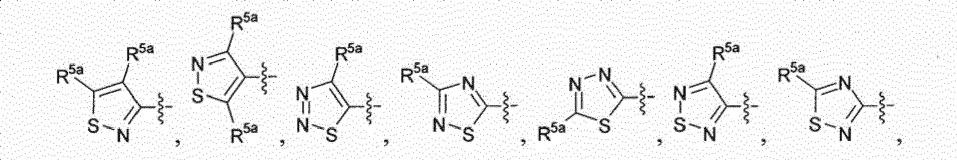 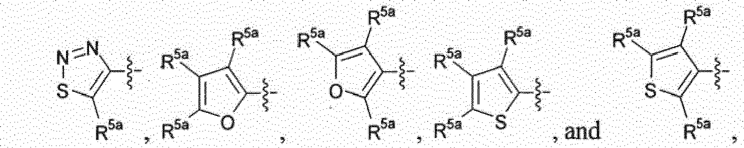 wherein 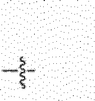 indicates the point of attachment of ring A₁ to the carbonyl carbon of the Formula I core structure; wherein:
R^{5a} at each occurrence is independently selected from the group consisting of hydrogen, and the options specified for R⁵ in claim 1.
R^{5b} is selected from the group consisting of hydrogen, and the options specified for R⁵ in claim 1.
R¹¹, R¹⁷, R¹⁸, R¹⁹, R²⁰, and m are as defined for Formula I in claim 1.

In some embodiments of a compound of Formula I, R^{5a} at each occurrence is independently selected from the group consisting of hydrogen, halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl; and R^{5b} is selected from the group consisting of hydrogen, C₁₋₆ alkyl, C₁₋₆ haloalkyl, C₂₋₆ alkenyl, C₂₋₆ haloalkenyl, C₂₋₆ alkynyl, and C₂₋₆ haloalkynyl. In some embodiments, R^{5a} at each occurrence is independently selected from the group consisting of hydrogen, halogen, C₁₋₆alkyl, C₁₋₆ haloalkyl, C₁₋₆ alkoxy, and C₁₋₆ haloalkoxy and R^{5b} is selected from the group consisting of hydrogen, C₁₋₆ alkyl, and C₁₋₆ haloalkyl. In some embodiments, one R^{5a} is selected from the group consisting of halogen, C₁₋₆ alkyl, C₁₋₆ haloalkyl, C₁₋₆ alkoxy, and C₁₋₆ haloalkoxy, any other R^{5a} is selected from the group consisting of hydrogen, halogen, C₁₋₆ halkyl, C₁₋₆ haloalkyl, C₁₋₆ alkoxy, and C₁₋₆ haloalkoxy, and R^{5b} is selected from the group consisting of hydrogen, C₁₋₆ alkyl, and C₁₋₆ haloalkyl.

In some embodiments of a compound of Formula I, A₁ is selected from the group consisting of: 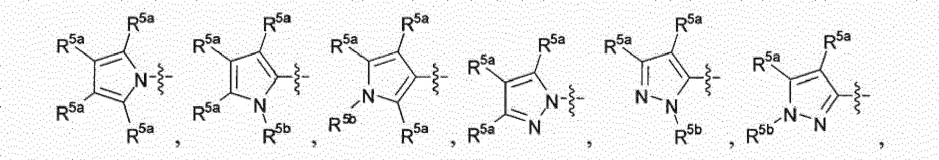 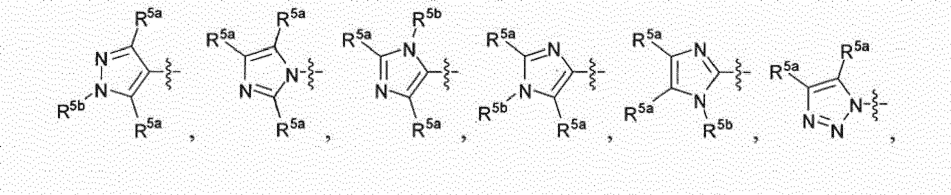 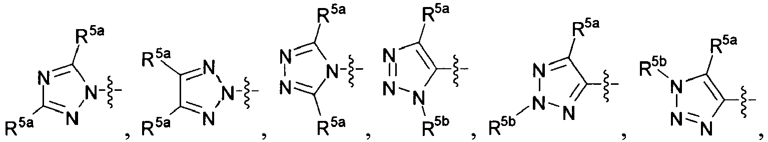 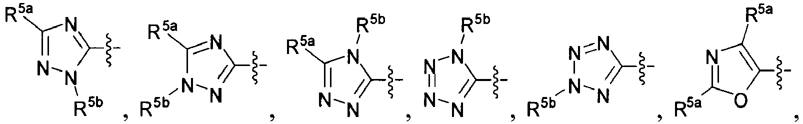 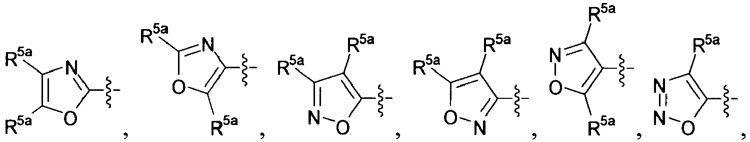 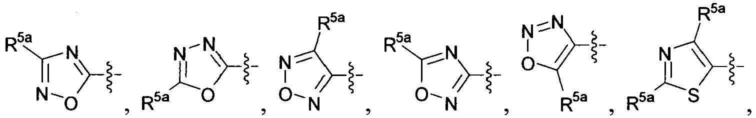 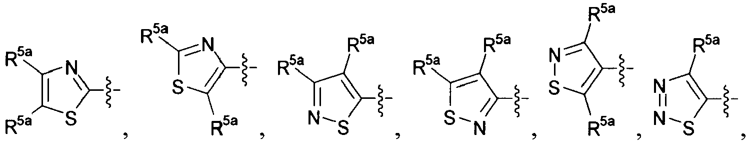 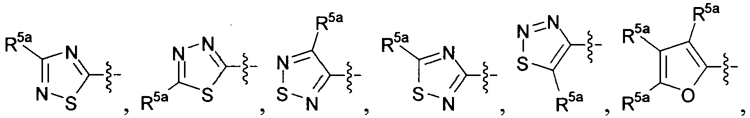 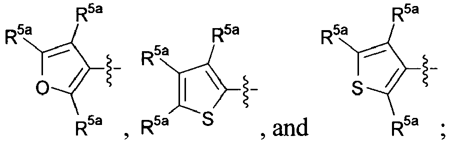 R^{5a} at each occurrence is independently selected from the group consisting of hydrogen, halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl; and R^{5b} is selected from the group consisting of hydrogen, C₁₋₆ alkyl, C₁₋₆ haloalkyl, C₂₋₆ alkenyl, C₂₋₆ haloalkenyl, C₂₋₆ alkynyl, and C₂₋₆ haloalkynyl. In some embodiments, R^{5a} at each occurrence is independently selected from the group consisting of hydrogen, halogen, C₁₋₆ alkyl, C₁₋₆ haloalkyl, C₁₋₆ alkoxy, and C₁₋₆ haloalkoxy and R^{5b} is selected from the group consisting of hydrogen, C₁₋₆ alkyl, and C₁₋₆ haloalkyl. In some embodiments, one R^{5a} is selected from the group consisting of halogen, C₁₋₆ alkyl, C₁₋₆ haloalkyl, C₁₋₆ alkoxy, and C₁₋₆ haloalkoxy, any other R^{5a} is selected from the group consisting of hydrogen, halogen, C₁₋₆ alkyl, C₁₋₆ haloalkyl, C₁₋₆ alkoxy, and C₁₋₆ haloalkoxy, and R^{5b} is selected from the group consisting of hydrogen, C₁₋₆ alkyl, and C₁₋₆ haloalkyl.

In some embodiments of a compound of Formula I, A₁ is selected from the group consisting of: 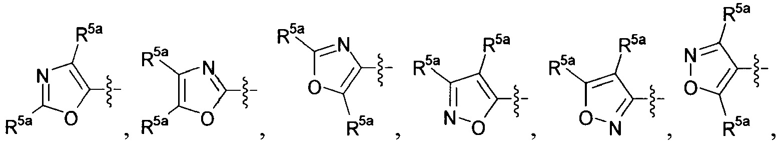 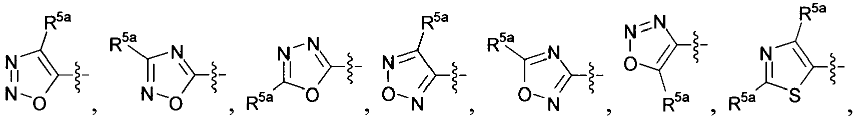 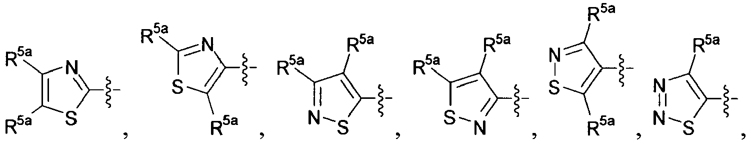 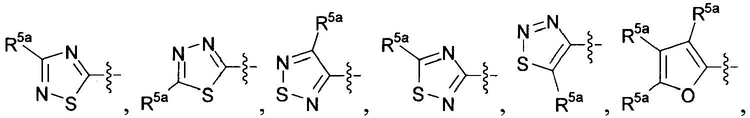 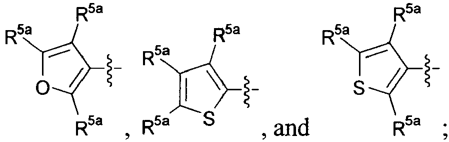 and R^{5a} at each occurrence is independently selected from the group consisting of hydrogen, halogen, C₁₋₆ alkyl, C₁₋₆haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl. In some embodiments, R^{5a} at each occurrence is independently selected from the group consisting of hydrogen, halogen, C₁₋₆ alkyl, C₁₋₆ haloalkyl, C₁₋₆ alkoxy, and C₁₋₆ haloalkoxy. In some embodiments, one R^{5a} is selected from the group consisting of halogen, C₁₋₆ alkyl, C₁₋₆ haloalkyl, C₁₋₆ alkoxy, and C₁₋₆ haloalkoxy, and any other R^{5a} is selected from the group consisting of hydrogen, halogen, C₁₋₆ alkyl, C₁₋₆ haloalkyl, C₁₋₆ alkoxy, and C₁₋₆ haloalkoxy. In some embodiments, one R^{5a} is selected from the group consisting of halogen, C₁₋₆ alkyl, C₁₋₆ haloalkyl, C₁₋₆ alkoxy, and C₁₋₆ haloalkoxy, and any other R^{5a} is hydrogen.

In some embodiments of a compound of Formula I, A₁ is selected from the group consisting of: 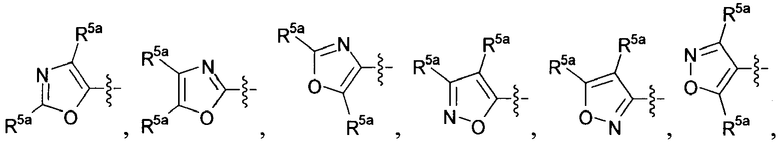 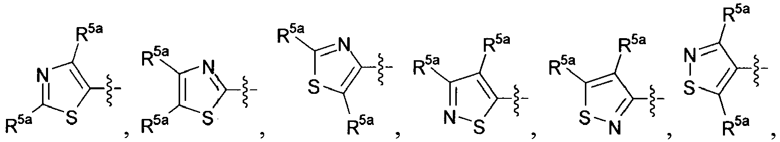 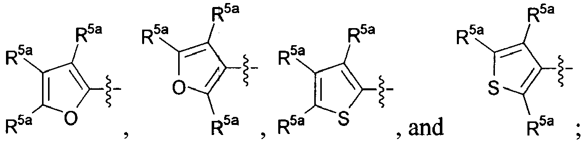 and R^{5a} at each occurrence is independently selected from the group consisting of hydrogen, halogen, C₁-₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl. In some embodiments, R^{5a} at each occurrence is independently selected from the group consisting of hydrogen, halogen, C₁₋₆ alkyl, C₁₋₆ haloalkyl, C₁₋₆ alkoxy, and C₁₋₆ haloalkoxy. In some embodiments, one R^{5a} is selected from the group consisting of halogen, C₁₋₆ alkyl, C₁₋₆ haloalkyl, C₁₋₆ alkoxy, and C₁₋₆ haloalkoxy, and any other R^{5a} is selected from the group consisting of hydrogen, halogen, C₁₋₆ alkyl, C₁₋₆ haloalkyl, C₁₋₆ alkoxy, and C₁₋₆ haloalkoxy. In some embodiments, one R^{5a} is selected from the group consisting of halogen, C₁₋₆ alkyl, C₁₋₆ haloalkyl, C₁₋₆ alkoxy, and C₁₋₆ haloalkoxy, and any other R^{5a} is hydrogen.

In some embodiments of a compound of Formula I, A₁ is selected from the group consisting of: 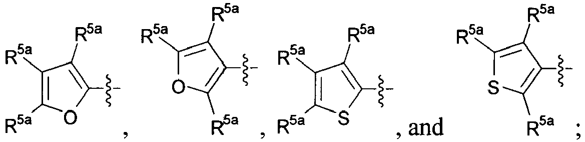 and R^{5a} at each occurrence is independently selected from the group consisting of hydrogen, halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl. In some embodiments, A₁ is selected from the group consisting of: 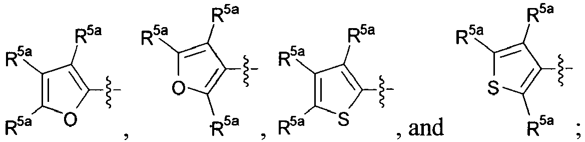 and R^{5a} at each occurrence is independently selected from the group consisting of hydrogen, halogen, C₁₋₆ alkyl, C₁₋₆ haloalkyl, C₁₋₆ alkoxy, and C₁₋₆ haloalkoxy. In some embodiments, A₁ is selected from the group consisting of: 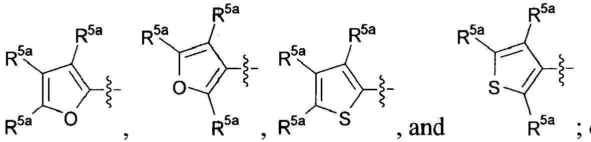 one R^{5a} is selected from the group consisting of halogen, C₁₋₆ alkyl, C₁₋₆ haloalkyl, C₁₋₆ alkoxy, and C₁₋₆ haloalkoxy, and the other two R^{5a} are selected from the group consisting of hydrogen, halogen, C₁₋₆ alkyl, C₁₋₆ haloalkyl, C₁₋₆ alkoxy, and C₁₋₆ haloalkoxy. In some embodiments, A₁ is selected from the group consisting of: 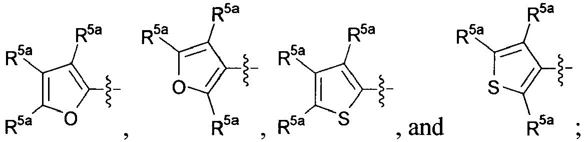 one R^{5a} is selected from the group consisting of halogen, C₁₋₆ alkyl, C₁₋₆ haloalkyl, C₁₋₆ alkoxy, and C₁₋₆ haloalkoxy, and the other two R^{5a} are hydrogen.

In some embodiments of a compound of Formula I, further to any of the above embodiments of Formula I, R¹ is C₁₋₆ alkyl, C₂₋₆ alkenyl, C₂₋₆ alkynyl, phenyl, or 5 or 6 membered heteroaryl, wherein C₁₋₆ alkyl, C₂₋₆ alkenyl, and C₂₋₆ alkynyl are optionally substituted with one or more substituents selected from the group consisting of halogen, phenyl, and 5 or 6 membered heteroaryl, and wherein phenyl and 5 or 6 membered heteroaryl (whether as R¹, or as substituents of C₁₋₆ alkyl, C₂₋₆ alkenyl, or C₂₋₆ alkynyl) are optionally substituted with 1 or 2 substituents independently selected from the group consisting of halogen, -CN, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl. In some embodiments, R¹ is C₁₋₆ alkyl, C₁₋₆ haloalkyl, C₂₋₆ alkenyl, C₂₋₆ haloalkenyl, C₂₋₆ alkynyl, C₂₋₆ haloalkynyl, or -(CH₂)₀₋₁-phenyl, wherein phenyl is optionally substituted with 1 or 2 substituents independently selected from the group consisting of halogen, -CN, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl. In some embodiments, R¹ is -(CH₂)₀₋₁-phenyl, wherein phenyl is optionally substituted with 1 or 2 substituents independently selected from the group consisting of halogen
n, -CN, C₁₋₆ alkyl, C₁₋₆ haloalkyl, C₁₋₆ alkoxy, C₁₋₆ haloalkoxy, C₁₋₆ alkylsulfonyl, C₁₋₆ haloalkylsulfonyl, C₁₋₆ alkylamino, C₁₋₆ dialkylamino, and N-linked-heterocycloalkyl. In some embodiments, R¹ is -(CH₂)₀₋₁-phenyl, wherein phenyl is optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy.

In some embodiments of a compound of Formula I, further to any of the above embodiments of Formula I, R² and R³ are independently selected from the group consisting of hydrogen, halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₃₋₆ cycloalkyl, and 3-6 membered heterocycloalkyl; or R² and R³ attached to the same carbon combine with the carbon to which they are attached to form C₃₋₆ cycloalkyl or 3-6 membered heterocycloalkyl. In some embodiments, R² and R³ are independently selected from the group consisting of hydrogen, halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, and C₁₋₆ haloalkoxy. In some embodiments, R² and R³ are independently selected from the group consisting of hydrogen, halogen, and C₁₋₆ alkyl.

In some embodiments of a compound of Formula I, further to any of the above embodiments of Formula I, R⁴ is hydrogen; n is 1; X is S; and Y is -C(O)-.

In one aspect, compounds of Formula I are provided having a structure according to Formula Ia: 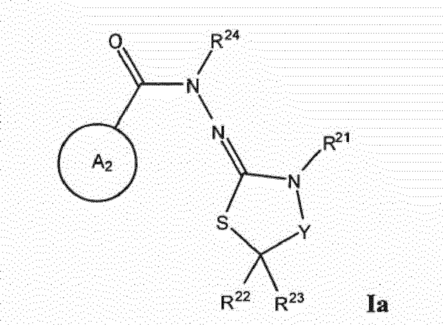 or a salt thereof, wherein:
A₂ is selected from the group consisting of: 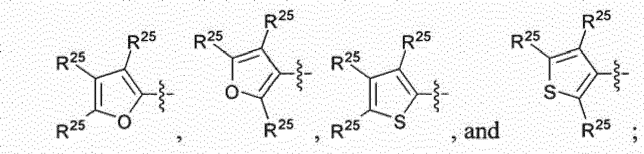 wherein 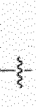 indicates the point of attachment of ring A₂ to the carbonyl carbon of the Formula Ia core structure;
Y is -C(O)- or -CH₂-;
R²¹ is -(CH₂)₀₋₁-phenyl, wherein phenyl is optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy;
R²² and R²³ are independently hydrogen or C₁₋₃ alkyl;
R²⁴ is hydrogen or C₁₋₃ alkyl; and
R²⁵ at each occurrence is independently selected from the group consisting of hydrogen, halogen, C₁₋₆ alkyl, C₁₋₆ haloalkyl, C₁₋₆ alkoxy, and C₁₋₆ haloalkoxy;

In some embodiments of a compound of Formula Ia, one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen.

In some embodiments of a compound of Formula Ia, one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 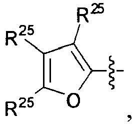 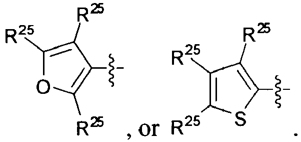 In some embodiments, ring A₁ is 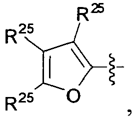 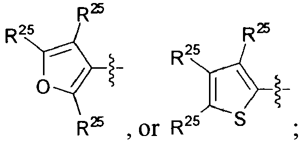 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula Ia, ring A₁ is 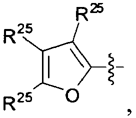 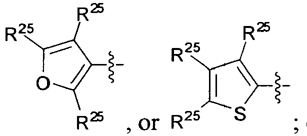 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 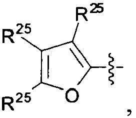 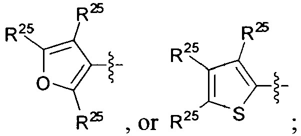 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 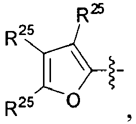 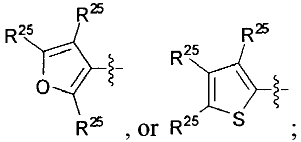 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 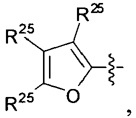 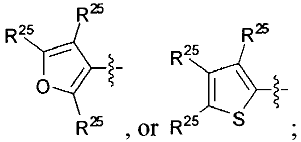 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 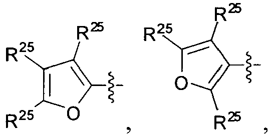 or 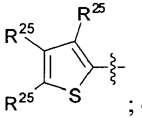 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 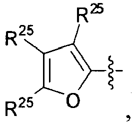 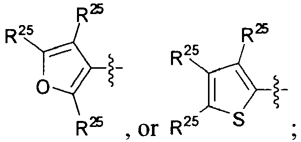 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 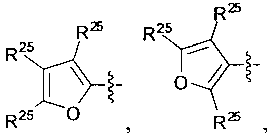 or 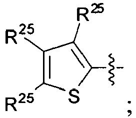 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 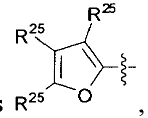 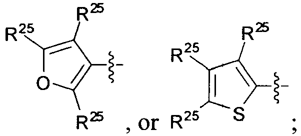 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 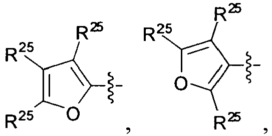 or 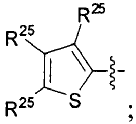 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 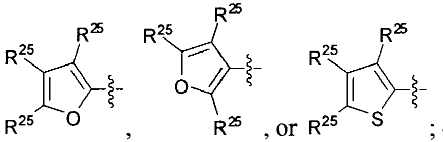 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 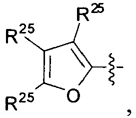 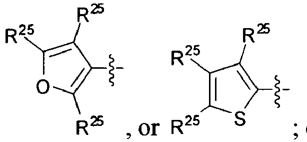 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 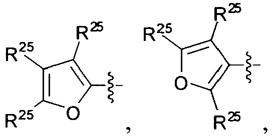 or 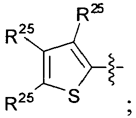 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 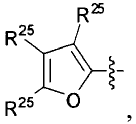 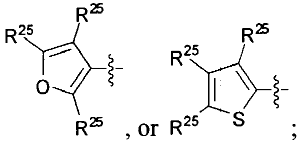 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 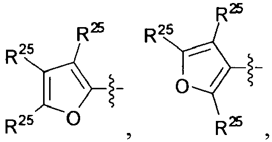 or 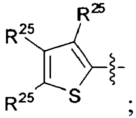 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 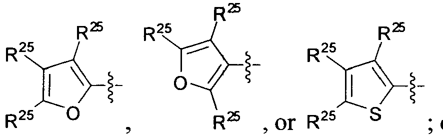 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 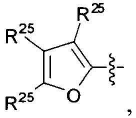 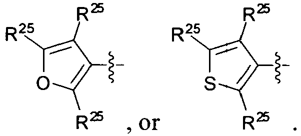 In some embodiments, ring A₁ is 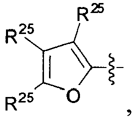 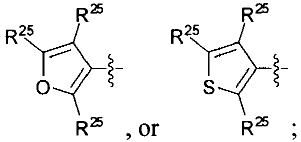 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula Ia, ring A₁ is 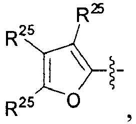 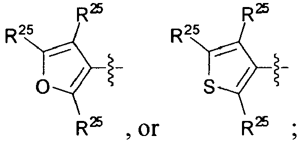 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 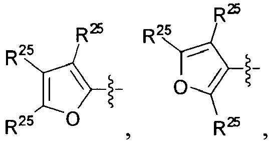 or 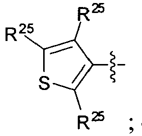 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 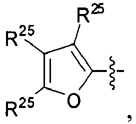 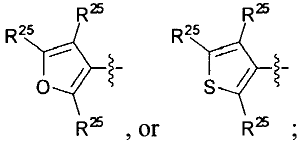 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 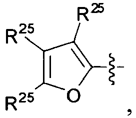 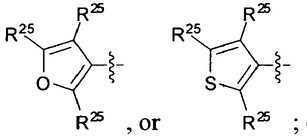 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 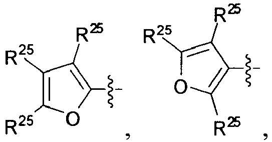 or 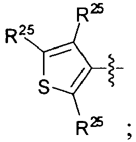 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 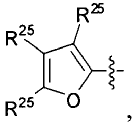 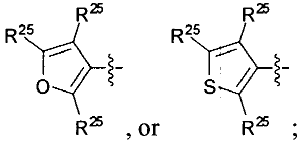 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 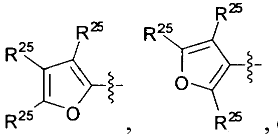 or 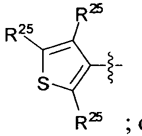 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 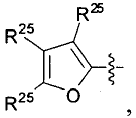 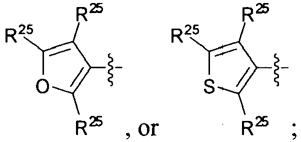 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 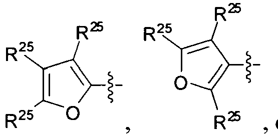 or 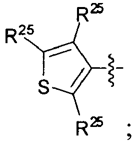 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 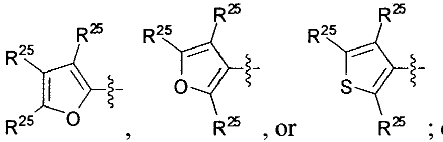 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 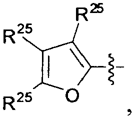 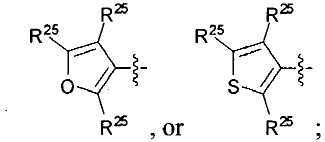 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 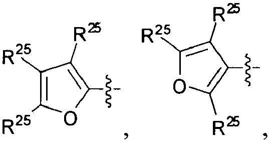 or 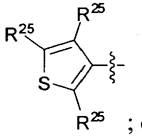 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 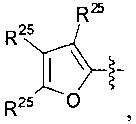 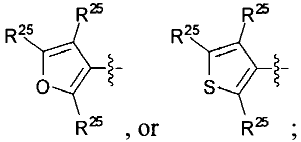 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 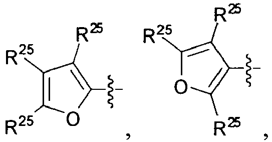 or 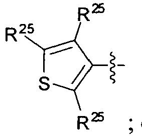 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 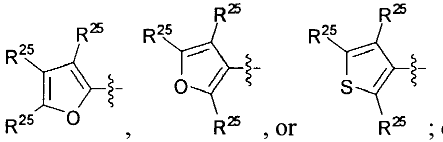 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 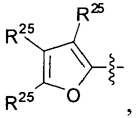 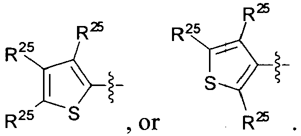 In some embodiments, ring A₁ is 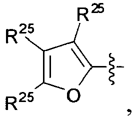 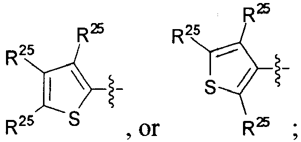 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula Ia, ring A₁ is 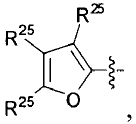 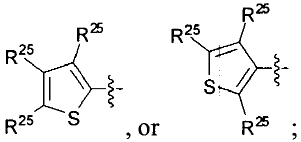 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 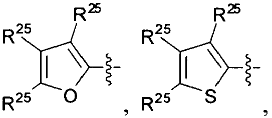 or 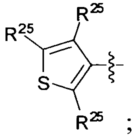 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 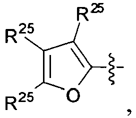 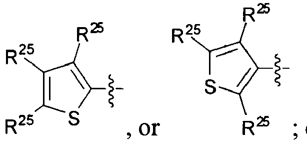 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 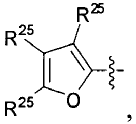 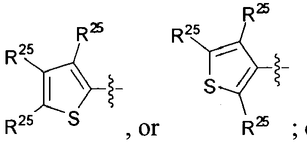 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 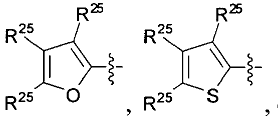 or 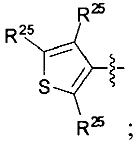 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 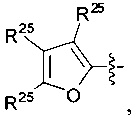 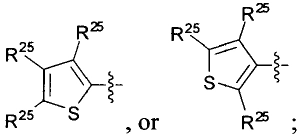 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 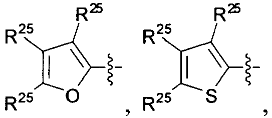 or 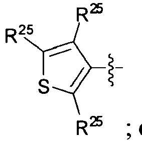 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 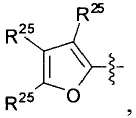 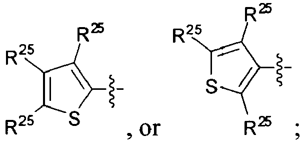 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 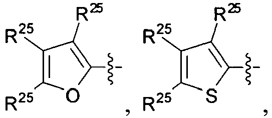 or 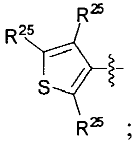 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 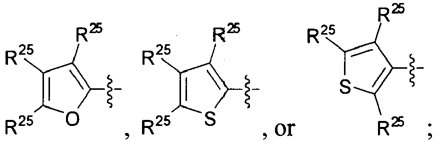 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 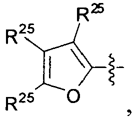 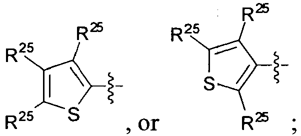 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 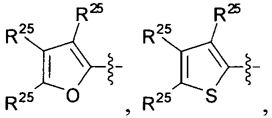 or 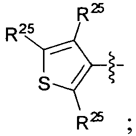 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 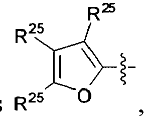 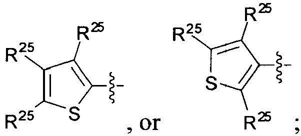 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 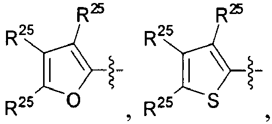 or 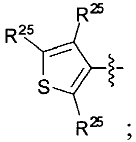 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 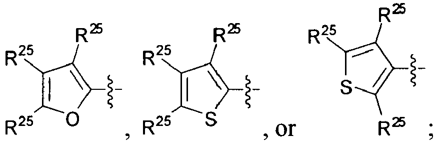 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 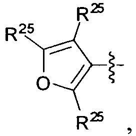 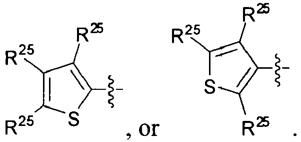 In some embodiments, ring A₁ is 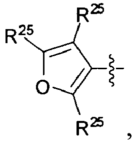 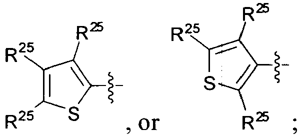 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula Ia, ring A₁ is 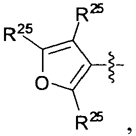 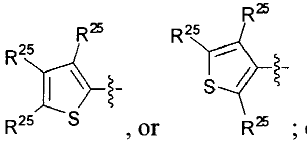 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 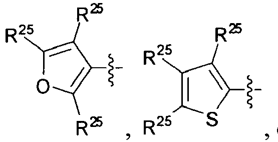 or 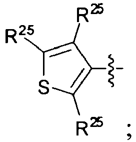 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 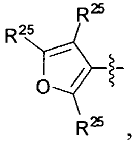 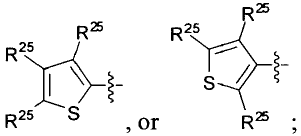 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 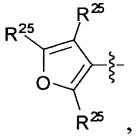 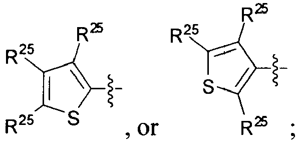 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 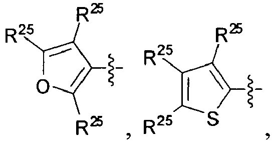 or 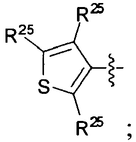 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 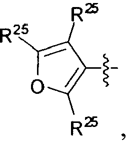 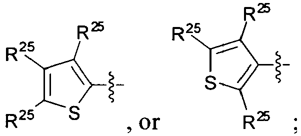 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 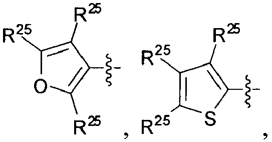 or 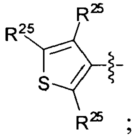 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 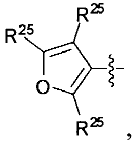 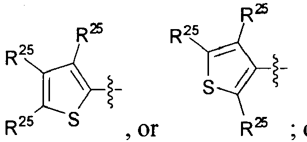 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 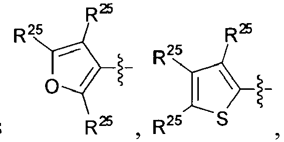 or 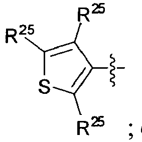 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 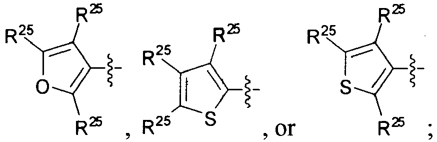 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 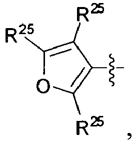 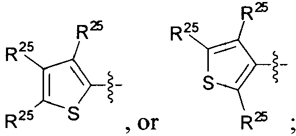 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 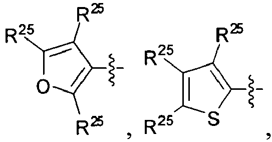 or 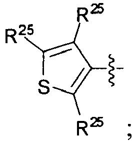 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 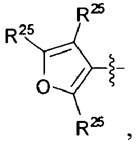 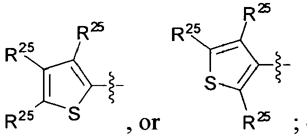 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 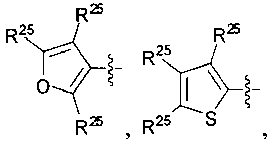 or 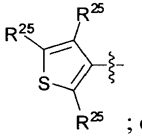 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 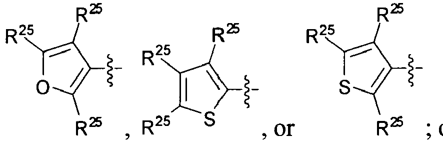 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 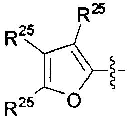 or 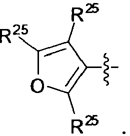 In some embodiments, ring A₁ is 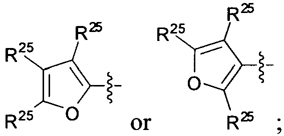 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula Ia, ring A₁ is 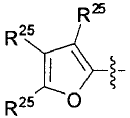 or 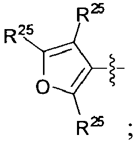 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 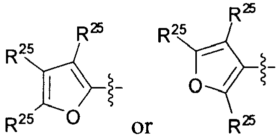 ; one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 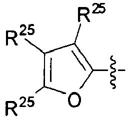 or 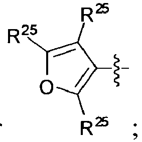 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 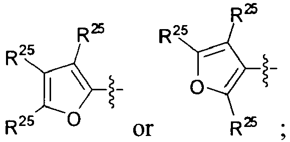 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 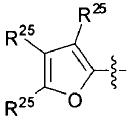 or 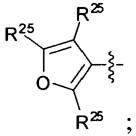 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 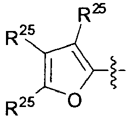 or 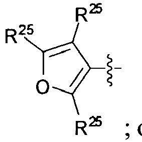 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 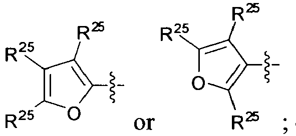 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 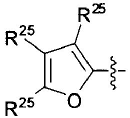 or 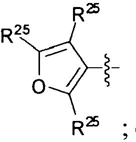 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 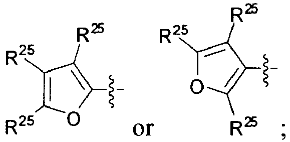 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 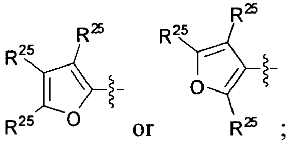 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 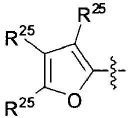 or 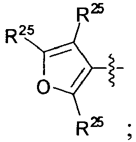 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 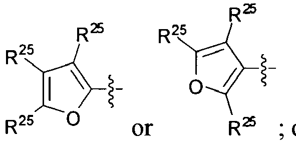 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 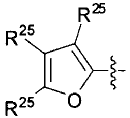 or 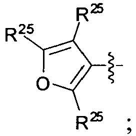 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 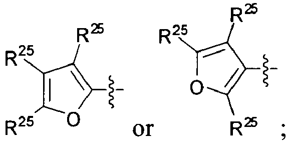 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 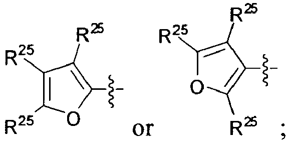 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 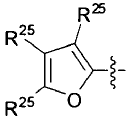 or 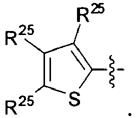 In some embodiments, ring A₁ is 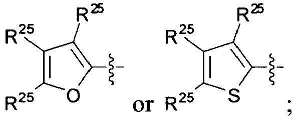 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula Ia, ring A₁ is 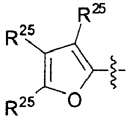 or 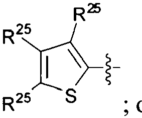 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 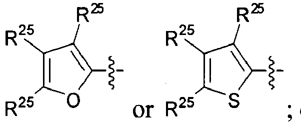 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 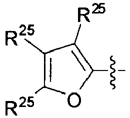 or 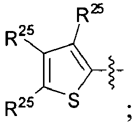 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 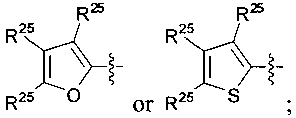 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 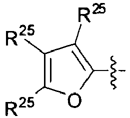 or 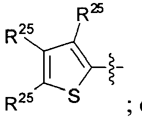 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 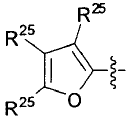 or 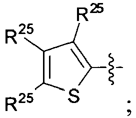 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 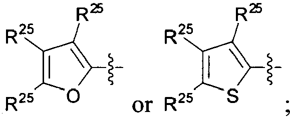 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 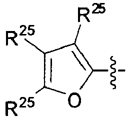 or 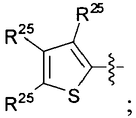 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 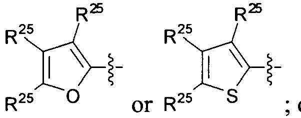 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 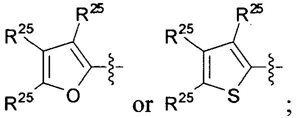 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 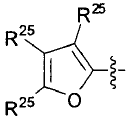 or 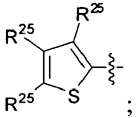 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 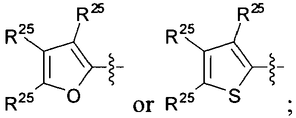 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 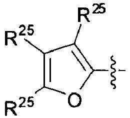 or 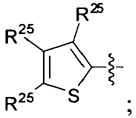 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 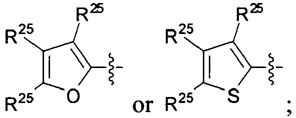 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 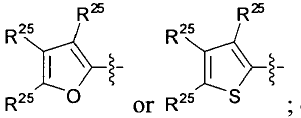 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 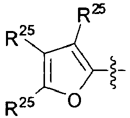 or 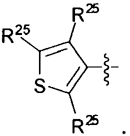 In some embodiments, ring A₁ is 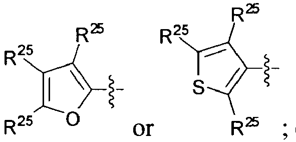 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula Ia, ring A₁ is 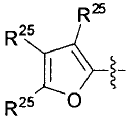 or 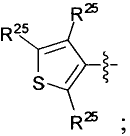 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 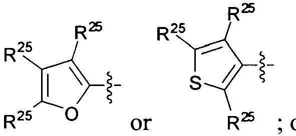 one R²⁵ is selected from the group consisting of halogen, C₁₋₆ alkyl, C₁₋₆ haloalkyl, C₁₋₆ alkoxy, and C₁₋₆haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 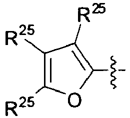 or 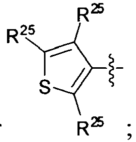 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 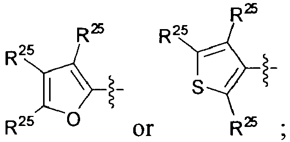 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 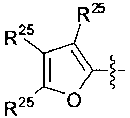 or 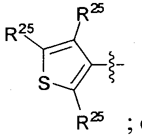 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 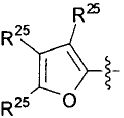 or 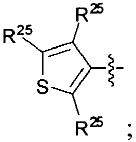 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 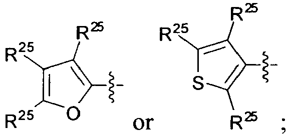 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 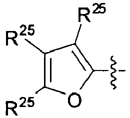 or 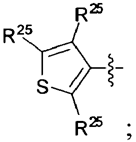 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 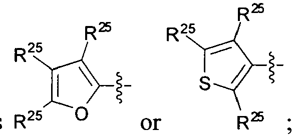 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 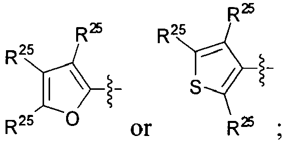 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 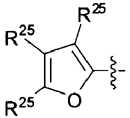 or 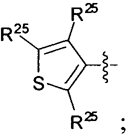 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 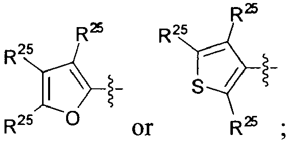 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 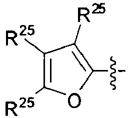 or 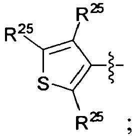 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 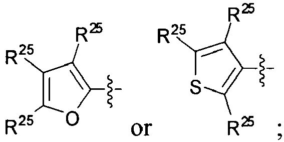 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 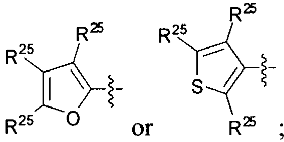 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 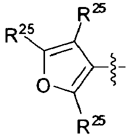 or 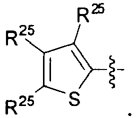 In some embodiments, ring A₁ is 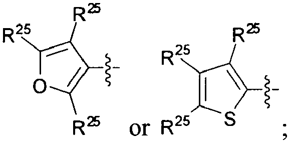 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula Ia, ring A₁ is 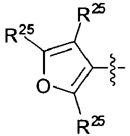 or 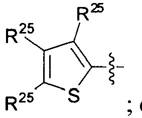 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 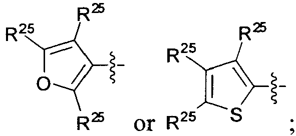 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 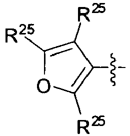 or 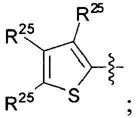 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 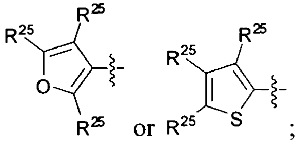 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 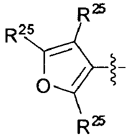 or 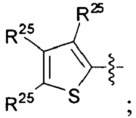 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 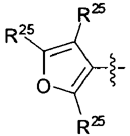 or 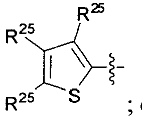 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 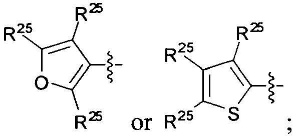 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 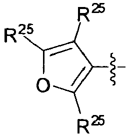 or 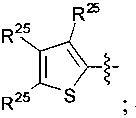 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 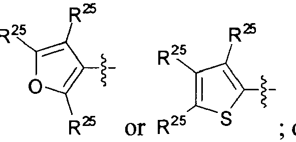 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 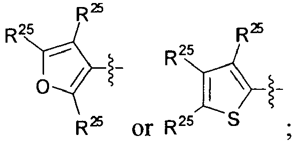 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 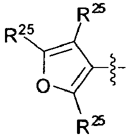 or 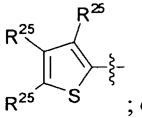 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 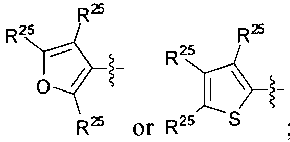 ; one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 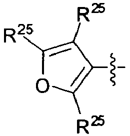 or 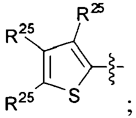 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆alkoxy, and C₁₋₆haloalkoxy; R²² and R²³ are independently hydrogen or C₁₋₃ alkyl; and R²⁴ is hydrogen. In some embodiments, ring A₁ is 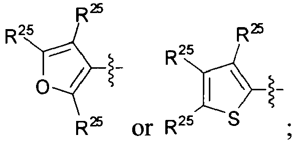 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 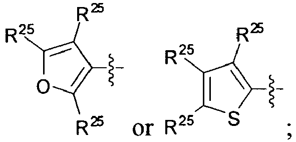 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 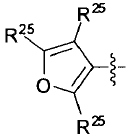 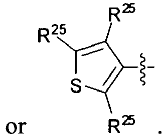 In some embodiments, ring A₁ is 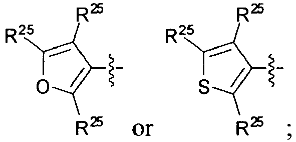 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula la, ring A₁ is 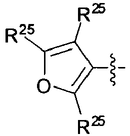 or 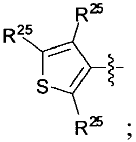 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 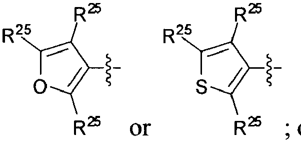 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 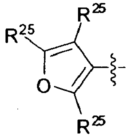 or 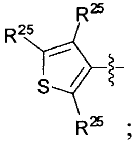 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 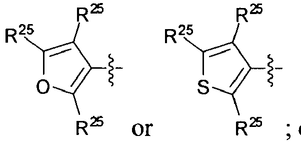 one R²⁵ is selected from the group consisting of halogen, C₁₋₆ alkyl, C₁₋₆ haloalkyl, C₁₋₆ alkoxy, and C₁₋₆haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 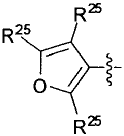 or 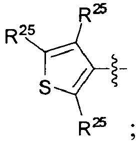 one R²⁵ is selected from the group consisting of halogen, C₁₋₆ alkyl, C₁₋₆ haloalkyl, C₁₋₆ alkoxy, and C₁₋₆haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 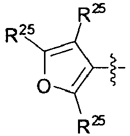 or 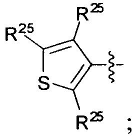 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 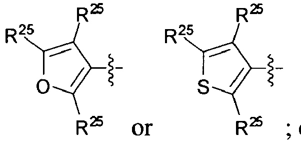 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 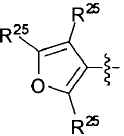 or 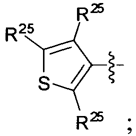 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 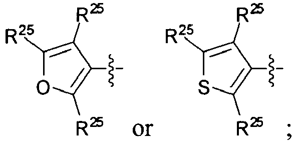 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 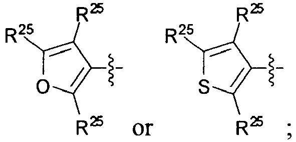 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 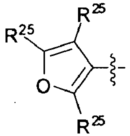 or 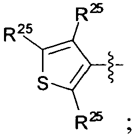 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 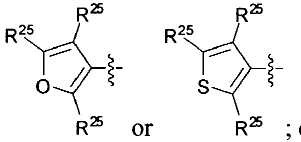 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 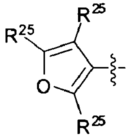 or 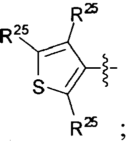 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆haloalkoxy; R²² and R²³ are independently hydrogen or C₁₋₃ alkyl; and R²⁴ is hydrogen. In some embodiments, ring A₁ is 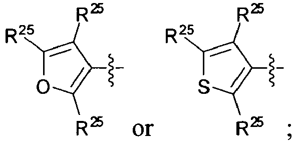 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 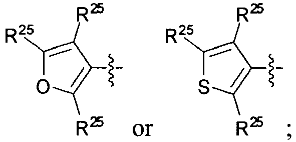 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 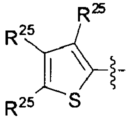 or 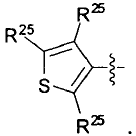 In some embodiments, ring A₁ is 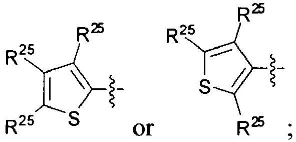 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula Ia, ring A₁ is 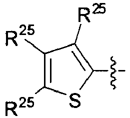 or 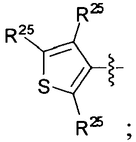 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 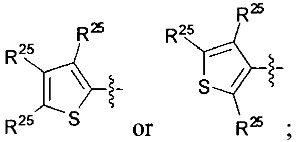 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 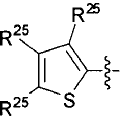 or 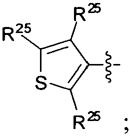 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 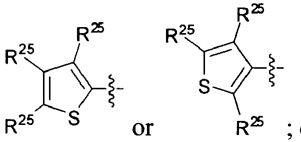 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 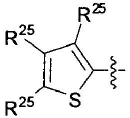 or 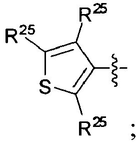 one R²⁵ is selected from the group consisting of halogen, C₁₋₆ alkyl, C₁₋₆ haloalkyl, C₁₋₆ alkoxy, and C₁₋₆haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 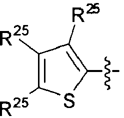 or 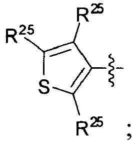 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 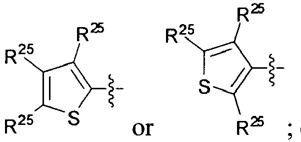 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 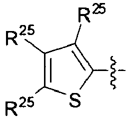 or 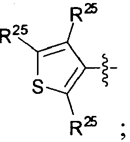 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 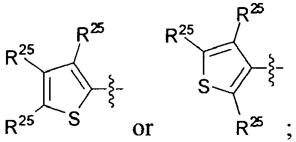 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 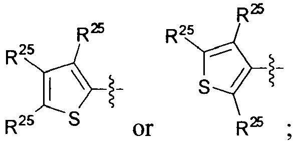 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 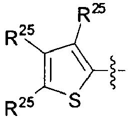 or 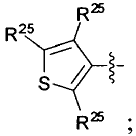 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 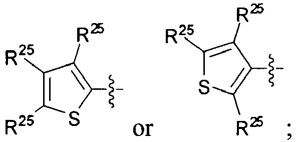 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 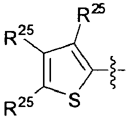 or 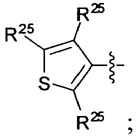 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 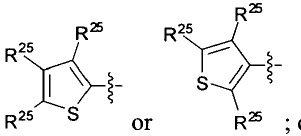 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 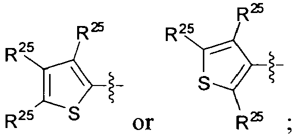 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 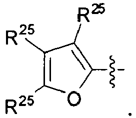 In some embodiments, ring A₁ is 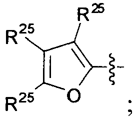 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula la, ring A₁ is 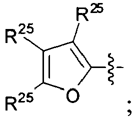 one R²⁵ is selected from the group consisting of halogen, C₁₋₆ alkyl, C₁₋₆ haloalkyl, C₁₋₆alkoxy, and C₁₋₆haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 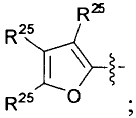 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 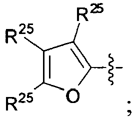 one R²⁵ is selected from the group consisting of halogen, C₁₋₆ alkyl, C₁₋₆ haloalkyl, C₁₋₆alkoxy, and C₁₋₆haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 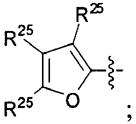 one R²⁵ is selected from the group consisting of halogen, C₁₋₆alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 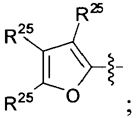 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 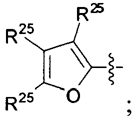 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 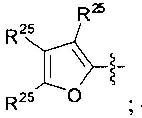 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 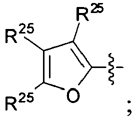 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 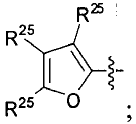 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 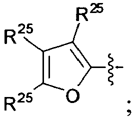 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 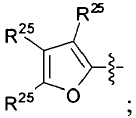 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 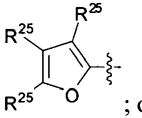 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 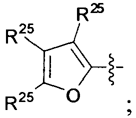 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 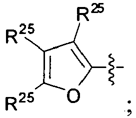 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 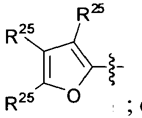 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 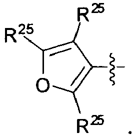 In some embodiments, ring A₁ is 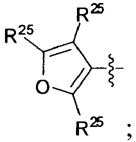 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula la, ring A₁ is 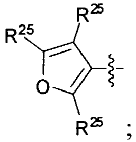 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 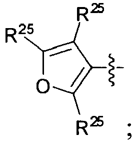 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 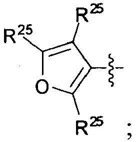 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 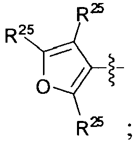 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 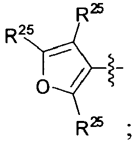 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 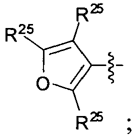 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 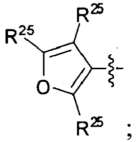 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 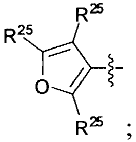 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 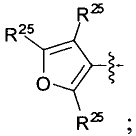 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 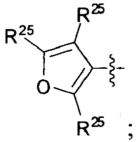 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 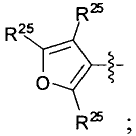 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 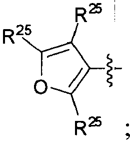 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 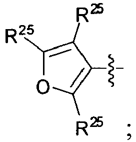 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 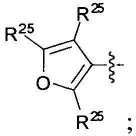 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 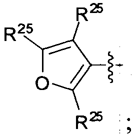 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 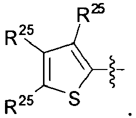 In some embodiments, ring A₁ is 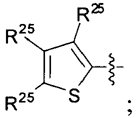 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula Ia, ring A₁ is 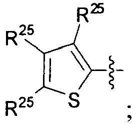 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 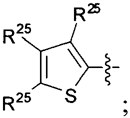 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 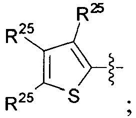 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 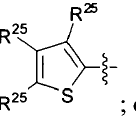 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 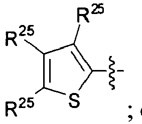 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 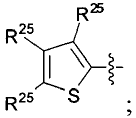 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 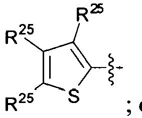 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 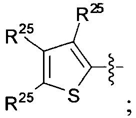 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 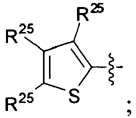 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 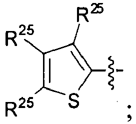 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 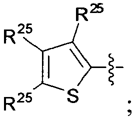 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 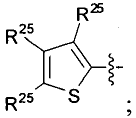 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 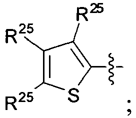 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 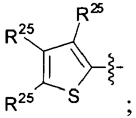 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 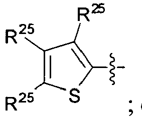 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 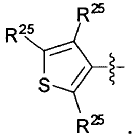 In some embodiments, ring A₁ is 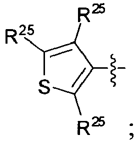 one R²⁵ is selected from the group consisting of halogen, C₁₋₆ alkyl, C₁₋₆ haloalkyl, C₁₋₆ alkoxy, and C₁₋₆ haloalkoxy; and the other two R²⁵ are hydrogen; also one R²⁵ is halogen; and the other two R²⁵ are hydrogen; also one R²⁵ is chloro; and the other two R²⁵ are hydrogen.

In some embodiments of a compound of Formula la, ring A₁ is 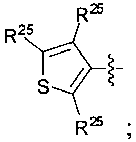 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 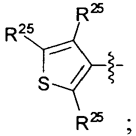 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula Ia, ring A₁ is 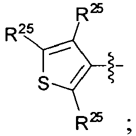 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 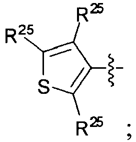 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 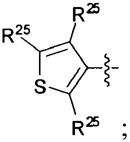 one R²⁵ is selected from the group consisting of halogen, C₁₋₆ alkyl, C₁₋₆ haloalkyl, C₁₋₆ alkoxy, and C₁₋₆ haloalkoxy; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 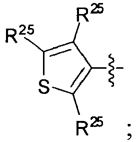 one R²⁵ is halogen; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 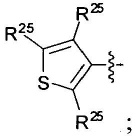 one R²⁵ is halogen; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 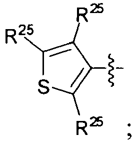 one R²⁵ is halogen; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 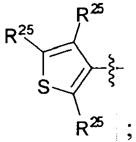 one R²⁵ is halogen; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 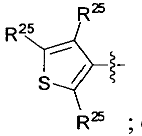 one R²⁵ is halogen; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 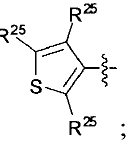 one R²⁵ is chloro; the other two R²⁵ are hydrogen; R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 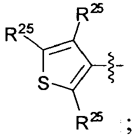 one R²⁵ is chloro; the other two R²⁵ are hydrogen; R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen.

In some embodiments of a compound of Formula la, ring A₁ is 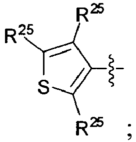 one R²⁵ is chloro; the other two R²⁵ are hydrogen; R²¹ is phenyl optionally substituted with 1 or 2 substituents independently selected from the group consisting of halogen, C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 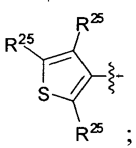 one R²⁵ is chloro; the other two R²⁵ are hydrogen; R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 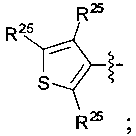 one R²⁵ is chloro; the other two R²⁵ are hydrogen; R²¹ is phenyl optionally substituted with 1 or 2 fluoro; R²² and R²³ are independently hydrogen or C₁₋₃ alkyl; and R²⁴ is hydrogen.

In some embodiments of a compound of Formula la, ring A₁ is 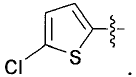 In some embodiments, ring A₁ is 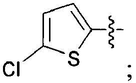 R²¹ is -(CH₂)₀₋₁-phenyl, wherein phenyl is optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 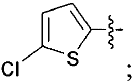 R²¹ is -(CH₂)₀₋₁-phenyl, wherein phenyl is optionally substituted with 1 or 2 fluoro; R²² and R²³ are independently hydrogen or C₁₋₃ alkyl; and R²⁴ is hydrogen. In some embodiments, ring A₁ is 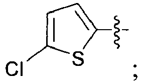 R²¹ is phenyl optionally substituted with 1 or 2 substituents independently selected from the group consisting of halogen; C₁₋₆ alkyl, C₁₋₆ haloalkyl, C₁₋₆ alkoxy, and C₁₋₆ haloalkoxy; R²² and R²³ are independently hydrogen or C₁₋₃ alkyl; and R²⁴ is hydrogen. In some embodiments, ring A₁ is 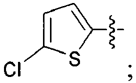 R²¹ is phenyl optionally substituted with 1 or 2 substituents independently selected from the group consisting of fluoro, methoxy, and trifluoromethyl; R²² and R²³ are independently hydrogen or C₁₋₃ alkyl; and R²⁴ is hydrogen. In some embodiments, ring A₁ is 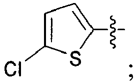 R²¹ is phenyl optionally substituted with 1 or 2 fluoro; R²² and R²³ are independently hydrogen or C₁₋₃ alkyl; and R²⁴ is hydrogen.

In some embodiments of a compound of Formula Ia, further to any of the above embodiments of a compound of Formula la, Y is -C(O)-. In some embodiments, Y is -CH₂-.

In some embodiments, the compound is any one or more compounds, or a salt thereof, as described in the Examples herein. In one embodiment the compound is any one or more compounds, or a salt thereof, selected from the group consisting of:
5-chloro-N'-(4-oxo-3-phenylthiazolidin-2-ylidene)thiophene-2-carbohydrazide,
5-chloro-N'-(3-(2,4-difluorophenyl)-4-oxothiazolidin-2-ylidene)thiophene-2-carbohydrazide,
5-chloro-N'-(3-(2-fluorophenyl)-4-oxothiazolidin-2-ylidene)thiophene-2-carbohydrazide,
5-chloro-N'-(3-(4-fluorophenyl)-4-oxothiazolidin-2-ylidene)thiophene-2-carbohydrazide,
5-chloro-N'-(3-(3-fluorophenyl)-4-oxothiazolidin-2-ylidene)thiophene-2-carbohydrazide,
5-chloro-N'-(5-methyl-4-oxo-3-phenylthiazolidin-2-ylidene)thiophene-2-carbohydrazide,
N'-(3-benzyl-4-oxothiazolidin-2-ylidene)-5-chlorothiophene-2-carbohydrazide,
5-chloro-N'-(3-phenylthiazolidin-2-ylidene)thiophene-2-carbohydrazide,
5-chloro-N'-(4-oxo-3-(3-(trifluoromethyl)phenyl)thiazolidin-2-ylidene)thiophene-2-carbohydrazide,
5-chloro-N'-(5-isopropyl-4-oxo-3-phenylthiazolidin-2-ylidene)thiophene-2-carbohydrazide,
5-chloro-N'-(5,5-dimethyl-4-oxo-3-phenylthiazolidin-2-ylidene)thiophene-2-carbohydrazide, and
5-chloro-N'-(3-(2-methoxyphenyl)-4-oxothiazolidin-2-ylidene)thiophene-2-carbohydrazide.

Exemplary compounds as described herein, e.g. compounds of Formula I, and their *in vitro* biological activities are listed in the table of Example A.

In some embodiments, the compounds as described herein, e.g. compounds of Formula I, are inhibitors of LRRK2 kinase activity, including LRRK2 mutant kinase activity, also LRRK2 mutant G2019S kinase activity, with an IC₅₀ of less than about 50 µM, less than about 40 µM, less than about 30 µM, less than about 20 µM or less than about 10 µM. In one embodiment, the compounds of Formula I exhibit inhibitory activity against LRRK2 kinase activity, including LRRK2 mutant kinase activity, also LRRK2 mutant G2019S kinase activity,with an IC₅₀ of less than about 9 µM, less than about 8 µM, less than about 7 µM, less than about 6 µM, less than about 5 µM, less than about 4 µM, less than about 3 µM, less than about 2 µM, or less than about 1 µM. In one embodiment, the compounds of Formula I exhibit inhibitory activity against LRRK2 kinase activity, including LRRK2 mutant kinase activity, also LRRK2 mutant G2019S kinase activity, with an IC₅₀ of less than about 0.9 µM, less than about 0.8 µM, less than about 0.7 µM, less than about 0.6 µM, less than about 0.5 µM, less than about 0.4 µM, less than about 0.3 µM, less than about 0.2 µM. In one embodiment, the compounds of Formula I exhibit inhibitory activity against LRRK2 kinase activity, including LRRK2 mutant kinase activity, also LRRK2 mutant G2019S kinase activity, with an IC₅₀ of less than about 0.1 µM (100 nM). In one embodiment, the compounds of Formula I exhibit inhibitory activity against LRRK2 kinase, inlcuding LRRK2 kinase mutant kinase activity, also LRRK2 mutant G2019S kinase activity, with an IC₅₀ of less than about 90 nM, less than about 80 nM, less than about 70 nM, less than about 60 nM, less than about 50 nM, less than about 40 nM, less than about 30 nM or less than about 20 nM. In one embodiment, the compounds of Formula I exhibit inhibitory activity against LRRK2 kinase activity, including LRRK2 mutant kinase activity, also LRRK2 mutant G2019S kinase activity, with an IC₅₀ of less than about 10 nM.

In some embodiments, compounds as described herein, e.g. compounds of Formula I, inhibit LRRK2 kinase activity, including LRRK2 mutant kinase activity, also LRRK2 mutant G2019S kinase activity, and are selective compared to LRRK1 kinase activity. In one embodiment, compounds of Formula I inhibit LRRK2 mutant G2019S kinase activity, and are selective compared to LRRK1 kinase activity and wild-type LRRK2 kinase activity. For the purpose of this application the selectivity of the instant compounds for LRRK2, including LRRK2 mutant G2019S, over LRRK1; or LRRK2 mutant G2019S over wild-type LRRK2 is expressed in a ratio of IC₅₀ values. Those can be determined using assays known in the art or those described herein (see e.g., Example A).

In some embodiments, compounds as described herein, e.g. compounds of Formula I, inhibit LRRK2 kinase activity, including LRRK2 mutant kinase activity, also LRRK2 mutant G2019S kinase activity, and are selective as compared to other kinases. Particularly, compounds of Formula I inhibit LRRK2 kinase activity, including LRRK2 mutant kinase activity, also LRRK2 mutant G2019S kinase activity, and are selective compared to one or more kinases selected from the group consisting of AKT1, AurA, BRAF, CAMK2A, CDK1, CDK2, CDK5, CLK1, FAK2, LCK, MARK2, MLK1, MST2/STK3, PKA, PKC beta, PLK1, PLK2, PLK3, and RET. In one embodiment, compounds are selective as compared to other kinases, such as one or more kinases selected from the group consisting of AKT1, AurA, BRAF, CAMK2A, CDK1, CDK2, CDK5, CLK1, FAK2, LCK, MARK2, MLK1, MST2/STK3, PKA, PKC beta, PLK1, PLK2, PLK3, and RET, and are selective compared to LRRK1 kinase activity. In some instances, compounds of Formula I inhibit LRRK2 mutant G2019S kinase activity, and are selective compared to one or more kinases selected from the group consisting of AKT1, AurA, BRAF, CAMK2A, CDK1, CDK2, CDK5, CLK1, FAK2, LCK, MARK2, MLK1, MST2/STK3, PKA, PKC beta, PLK1, PLK2, PLK3, and RET, and are selective compared to LRRK1 kinase activity and wild-type LRRK2 kinase activity. For the purpose of this application the selectivity of the instant compounds for the inhibition of LRRK2 kinase activity over other kinases is expressed in a ratio of IC₅₀ values, or in some instances as a ratio of % inhibition at a given concentration of compound, such as at 10 µM, which can be determined using assays known in the art or those described herein (see e.g., Example A).

In one aspect, any tautomer, stereoisomer, conjugate, polymorph, isotopically enhanced form, salt or pharmaceutically acceptable solvate of a compound of Formula I is provided. In one embodiment, any tautomer of a compound of Formula I is provided. In one embodiment, any stereoisomer of a compound of Formula I is provided. In one embodiment, any conjugate of a compound of Formula I is provided. In one embodiment, any polymorph of a compound of Formula I is provided. In one embodiment, any isotopically enhanced form of a compound of Formula I is provided. In one embodiment, any salt of a compound of Formula I is provided. In one embodiment, any pharmaceutically acceptable salt of a compound of Formula I is provided. In one embodiment, any polymorph of any salt of a compound of Formula I is provided. In one embodiment, any polymorph of any pharmaceutically acceptable salt of a compound of Formula I is provided. In one embodiment, any pharmaceutically acceptable solvate of a compound of Formula I is provided. In one embodiment, any polymorph of any pharmaceutically acceptable solvate of a compound of Formula I is provided.

In one aspect, a pharmaceutical composition comprising a compound as described herein, e.g. a compound of Formula I, and a pharmaceutically acceptable carrier is provided. In one embodiment, a pharmaceutical composition comprising any tautomer of a compound of Formula I and a pharmaceutically acceptable carrier is provided. In one embodiment, a pharmaceutical composition comprising any stereoisomer of a compound of Formula I and a pharmaceutically acceptable carrier is provided. In one embodiment, a pharmaceutical composition comprising any conjugate of a compound of Formula I and a pharmaceutically acceptable carrier is provided. In one embodiment, a pharmaceutical composition comprising any polymorph of a compound of Formula I and a pharmaceutically acceptable carrier is provided. In one embodiment, a pharmaceutical composition comprising any isotopically enhanced form of a compound of Formula I and a pharmaceutically acceptable carrier is provided. In one embodiment, a pharmaceutical composition comprising any pharmaceutically acceptable salt of a compound of Formula I and a pharmaceutically acceptable carrier is provided. In one embodiment, a pharmaceutical composition comprising any polymorph of any pharmaceutically acceptable salt of a compound of Formula I and a pharmaceutically acceptable carrier is provided. In one embodiment, a pharmaceutical composition comprising any pharmaceutically acceptable solvate of a compound of Formula I and a pharmaceutically acceptable carrier is provided. In one embodiment, a pharmaceutical composition comprising any polymorph of any pharmaceutically acceptable solvate of a compound of Formula I and a pharmaceutically acceptable carrier is provided.

In one aspect, a kit is provided that includes a compound or composition thereof as described herein, e.g. a compound of Formula I, or a salt or solvate thereof, or a composition comprising such a compound or salt or solvate thereof. In some embodiments, the compound or composition thereof is packaged, e.g., in a vial, bottle or similar container, which may be further packaged, e.g., within a box, envelope, or similar container. In some embodiments, the compound or composition thereof is approved by the U.S. Food and Drug Administration or similar regulatory agency for administration to a mammal, e.g., a human. In some embodiments the compound or composition thereof is approved for administration to a mammal, e.g., a human, for a LRRK2 mediated disease or condition, including a LRRK2 mutant mediated condition. In one embodiment, such a kit includes written instructions for use and/or other indication that the compound or composition is suitable or approved for administration to a mammal, e.g., a human, for a suitable disease or condition. In some embodiments, the compound or composition is packaged in unit dose or single dose form, e.g., single dose pills, capsules, or the like.

In an aspect of the invention, a compound according to Formula I or Formula Ia as defined herein, or a composition comprising such a compound or a salt thereof, is provided for use in treating a neurodegenerative disease in a mammalian subject (e.g. human).

In one embodiment the disease is selected from the group consisting of Parkinson's disease, Parkinson disease with dementia, Parkinson's disease at risk syndrome, dementia with Lewy bodies, Lewy body variant of Alzheimer's disease, combined Parkinson's disease and Alzheimer's disease, multiple system atrophy, striatonigral degeneration, olivopontocerebellar atrophy, Shy-Drager syndrome, Hallervorden-Spatz syndrome, fronto-temporal dementia, Sandhoff disease, progressive supranuclear palsy, corticobasal degeneration, postural hypotension, orthostatic hypotension, cerebellar dysfunctions, ataxia, movement disorders, cognitive deterioration, sleep disorders, hearing disorders, tremors, rigidity, bradykinesia, akinesia, postural instability, melanoma, acute myelogenous leukemia, breast carcinoma, lung adenocarincoma, prostate adenocarcinoma, renal cell carcinoma, papillary thyroid carcinoma, Crohn's disease, ulcerative colitis, and leprosy.

In one embodiment, the disease is a neurological disease and is selected from the group consisting of Parkinson's disease, Parkinson disease with dementia, Parkinson's disease at risk syndrome, dementia with Lewy bodies, Lewy body variant of Alzheimer's disease, combined Parkinson's disease and Alzheimer's disease, multiple system atrophy, striatonigral degeneration, olivopontocerebellar atrophy, Shy-Drager syndrome, Hallervorden-Spatz syndrome, fronto-temporal dementia, Sandhoff disease, progressive supranuclear palsy, corticobasal degeneration, postural hypotension, orthostatic hypotension, cerebellar dysfunctions, ataxia, movement disorders, cognitive deterioration, sleep disorders, hearing disorders, tremors, rigidity, bradykinesia, akinesia, and postural instability.

In one embodiment, the disease is a neurological disease and is selected from the group consisting of Parkinson's disease, Parkinson disease with dementia, Parkinson's disease at risk syndrome, dementia with Lewy bodies, Lewy body variant of Alzheimer's disease, combined Parkinson's disease and Alzheimer's disease, multiple system atrophy, striatonigral degeneration, olivopontocerebellar atrophy, and Shy-Drager syndrome.

In one embodiment, a compound selected from the group consisting of Formula I or Formula la, or a salt thereof, or a composition comprising such compound or salt thereof is provided for use in the treatment of Parkinson's disease in a mammalian subject.

In one embodiment, a compound selected from the group consisting of Formula I or Formula Ia, or a salt thereof, or a composition comprising such a compound or salt thereof is provided for use in the treatment of cancer i na mammalian subject wherein the cancer is selected from the group consisting of melanoma, acute myelogenous leukemia, breast carcinoma, lung adenocarincoma, prostate adenocarcinoma, renal cell carcinoma, and papillary thyroid carcinoma.

In one embodiment, a compound selected from the group consisting of Formula I or Formula Ia, or a salt thereof, or a composition comprising such a compound or salt thereof is provided for use in the treatment of an autoimmune disease in a mammalian subject, wherein the autoimmune disease is selected from the group consisting of Crohn's disease and ulcerative colitis.

In one embodiment, a compound selected from the group consisting of Formula I or Formula Ia, or a salt thereof, or a composition comprising such compound or salt thereof is provided for use in the treatment of leprosy in a mammalian subject.

In one embodiment, further to any of the above embodiments for use in treating a disease, the compound is a compound of Formula I, or a salt thereof, or a composition comprising such compound or salt thereof.

In one embodiment, further to any of the above embodiments for use in treating a disease, the compound is a compound of Formula Ia, or a salt thereof, or a composition comprising such compound or salt thereof.

In one embodiment, further to any of the above embodiments for use in treating a disease, the compound is a compound of Formula I, or a salt thereof, or a composition comprising such compound or salt thereof.

In one embodiment, further to any of the above embodiments for use in treating a disease, the compound is a compound of Formula Ia, or a salt thereof, or a composition comprising such compound or salt thereof.

In another aspect of the invention, there is provided a compound of Formula I or Ia for use in inhibiting the kinase activity of LRRK2 or a mutant form thereof. In an embodiment, the LRRK2 or mutant form thereof is in a solution. In another embodiment, the LRRK2 or mutant form thereof is in a cell. In another embodiment, the LRRK2 or mutant form thereof is in a mammal and said compound is administered to said mammal in an amount effective to inhibit kinase activity of LRRK2 or a mutant form of LRRK2.

### Compound Forms and Derivatives

In one aspect, various forms of compounds as described herein are provided. In one embodiment, a compound of Formula I may exist in a number of different forms, for example, tautomers, isomers, racemic mixtures, salts, pharmaceutically acceptable solvates, isotopically enhanced forms, conjugates, and other solid forms thereof, including different crystal forms, polymorphs or amorphous solids.

A compound as described herein, e.g. a compound of Formula I, can exist in particular geometric, conformational or stereoisomeric forms. The compound of Formula I includes all such isomeric forms, including *cis*- and *trans*-isomers, atropisomers, (-)- and (+)-enantiomers, diastereomers, (D)-isomers, (L)-isomers, the racemic mixtures thereof, and other mixtures thereof, such as enantiomerically or diastereomerically enriched mixtures. Such regioisomers and stereoisomers may be isolated in enriched form by standard separation methods known to those skilled in the art. Additional asymmetric carbon atoms can be present in a substituent such as an alkyl group. When the compounds described herein contain olefinic double bonds or other centers of geometric asymmetry, and unless specified otherwise, it is intended that the compounds include both E and Z geometric isomers. Compounds may also include regions that are sterically constrained such that atropisomers may be isolated by standard separation methods known to those skilled in the art. Likewise, all tautomeric forms and mixtures of tautomers are included.

Optically active (*R*)- and (*S*)-isomers and *d* and *l* isomers can be prepared using chiral synthons or chiral reagents, or resolved using conventional techniques. Resolution of the racemates can be accomplished, for example, by conventional methods such as crystallization in the presence of a resolving agent; chromatography, using, for example a chiral HPLC column; or derivatizing the racemic mixture with a resolving reagent to generate diastereomers, separating the diastereomers via chromatography, and removing the resolving agent to generate the original compound in enantiomerically enriched form. Any of the above procedures can be repeated to increase the enantiomeric purity of a compound. If, for instance, a particular enantiomer of a compound as described herein is desired, it can be prepared by asymmetric synthesis, or by derivatization with a chiral auxiliary, where the resulting diastereomeric mixture is separated and the auxiliary group cleaved to provide the desired enantiomers. Alternatively, where the molecule contains a basic functional group, such as an amino group, or an acidic functional group, such as a carboxyl group, diastereomeric salts can be formed with an appropriate optically active acid or base, followed by resolution of the diastereomers thus formed by fractional crystallization or chromatographic means known in the art, and subsequent recovery of the enantiomers in enriched form. In addition, separation of enantiomers and diastereomers is frequently accomplished using chromatography employing chiral, stationary phases, optionally in combination with chemical derivatization (e.g., formation of carbamates from amines).

A compound as described herein, e.g. a compound of Formula I, can exist in a salts form. A compound of Formula I may be prepared with relatively nontoxic acids or bases, depending on the particular substituents found on the compounds described herein. Such salts and their preparation for use as pharmaceuticals are readily known to those of skill in the art. Such salts may provide improved properties, e.g. solubility or pharmacokinetic properties, such that the activity of the compound of Formula I is enhanced upon administration to a subject. It is understood that such salts are effectively equivalent to a compound of Formula I, i.e. when such a salt is administered into a subject, such administration effectively encompasses the use of a compound of Formula I. When a compound as described herein (e.g. a compound of Formula I) contain relatively acidic functionalities (e.g., -COOH group), base addition salts can be obtained by contacting the compound (e.g., neutral form of such compound) with a sufficient amount of the desired base, either neat or in a suitable inert solvent. Examples of pharmaceutically acceptable base addition salts include lithium, sodium, potassium, calcium, ammonium, organic amino (e.g. ethylenediamine, diethylamine, piperazine, ethanolamine, diethanolamine, triethanolamine, tromethamine, choline, meglumine, benzathine, 4-phenylcyclohexylamine), zinc, magnesium and aluminum salts and the like. When a compound as described herein (e.g. a compound of Formula I) contains relatively basic functionalities (e.g., amines), acid addition salts can be obtained, e.g., by contacting the compound (e.g., neutral form of such compound) with a sufficient amount of the desired acid, either neat or in a suitable inert solvent. Examples of pharmaceutically acceptable acid addition salts include those derived from inorganic acids like hydrochloric, hydrobromic, hydroiodic, nitric, carbonic, monohydrogencarbonic, phosphoric, diphosphoric, monohydrogenphosphoric, dihydrogenphosphoric, sulfuric, monohydrogensulfuric, thiocyanic, hydriodic and the like, as well as the salts derived from relatively nontoxic organic acids like formic, acetic, alginic, propionic, isobutyric, ascorbic, aspartic, gentisic, galactaric, D-glucoheptanoic, D-gluconic, D-glucoronic, D-galactunoric, malic, maleic, malonic, benzoic, succinic, suberic, fumaric, glutaric, 2-oxoglutaric, adipic, capric, caproic, caprylic, dodecylsulfuric, lactic, lactobionic, mandelic, naphthylene-1,5-disulfonic, naphthalene-2-sulfonic, 1-hydroxy-2-napthoic, orotic, oxalic, phthalic, pyroglutamic, glycerophosphoric, hippuric, benzenesulfonic, p-toluenesulfonic, camphorsulfonic, camphoric, cinnamic, citric, tartaric, methanesulfonic, nicotinic, ethanesulfonic, ethane-1,2-disulfonic, 2-hydroxyethanesulfonic, salicylic, lauric, oleic, palmitic, pamoic, sebacic, undecylenic, stearic and the like. Also included are salts of amino acids such as glutamate, lysinate, arginate and the like (see, for example, Berge et al., Journal of Pharmaceutical Science, 1977, 66: 1-19). Certain specific compounds as described herein (e.g. a compound of Formula I) contain both basic and acidic functionalities that allow the compounds to be converted into either base or acid addition salts.

The neutral forms of the compounds can be regenerated, for example, by contacting the salt with a base or acid and isolating the parent compound in the conventional manner. The parent form of the compound can differ from the various salt forms in certain physical properties, such as solubility in polar solvents, but otherwise the salts are equivalent to the parent form of the compound for the purposes of the present invention.

When a substituent includes a negatively charged oxygen atom "O⁻", e.g., in "-COO⁻", then the formula is meant to optionally include a proton or an organic or inorganic cationic counterion. In one example, the resulting salt form of the compound is pharmaceutically acceptable. Further, when a compound of Formula I includes an acidic group, such as a carboxylic acid group, e.g., written as the substituent "-COOH", "-CO₂H" or "-C(O)₂H", then the formula is meant to optionally include the corresponding "de-protonated" form of that acidic group, e.g., "-COO⁻", "-CO₂" or "-C(O)₂⁻", respectively.

A compound as described herein, e.g. a compound of Formula I, can exist in unsolvated forms as well as solvated forms, including hydrated forms. Such solvates may provide improved properties, e.g. solubility or pharmacokinetic properties, such that the activity of the compound of Formula I is enhanced upon administration to a subject. It is understood that such solvated forms are effectively equivalent to a compound of Formula I, i.e. when such a solvate is administered into a subject, such administration effectively encompasses the use of a compound of Formula I.

A compound as described herein, e.g. a compound of Formula I, can exist in multiple crystalline forms, i.e. polymorphs, or in an amorphous form, and a compound of Formula I encompasses all such forms of the compound. In general, all physical forms are of use in the methods contemplated herein. Such physical forms may provide improved properties, e.g. solubility or pharmacokinetic properties, such that the activity of the compound of Formula I is enhanced upon administration of the particular form to a subject.

A compound as described herein, e.g. a compound of Formula I, can contain natural or unnatural proportions of atomic isotopes at one or more of the atoms that constitute such compounds. For example, the compounds can be radiolabeled with radioactive isotopes, such as for example tritium (³H), iodine-125 (¹²⁵I) or carbon-14 (¹⁴C). All isotopic variations of a compound as described herein, whether radioactive or not, are effectively encompassed by a compound as described herein, e.g., a compound in which one or more of the hydrogen atoms are replaced with another stable isotope of hydrogen (i.e., deuterium) or a radioactive isotope (i.e., tritium), is expected to have similar activity as it relates to LRRK2 kinase inhibition, and is effectively equivalent to a compound of Formula I. Such an isotopically enhanced compound may be useful, for example, in detection of the compound in vivo or in biological tissue, such as a radiolabelled compound containing ³H or ¹⁴C to assess tissue distribution, or a positron emitting compound containing ¹¹C, ¹⁵O, ¹³N, ¹⁸F or the like useful in positron emission tomography for in vivo imaging. Similarly, a deuterated compound my provide the compound with greater metabolic stability relative to the non-deuterated compound to provide improved pharmacokinetic properties. Such a compound is readily prepared by the methods as described herein, where suitable isotopically enhanced reagents may be used in place of non-enhanced reagents. For example, alkyl groups may include isotopic variants of hydrogen and carbon, such that methyl, for example, includes -CH₃, or may include the analogous structure in which any atoms can include any isotopes thereof, for example -CD₃, -¹⁴CH₃, and the like.

### Pharmaceutical Compositions:

Pharmaceutical compositions are provided, including a compound as described herein, e.g. compounds of Formula I, or any salts or solvates thereof, and at least one pharmaceutically acceptable carrier. The term "pharmaceutically acceptable carrier" means all pharmaceutically acceptable ingredients known to those of skill in the art, which are typically considered non-active ingredients. The term "pharmaceutically acceptable carrier" includes solvents, solid or liquid diluents, vehicles, adjuvants, excipients, glidants, binders, granulating agents, dispersing agents, suspending agents, wetting agents, lubricating agents, disintegrants, solubilizers, stabilizers, emulsifiers, fillers, preservatives (e.g., anti-oxidants), flavoring agents, sweetening agents, thickening agents, buffering agents, coloring agents and the like, as well as any mixtures thereof. Exemplary carriers (i.e., excipients) are described in, e.g., Handbook of Pharmaceutical Manufacturing Formulations, Volumes 1-6, Niazi, Sarfaraz K., Taylor & Francis Group 2005. A pharmaceutical composition may include one or more compounds of Formula I, or any salt or solvate thereof, in association with one or more pharmaceutically acceptable carrier and optionally other active ingredients.

The compounds of Formula I may be administered orally, topically, parenterally, by inhalation or spray or rectally in dosage unit formulations containing at least one pharmaceutically acceptable carrier. The term "parenteral" as used herein includes percutaneous, subcutaneous, intravascular (e.g., intravenous), intramuscular, or intrathecal injection or infusion techniques and the like. The pharmaceutical compositions containing compounds of Formula I may be in a form suitable for oral use, for example, as tablets, troches, lozenges, aqueous or oily suspensions, dispersible powders or granules, emulsion, hard or soft capsules, or syrups or elixirs.

Compositions intended for oral use may be prepared according to any method known to the art for the manufacture of pharmaceutical compositions and such compositions may contain one or more agents selected from the group consisting of sweetening agents, flavoring agents, coloring agents and preservative agents in order to provide pharmaceutically elegant and palatable preparations. Tablets contain the active ingredient in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques. In some cases such coatings may be prepared by known techniques to delay disintegration and absorption in the gastrointestinal tract and thereby provide a sustained action over a longer period. For example, a time delay material such as glyceryl monosterate or glyceryl distearate may be employed.

Formulations for oral use may also be presented as hard gelatin capsules, wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil. Formulations for oral use may also be presented as lozenges.

Aqueous suspensions contain the active materials in admixture with excipients suitable for the manufacture of aqueous suspensions. Such excipients are suspending agents, for example sodium carboxymethylcellulose, methylcellulose, hydropropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Oily suspensions may be formulated by suspending the active ingredients in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide palatable oral preparations. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, may also be present.

Pharmaceutical compositions of compounds of Formula I, or any salts or solvates thereof, may also be in the form of oil-in-water emulsions. The oily phase may be a vegetable oil or a mineral oil or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol, glucose or sucrose. Such formulations may also contain a demulcent, a preservative, a flavoring agent or a coloring agent. The pharmaceutical compositions may be in the form of a sterile, injectable, aqueous or oleaginous suspension. This suspension may be formulated according to the known art using those suitable dispersing or wetting agents and suspending agents that have been mentioned above. The sterile injectable preparation may also be a sterile injectable solution or suspension in a non-toxic parentally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

The compounds of Formula I may also be administered in the form of suppositories, e.g., for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

Compounds of Formula I may be administered parenterally in a sterile medium. The compound, depending on the vehicle and concentration used, can either be suspended or dissolved in the vehicle. Advantageously, adjuvants such as local anesthetics, preservatives and buffering agents can be dissolved in the vehicle.

For disorders of the eye or other external tissues, e.g., mouth and skin, the formulations are readily applied as a topical gel, spray, ointment or cream, or as a scleral suppository, containing the active ingredients in a total amount of, for example, 0.075 to 30% w/w, also 0.2 to 20% w/w and also 0.4 to 15% w/w. When formulated in an ointment, the active ingredients may be employed with either paraffinic or a water-miscible ointment base.

Alternatively, the active ingredients may be formulated in a cream with an oil-in-water cream base. If desired, the aqueous phase of the cream base may include, for example at least 30% w/w of a polyhydric alcohol such as propylene glycol, butane-1,3-diol, mannitol, sorbitol, glycerol, polyethylene glycol and mixtures thereof. The topical formulation may desirably include a compound, which enhances absorption or penetration of the active ingredient through the skin or other affected areas. Examples of such dermal penetration enhancers include dimethylsulfoxide and related analogs. The compounds of Formula I can also be administered by a transdermal device. In one example topical administration will be accomplished using a patch either of the reservoir and porous membrane type or of a solid matrix variety. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane. The transdermal patch may include the compound in a suitable solvent system with an adhesive system, such as an acrylic emulsion, and a polyester patch. The oily phase of the emulsions may be constituted from known ingredients in a known manner. While the phase may comprise merely an emulsifier, it may comprise a mixture of at least one emulsifier with a fat or oil or with both a fat and an oil. In one example, a hydrophilic emulsifier is included together with a lipophilic emulsifier, which acts as a stabilizer. In one example, both an oil and a fat are included. Together, the emulsifier(s) with or without stabilizer(s) make-up the so-called emulsifying wax, and the wax together with the oil and fat make up the so-called emulsifying ointment base, which forms the oily, dispersed phase of the cream formulations. Emulsifiers and emulsion stabilizers suitable for use in the formulation of compounds as described herein include Tween 60, Span 80, cetostearyl alcohol, myristyl alcohol, glyceryl monostearate, and sodium lauryl sulfate, among others. The choice of suitable oils or fats for the formulation is based on achieving the desired cosmetic properties, since the solubility of the active compound in most oils likely to be used in pharmaceutical emulsion formulations is very low. Thus, an exemplary cream is a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredients are dissolved or suspended in suitable carrier, especially an aqueous solvent for the active ingredients. The anti-inflammatory active ingredients are can be present in such formulations in a concentration of 0.5 to 20%, advantageously 0.5 to 10% and particularly about 1.5% w/w. For therapeutic purposes, the active compounds, i.e. compounds of Formula I, are ordinarily combined with one or more adjuvants appropriate to the indicated route of administration. The compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

Dosage levels of the order of from about 0.005 mg to about 100 mg per kilogram of body weight per day are useful in the treatment of the diseases and conditions described herein (e.g., about 0.35 mg to about 7 g per human patient per day, based on an average adult person weight of 70 kg). The amount of active ingredient that may be combined with the carrier materials to produce a single dosage form will vary depending upon the host treated and the particular mode of administration. Dosage unit forms will generally contain between from about 1 mg to about 500 mg of an active ingredient. The daily dose can be administered in one to four doses per day. In the case of skin conditions, it may be efficacious to apply a topical preparation of compounds of Formula I to the affected area one to four times a day.

Formulations suitable for inhalation or insufflation include solutions and suspensions in pharmaceutically acceptable aqueous or organic solvents, or mixtures thereof, and powders. The liquid or solid compositions may contain suitable pharmaceutically acceptable excipients as describe above. The compositions may be administered by oral or nasal respiratory route for local or systemic effect. Compositions may be nebulized by use of inert gases or vaporized, and breathed directly from the nebulizing/vaporizing device or the nebulizing device may be attached to a facemask tent or intermittent positive pressure-breathing machine.

It will be understood, however, that the specific dose level for any particular patient will depend upon a variety of factors including the activity of the specific compound employed, the age, body weight, general health, sex, diet, time of administration, route bf administration, and rate of excretion, drug combination and the severity of the particular disease undergoing therapy.

For administration to non-human animals, the composition may also be added to the animal feed or drinking water. It may be convenient to formulate the animal feed and drinking water compositions so that the animal takes in a therapeutically appropriate quantity of the composition along with its diet. It may also be convenient to present the composition as a premix for addition to the feed or drinking water.

### Methods of use:

Over-activation of LRRK2 kinase activity, e.g. in kinase mutant G2019S, is believed to be an important mechanism in α-synuclein related neurodegeneration, and is implicated in diseases that are characterized by the formation of Lewy bodies. Compounds as described herein exhibit inhibitory activity against LRRK2 kinase, including LRRK2 mutant kinase, including mutant G2019S. Kinase activity can be determined using a kinase assay, which typically employs a kinase substrate and a phosphate group donor, such as ATP (or a derivative thereof). Exemplary kinase assays are described in Example A. The kinase catalyzes the transfer of a phosphate group from the phosphate group donor (e.g., ATP) onto the substrate forming a covalent bond. Compounds as described herein can inhibit the activity of the kinase, slowing the above described reaction and resulting in a smaller number of phosphate groups being transferred. In one example, a method (i.e., an *in vitro* assay) is provided that includes: (i) contacting a compound of Formula I with a LRRK2 kinase, thereby forming a mixture. The method may further include (ii) contacting the mixture with a kinase substrate (e.g., peptide substrate) and ATP (or a derivative thereof), thereby forming an amount of phosphorylated kinase substrate. The method can further include (iii) measuring the amount of phosphorylated kinase substrate. The amount of phosphorylated substrate may be measured using a detection reagent. Suitable detection reagents can include a metal reagent, such as a lanthanoid (e.g., Eu-63), a radioactive probe, a labeled (e.g., fluorescently labelled) antibody and combinations thereof. In one example, the assay is a fluorescence resonance energy transfer (FRET) assay (e.g., TR-FRET). Examples of such assays are described in Example A. In a particular embodiment, a compound of Formula I is used as a reference standard to determine the *in vitro* activity of other compounds in a kinase assay as described above. Thus, in another example, the compound of Formula I is used in an *in vitro* assay for identifying candidate compounds that are capable of inhibiting LRRK2 kinase, including LRRK2 mutant kinase, including mutant G2019S kinase.

Compounds and compositions as described herein (i.e. Compounds of Formula I, or a salt or solvate thereof) are useful in the treatment and/or prevention of LRRK2 kinase mediated disorders, including LRRK2 kinase mutant mediated diseases, such as LRRK2 kinase mutant G2019S mediated diseases, including neurological diseases such as Parkinson's disease and other Lewy body diseases such as Parkinson disease with dementia, Parkinson's disease at risk syndrome, dementia with Lewy bodies (i.e., diffuse Lewy body disease (DLBD), Lewy body dementia, Lewy body disease, cortical Lewy body disease or senile dementia of Lewy type), Lewy body variant of Alzheimer's disease (i.e., diffuse Lewy body type of Alzheimer's disease), combined Parkinson's disease and Alzheimer's disease, as well as diseases associated with glial cortical inclusions, such as syndromes identified as multiple system atrophy, including striatonigral degeneration, olivopontocerebellar atrophy, and Shy-Drager syndrome, or other diseases associated with Parkinsonism, such as Hallervorden-Spatz syndrome (also referred to as Hallervorden-Spatz disease), fronto-temporal dementia, Sandhoff disease, progressive supranuclear palsy, corticobasal degeneration, autonomic dysfunctions (e.g., postural or orthostatic hypotension), cerebellar dysfunctions, ataxia, movement disorders, cognitive deterioration, sleep disorders, hearing disorders, tremors, rigidity (e.g., joint stiffness, increased muscle tone), bradykinesia, akinesia and postural instability (failure of postural reflexes, along other disease related factors such as orthostatic hypotension or cognitive and sensory changes, which lead to impaired balance and falls); cancers, including melanoma, acute myelogenous leukemia, breast carcinoma, lung adenocarincoma, prostate adenocarcinoma, renal cell carcinoma, and papillary thyroid carcinoma; autoimmune diseases such as Inflammatory Bowel Disease (e.g. Crohn's disease and ulcerative colitis); and leprosy. An *in vivo* model, which can be used to assess the potential *in vivo* beneficial effect of the compounds of Formula I, is described in Example C.

### In Vitro Activities:

Certain compounds as described herein, e.g. compounds of Formula I, exhibit various *in vitro* biological activities (see, e.g., Example A), such as inhibition of LRRK2 kinase activity and LRRK2 mutant G2019S kinase activity. *In vitro* assays for the determination of LRRK2 kinase activities are known in the art and exemplary assay formats are described herein (see e.g., Example A).

### In Vivo Activities:

Certain compounds as described herein exhibit cellular biological activities, such as reduction in phosphorylation of ser910 or ser935 in HEK-293 cells transfected with either wild-type LRRK2 or LRRK2 G2019S mutant. For example, the phosphorylation of ser910 or ser935 in such HEK-293 transfected cells treated with a compound of Formula I will be less than 90%, less than 80%, less than 70%, less than 60%, less than 50%, less than 40%, less than 30%, less than 20%, less than 10%, less than 5%, or less than 1% of the phosphorylation of ser910 or ser935 control cells that are not treated with compound.

Certain compounds as described herein exhibit *in vivo* biological activities, such as reduction in phosphorylation of ser910 or ser935 in a mouse model. For example, following peritoneal injection of a compound of Formula I (e.g., at a dose of about 50 mg, about 100 mg, about 200 mg or about 300mg/kg), or a vehicle control, kidney and brain tissue can be harvested and assessed for the phosphorylation of ser910 or ser935. In one example, the phosphorylation of ser910 or ser935 from the kidneys of mice treated with a compound of Formula I will be less than 90%, less than 80%, less than 70%, less than 60%, less than 50%, less than 40%, less than 30%, less than 20%, less than 10%, less than 5%, or less than 1% of the phosphorylation of ser910 or ser935 from the kidneys of control mice that are not treated with compound, and the phosphorylation of ser910 or ser935 from the brains of mice treated with a compound of Formula I will be less than 90%, less than 80%, less than 70%, less than 60%, less than 50%, less than 40%, less than 30%, less than 20%, less than 10%, less than 5%, or less than 1% of the phosphorylation of ser910 or ser935 from the brains of control mice that are not treated with compound.

### Synthesis of the Compounds:

The compounds as described herein, e.g. compounds of Formula I, can be prepared using methods known in the art of organic synthesis and those described herein in the Examples. The starting materials and various intermediates may be obtained from commercial sources, prepared from commercially available compounds, and/or prepared using known synthetic methods. For example, the compounds as described herein, as well as all intermediates, can be synthesized by known processes using either solution or solid phase techniques. Exemplary procedures for preparing compounds as described herein are outlined in the following schemes.

Additionally, as will be apparent to those skilled in the art, conventional protecting groups may be necessary to prevent certain functional groups from undergoing undesired reactions. Suitable protecting groups for various functional groups as well as suitable conditions for protecting and deprotecting particular functional groups are well known in the art. For example, numerous protecting groups are described in T. W. Greene and P.G. M. Wuts, Protecting Groups in Organic Synthesis, Third Edition, Wiley, New York, 1999, and references cited therein.

In one example, compounds of Formula I wherein X is S, Y is -C(O)-, R² and R³ are R²² and R²³ respectively, and R⁴ is H, are prepared from a suitable heteroaryl carboxylic acid ester in four steps by methods as described in the following Scheme 1.

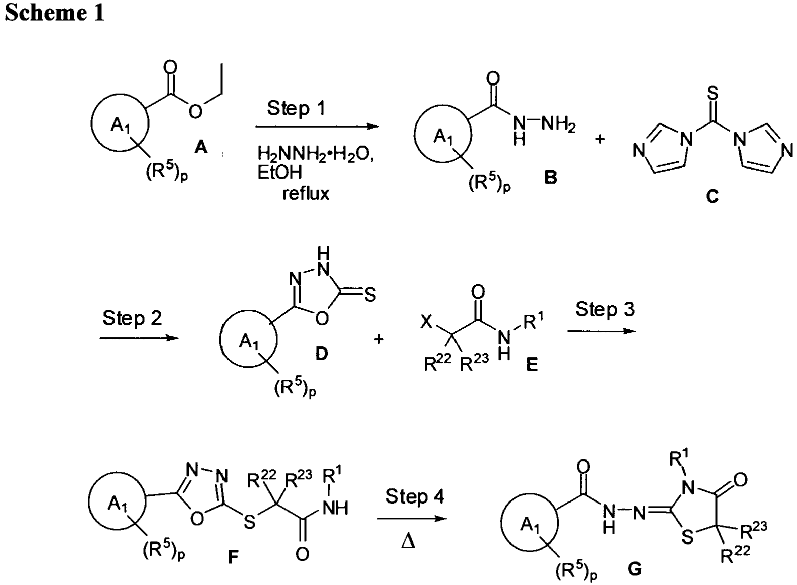

In a first step, heteroaryl carboxylic acid **A** (A₁, R⁵ and p are as described for compounds of Formula I) is refluxed with hydrazine hydrate either neat or in an alcoholic solvent to provide heteroaryl carbohydrazides such as **B** (J. Heterocyclic Chem. 2010, 47(4), 745). Heteroaryl substituted oxadiazolethiones such as **D** may be prepared in step 2 by treating **B** with CS₂ and base (Bioorg. Med. Chem. 2004, 12(19), 5107) or with a reagent such as di(1H-imidazol-1-yl)methanethione **C**, in a suitable solvent such as THF with heating. In step 3, addition of **D** to a haloacetamide such as **E** (R¹ is as described for compounds of Formula I, R²² and R²³ as described for compounds of Formula la, X is a halogen) with e.g. sodium acetate, in a suitable solvent such as ethanol provides thioacetamides such as **F** which, upon heating in step 4, may rearrange to give acylhydrazones such as **G.**

In one example, compounds of Formula I wherein X is S, Y is -C(O)-, and R², R³ and R⁴ are H, are prepared from a suitable thiosemicarbazide **H** in two steps by methods as described in the following Scheme 2.

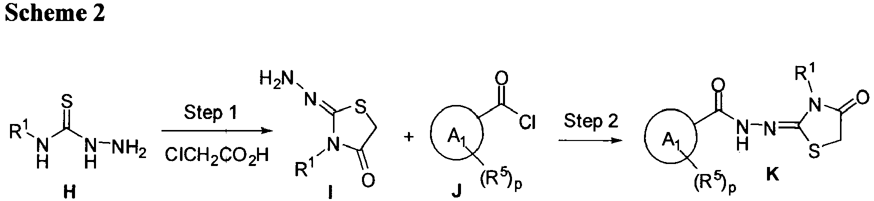

In a first step, thiosemicarbazide **H** (R¹ is as described for compounds of Formula I) may be reacted with chloroacetic acid to afford hydrazone **I** (Sulfur Letters 1991, 13(3), 101). Addition of **I** to an activated carbonyl heteroaryl compound such as **J** (A₁, R⁵ and p are as described for compounds of Formula I) in step 2 provides acylhydrazone **K.**

In one example, compounds of Formula I wherein X is S, Y is -C(O)-, and R², R³ and R⁴ are H, are prepared from a suitable heteroaryl carbohydrazide **B** (as prepared in Scheme 1 or commercially available) in two steps by methods as described in the following Scheme 3.

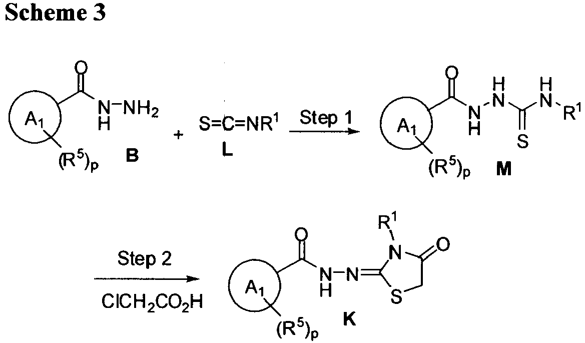

In a first step, addition of an isothiocyanate **L** (R¹ is as described for compounds of Formula I) to heteroaryl carbohydrazides **B** in a suitable solvent such as ethanol, with heating, provides a thiosemicarbazide such as **M.** Addition of chloroacetic acid in step 2 provides the desired acylhydrazone **K.**

The analogous compound wherein X is O is prepared similarly replacing isothiocyanate **L** with isocyanate **N** as shown in the following Scheme 4.

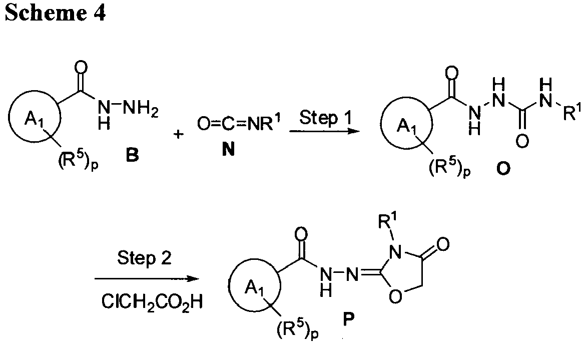

In a first step, isocyanates **N** may be used in place of isothiocyanate L to provide the corresponding acylhydrazones **O** (J. Heterocycl. Chem. 2007, 44(6), 1271.), which is similarly reacted in step 2 to provide **P.**

In one example, compounds of Formula I wherein X is S, Y is -CH₂-, and R², R³ and R⁴ are H, are prepared from a suitable heteroaryl carbohydrazide **B** (e.g. as prepared in Scheme 1 or commercially available) in two steps by methods as described in the following Scheme 5.

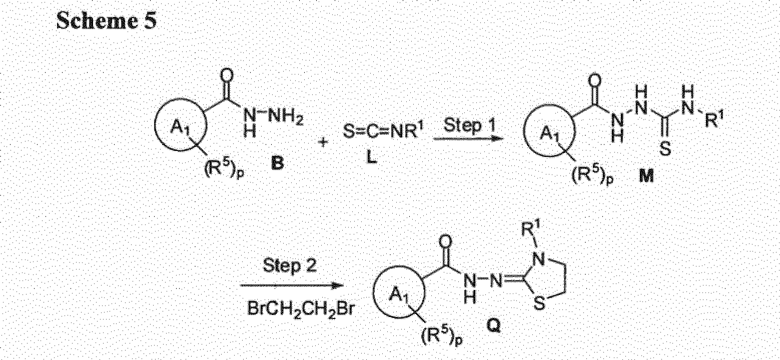

The first step is identical to Step 1 of Scheme 3 to provide compound **L.** Addition of 1,2-dibromoethane with e.g. sodium acetate, in a suitable solvent such as ethanol and heating in step 2 provides the desired acylhydrazone **Q.**

The invention is illustrated further by the following examples, which are not to be construed as limiting the invention. Analogous structures and alternative synthetic routes within the scope of the invention will be apparent to those skilled in the art.

### EXAMPLES

### General:

Reagents and solvents obtained from commercial suppliers are used without further purification unless otherwise stated. Thin layer chromatography is performed on precoated 0.25 mm silica gel plates (E. Merck, silica gel 60, F₂₅₄) or similar. Visualization is achieved using UV illumination or staining with phosphomolybdic acid, ninhydrin or other common staining reagents. Flash chromatography is performed using either a Biotage Flash 40 system and prepacked silica gel columns or hand packed columns (E. Merck silica gel 60, 230-400 mesh) or similar. Preparatory HPLC is performed on a Varian Prepstar high performance liquid chromatograph. ¹H NMR spectra are recorded at 400 MHz Bruker Avance spectrometer. Chemical shifts are reported in parts per million (ppm) downfield relative to tetramethylsilane (TMS) or to proton resonances resulting from incomplete deuteration of the NMR solvent (δ scale). In some instances the synthetic examples give a racemic mixture of stereoisomers, which are readily separated by chiral HPLC.

LCMS is performed on an Agilent 1100 Series HPLC with a Series 1100 MSD with electrospray ionization using a Phenomenex Luna C18 4.6 mm i.d. x 30 mm length, 3µ particle size column or similar. Compound purity is typically determined by HPLC/MS analysis using a variety of analytical methods.

The examples are intended to be illustrative and are not limiting or restrictive to the scope of the invention. For example, where additional compounds are prepared similarly to synthetic methods of another example, or in the same manner as another example, it is understood that conditions may vary, for example, any of the solvents, reaction times, reagents, temperatures, work up conditions, or other reaction parameters may be varied employing alternate solvents, reagents, reaction times, temperatures, work up conditions, and the like, as are readily available to one skilled in the art.

### Example 1

### Synthesis of 5-chloro-N'-(4-oxo-3-phenylthiazolidin-2-ylidene)thiophene-2-carbohydrazide (6)

5-Chloro-N'+(4-oxo-3-phenylthiazolidin-2-ylidene)thiophene-2-carbohydrazide **6** was prepared from 5-chlorothiophene-2-carbohydrazide 1 in 3 steps as follows:

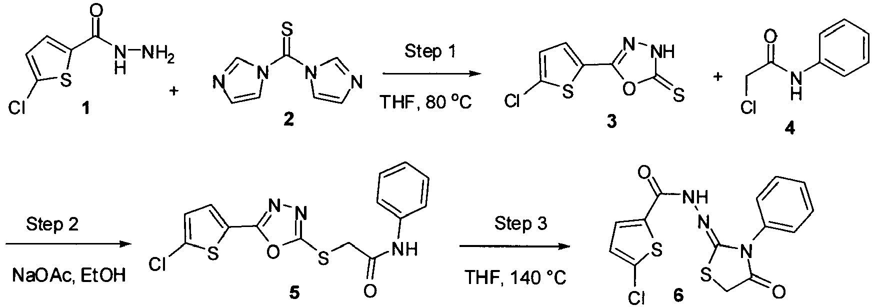

Step 1 - synthesis of 5-(5-chlorothiophen-2-yl)-1,3,4-oxadiazole-2(3H)-thione (**3**): 5-Chlorothiophene-2-carbohydrazide (**1**, 1.00 g, 5.66 mmol) and di(imidazol-1-yl)methanethione (**2**, 1.11 g, 6.23 mmol) were suspended in 20 mL of THF. The reaction mixture was plunged into a pre-heated 80 °C oil bath and stirred for 24 hours. The reaction mixture was concentrated under vacuum, and the desired compound was isolated as a yellow solid (**3**, 1.24g, 100%).

Step 2 - synthesis of 2-(5-(5-chlorothiophen-2-yl)-1,3,4-oxadiazol-2-ylthio-N-phenylacetamide (**5**): The 5-(5-chlorothiophen-2-yl)-1,3,4-oxadiazole-2(3H)-thione (3, 0.1651 g, 0.7549 mmol) was dissolved in 1 mL of ethanol and sodium acetate (0.06812 g, 0.8304 mmol) and 2-chloro-*N*-phenyl-acetamide (**4**, 0.1280 g, 0.7549 mmol) were added. The reaction mixture was stirred at room temperature overnight, then concentrated under vacuum and the residue was diluted with CH₂Cl₂ and water. The organic layer was isolated and dried with Na₂SO₄, filtered and the resulting filtrate was concentrated under vacuum. The resulting residue was purified by preparative HPLC (Solvent A-water (0.05% TFA), Solvent B- acetonitrile (0.05% TFA) with a gradient of 20% B to 70% B in 10 minutes, flow rate 1.5 mL/min at 35 °C, column: Phenomenex Luna C18 30 mm X 4.6 mm, 3µ). Appropriate fractions were combined and concentrated under vacuum to provide the desired compound (**5**, 0.074g, 28%).

Step 3 - synthesis of 5-chloro-N'-(4-oxo-3-phenylthiazolidin-2-ylidene)thiophene-2-carbohydrazide (**6**): The 2-(5-(5-chlorothiophen-2-yl)-1,3,4-oxadiazol-2-ylthio)-*N*-phenylacetamide (**5**, 1.99 g, 5.67 mmol) was dissolved in 15 mL of ethanol and sodium acetate (0.512 g, 6.24 mmol) was added. The reaction mixture was heated in a microwave for 10 minutes at 140 °C, then transferred to a round bottom and concentrated under vacuum. The resulting residue was suspended in CH₂Cl₂, filtered, and the resulting residue was purified by preparative HPLC (Solvent A-water (0.05% TFA), Solvent B- acetonitrile (0.05% TFA) with a gradient of 20% B to 70% B in 10 min, flow rate: 1.5 mL/min at 35 °C, column: Phenomenex Luna C18 30 mm X 4.6 mm, 3µ). Appropriate fractions were combined and concentrated under vacuum to provide the desired compound (**6**, 0.938g, 47%). ¹H NMR (400MHz, CDCl₃) δ: 8.07 (s, 1H), 7.67 (s, 1H), 7.59 (m, 3H), 7.35 (m, 2H), 6.81 (d, J = 4 Hz, 1H), 4.16 (s, 2H). MS: 352.0 m/z (M+H)⁺.

Additional compounds are prepared similarly to this method, replacing 2-chloro-*N*-phenyl-acetamide **4** with a suitable haloalkylamide in Step 2. The following compounds are prepared:
5-chloro-N'-(5-methyl-4-oxo-3-phenylthiazolidin-2-ylidene)thiophene-2-carbohydrazide (7),
5-chloro-N'-(3-(3-fluorophenyl)-4-oxothiazolidin-2-ylidene)thiophene-2-carbohydrazide (**8**),
5-chloro-N'-(3-(2-methoxyphenyl)-4-oxothiazolidin-2-ylidene)thiophene-2-carbohydrazide (**9**),
5-chloro-N'-(3-(2-fluorophenyl)-4-oxothiazolidin-2-ylidene)thiophene-2-carbohydrazide (**10**),
5-chloro-N'-(3-(4-fluorophenyl)-4-oxothiazolidin-2-ylidene)thiophene-2-carbohydrazide (**11**),
5-chloro-N'-(3-(2,4-difluorophenyl)-4-oxothiazolidin-2-ylidene)thiophene-2-carbohydrazide (**12**),
5-chloro-N'-(5-isopropyl-4-oxo-3-phenylthiazolidin-2-ylidene)thiophene-2-carbohydrazide (**13**),
5-chloro-N'-(5,5-dimethyl-4-oxo-3-phenylthiazolidin-2-ylidene)thiophene-2-carbohydrazide (**14**),
N'-(3-benzyl-4-oxothiazolidin-2-ylidene)-5-chlorothiophene-2-carbohydrazide (**15**),
5-chloro-N'-(4-oxo-3-(3-(trifluoromethyl)phenyl)thiazolidin-2-ylidene)thiophene-2-carbohydrazide (**16**).
The following table provides the compound number (column 1), haloalkylamide used in Step 2 (column 2) to give the compound shown in column 3. Identification data is provided in column 4.

| Comp. No. | Halo acetamide | Compound structure | Identification |
|---|---|---|---|
| **7** | 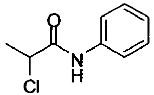 | 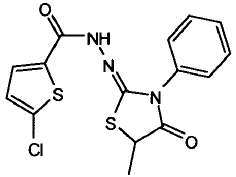 | MS: 366.0 m/z (M+H)^{+ 1}H-NMR (400MHz, CDCl₃) δ: 9.16 (s, 1H), 7.57 (m, 2H), 7.48 (d, J = 4 Hz, 1H), 7.31 (m, 2H), 7.10 (m, 1H), 6.99 (d, J = 4 Hz, 1H), 4.51 (m, 1H), 1.74 (d, J = 7.6 Hz, 3H) |
| **8** | 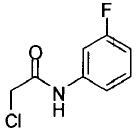 | 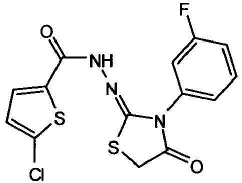 | MS: 370.0 m/z (M+H)^{+ 1}H-NMR (400MHz, CDCl₃) δ: 8.23 (s, 1H), 7.70 (s, 1H), 7.56 (m, 1H), 7.28 (m, 1H), 7.16 (m, 1H), 7.11 (m, 1H), 6.84 (d, J = 4 Hz, 1H), 4.16 (s, 2H) |
| **9** | 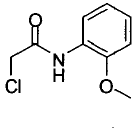 | 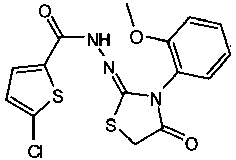 | MS: 382.0 m/z (M+H)^{+ 1}H-NMR (400MHz, CDCl₃) δ: 8.36 (s, 1H), 7.69 (s, 1H), 7.54 (m, 1H), 7.28 (m, 1H), 7.14 (m, 2H), 6.81 (d, J = 4 Hz, 1H), 4.13 (dd, J = 16.8, 6.4 Hz, 2H), 3.82 (s, 3H) |
| **10** | 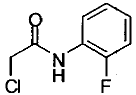 | 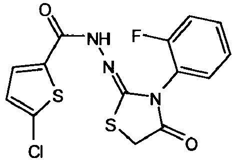 | MS: 370.0 m/z (M+H)^{+ 1}H-NMR (400MHz, CDCl₃) δ: 8.43 (s, 1H), 7.70 (s, 1H), 7.57 (m, 1H), 7.37 (m, 3H), 6.82 (d, J = 4 Hz, 1H), 4.19 (dd, J = 16.8, 6.4 Hz, 2H) |
| **11** | 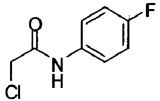 | 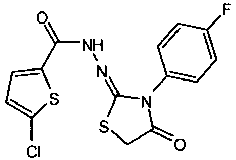 | MS: 370.0 m/z (M+H)^{+ 1}H-NMR (400MHz, CDCl₃) δ: 8.11 (s, 1H), 7.69 (s, 1H), 7.32 (m, 5H), 6.84 (d, J = 4 Hz, 1H), 4.15 (s, 2H) |
| **12** | 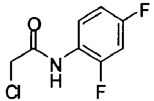 | 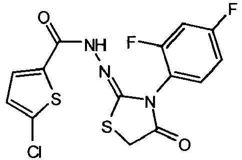 | MS: 388.0 m/z (M+H)^{+ 1}H-NMR (400MHz, CDCl₃) δ: 8.43 (s, 1H), 7.73 (s, 1H), 7.38 (m, 1H), 7.12 (m, 2H), 6.86 (d, J = 4 Hz, 1H), 4.19 (dd, J = 17.2, 6.4 Hz, 2H) |
| **13** | 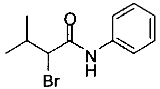 | 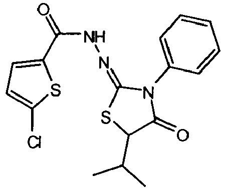 | MS: 394.1 m/z (M+H)^{+ 1}H-NMR (400MHz, CDCl₃) δ: 7.92 (s, 1H), 7.67 (s, 1H), 7.56 (m, 3H), 7.31 (m, 2H), 6.80 (d, J = 4 Hz, 1H), 4.46 (m, 1H), 2.73 (m, 1H), 1.15 (d, J = 6.8 Hz, 3H), 1.08 (d, J = 6.4 Hz, 3H) |
| **14** | 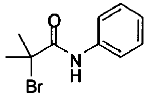 | 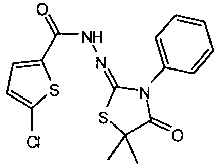 | MS: 380.0 m/z (M+H)^{+ 1}H-NMR (400MHz, CDCl₃) δ: 8.02 (s, 1H), 7.67 (s, 1H), 7.59 (m, 3H), 7.35 (m, 2H), 6.80 (d, J = 4 Hz, 1H), 1.84 (s, 6H) |
| **15** | 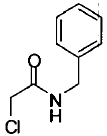 | 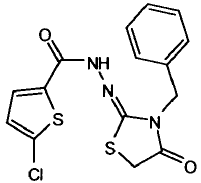 | MS: 366.0 m/z (M+H)^{+ 1}H-NMR (400MHz, CDCl₃) δ: 7.94 (s, 1H), 7.40 (m, 2H), 7.32 (m, 3H), 6.92 (d, J = 4 Hz, 1H),5.07 (s, 2H), 4.00 (s, 2H) |
| **16** | 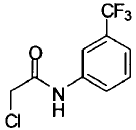 | 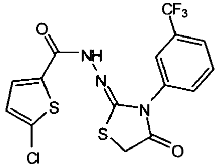 | MS: 420.0 m/z (M+H)^{+ 1}H-NMR (400MHz, CDCl₃) δ: 7.91 (s, 1H), 7.83 (m, 1H), 7.74 (m, 1H), 7.67 (m, 2H), 7.57 (m, 2H), 6.83 (d, J = 4 Hz, 1H), 4.19 (s, 2H) |

### Example 2

### Synthesis of 5-,chloro-N'-(3-phenylthiazolidin-2-ylidene)thiophene-2-carbohydrazide (19)

5-chloro-N'-(3-phenylthiazolidin-2-ylidene)thiophene-2-carbohydrazide **9** was prepared from 5-chlorothiophene-2-carbohydrazide **1** and Isothiocynatobenzene **17** in two steps as follows:

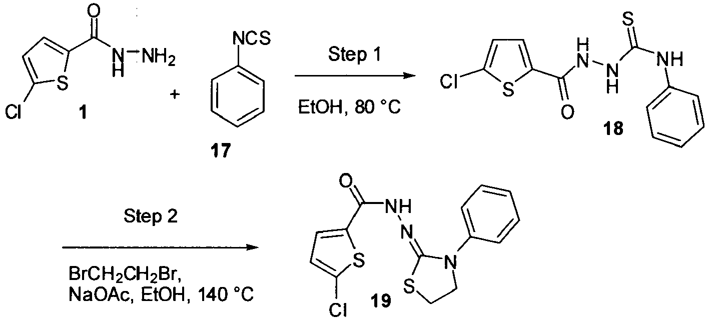

Step 1 - synthesis of 2-(5-chlorothiophene-2-carbonyl)-N-phenylhydrazinecarbothioamide (**18**): Isothiocyanatobenzene (**17**, 0.4g, 3 mmol) and 5-chlorothiophene-2-carbohydrazide (**1**, 0.5 g, 3 mmol) were combined in 10 mL of ethanol and the reaction mixture was heated at 80 °C overnight. The reaction mixture was diluted with EtOH and filtered, and the desired compound was isolated from the filtrate (**18**, 0.539g, 60%).

Step 2 - synthesis of 5-chloro-N'-(3-phenylthiazolidin-2-ylidene)thiophene-2-carbohydrazide (**19**): 2-(5-chlorothiophene-2-carbonyl)-*N-*phenylhydrazinecarbothioamide (**18**, 0.139g, 0.4458 mmol) and sodium acetate (0.073g, 0.891 mmol) were combined in 3 mL of ethanol, and 1,2-dibromoethane (0.083g, 0.445 mmol) was added. The reaction mixture was plunged into a pre-heated 80 °C oil bath and stirred for 1 hour, then concentrated under vacuum. The resulting residue was dissolved in CH₂Cl₂, and was washed with water. The organic layer was dried with Na₂SO₄, filtered and the filtrate concentrated under vacuum. The resulting residue was purified by preparative HPLC (Solvent A-water (0.05% TFA), Solvent B-acetonitrile (0.05% TFA) with a gradient of 20% B to 70% B in 10 min, flow rate 1.5 mL/min at 35 °C, column: Phenomenex Luna C18 30 mm X 4.6 mm, 3µ). Appropriate fractions were combined and concentrated under vacuum to provide the desired compound (**19**, 0.017g, 11%). ¹H-NMR (400MHz, DMSO-d₆, 60°C) δ: 8.47(s, 1H), 8.42 (d, *J*=1 Hz,1H), 8.27 (d, *J*=9Hz, 1H), 8.07 (dd, *J*=9,1Hz, 1H), 7.87 (bs, 1H). MS: 314.9 m/z (M+H)⁺.

### Example A

### In vitro Kinase Activities (LRRK2 TR-FRET Peptide Assay)

Compounds as described herein (compounds of Formula I, e.g., compounds of the above Examples) are tested for their *in vitro* kinase activities using various LRRK2 (including LRRK2 G2019S mutant) assays. For example, assays were performed in a total volume of 20 µL using the same kinase reaction buffer (50 mM HEPES pH 7.5, 10 mM MgCl₂, 1 mM EGTA, 2 mM DTT and 0.01% Tween-20) for wild-type or G2019S mutant LRRK2. Serially diluted compounds (1% DMSO as cosolvent) were pre-incubated with recombinant GST-LRRK2 (wild-type, or G2019S mutant, Invitrogen) for 15 minutes at room temperature in 384-well Corning black plates. Mixtures of ATP and biotin-LRRKtide substrate (biotin-RLGRDKYKTLRQIRQ) were added to the wells at a final concentration of 100 µM ATP and 100 nM substrate, with final kinase concentration of 10 nM. The kinase reactions were carried out at room temperature for 60 minutes, then the reaction was stopped with the addition of 10 µL/well of stop/detection buffer (25 mM HEPES pH 7.5, 66 mM EDTA, 0.8 M KF and 0.1% BSA) that contains streptavidin-XL665 (12.5 nM) and europium-conjugated phospho-specific antibody (2nM). The plates were read 1 hour later and the time-resolved fluorescence (665 nm to 615 nm ratio) measured using an Envision reader. The inhibition of each well was calculated using the control and background readings for that plate. IC₅₀ values were determined from dose-response curves using eight concentrations from the serial dilution of the test compounds.

Compounds of the Examples typically demonstrated inhibition of LRRK2 with an IC₅₀ below 1 µM for both wild type and G2019S mutant kinase activity. The following table summarizes exemplary compounds from the Examples above and their *in vitro* IC₅₀ values for LRRK2 as determined using the procedures of Example A. Compounds are identified by the Example number and compound number identification given in the Example.

| **Example/ Comp. No.** | **LRRK2 wild-type** IC₅₀ (µM) |
|---|---|
| **1-6** | 0.0009 |
| **1-7** | 0.011 |
| **1-8** | 0.0011 |
| **1-9** | 1.17 |
| **1-10** | 0.0005 |
| **1-11** | 0.0009 |
| **1-12** | 0.0005 |
| **1-13** | 0.21 |
| **1-14** | 0.33 |
| **1-15** | 0.013 |
| **1-16** | 0.111 |
| **2-19** | 0.051 |

Additional assays for LRRK2 kinase activity are known, for example, assays involving monitoring of the phosphorylation of the substrate Nictide, or LRRK2 autophosphorylation or phosphorylation of myelin basic protein are known (Deng et al., Nature Chemical Biology 2011, 7(4):203-5; Dzamko et al., Biochemical Journal 2010,430(3):405-13; and Lee et al., Nature Medicine 2010, 16(9):998-1000, which include descriptions of LRRK2 biochemical, cell based and *in vivo* assays). Compounds can also be readily assayed using other kinases in order to determine IC₅₀ values against these kinases and determine selectivity of the compound activity against LRRK2 relative to these other kinases. Alternatively, compounds can be similarly tested at a particular concentration, such as at 1 µM or 10 µM test compound, against a variety of kinases, including LRRK2 and LRRK2 G2019S, and the % inhibition of the kinase activity at the given concentration can be used to assess the selectivity of compounds for LRRK2 relative to other kinases.

### Example B

### Cell Activities

Compounds as described herein (compounds of Formula I, e.g., compounds of the above Examples) may also be tested for their activity in a cell based assay. For example, LRRK2, both wild-type and G2019S mutant, is stably transfected into HEK-293. It is known that LRRK2 binds to isoforms of 14-3-3 proteins, an interaction that depends on the phosphorylation of LRRK2 at serine 910 (ser910) and serine 935 (ser935), and further that LRRK2 kinase indirectly affects this phosphorylation of ser910 and ser935. As such, the transfected HEK-293 cells may be used to assess the cellular activity of LRRK2 inhibitors. Three separate readouts may be monitored to assess the compounds for ability to inhibit LRRK2 kinase. Firstly, antibodies to phosphorylated ser910 and ser935 can be used to assess the effect of compounds on ser910 and ser935 phosphorylation, which decreases with inhibition of LRRK2 kinase activity. Secondly, the dephosphorylated LRRK2 has reduced binding of 14-3-3 protein, such that the binding of 14-3-3 protein may be monitored to assess LRRK2 inhibition. Thirdly, LRRK2 that is not bound to 14-3-3 protein is seen to localize within cytoplasmic pools. Green fluorescent protein labeled LRRK2 (GFP-LRRK2) can be used in order to monitor the LRRK2, where LRRK2 kinase activity inhibition results in observation of GFP-LRRK2 localized in such cytoplasmic pools (Deng et al., *op. cit*., p 203-5; and Dzamko et al., *op. cit*., p 405-13). These studies also demonstrate the use of other cell lines, for example, compounds can be assessed for inhibition of endogenous LRRK2 in human-derived neuroblastoma SHSY5Y cells, mouse Swiss 3T3 cells, and human lymphoblastoid cells, where the latter are derived from a control individual, or from an individual with Parkinson's disease who is homozygous for the LRRK2 G2019S mutation (Deng et al., *op. cit*., p 203-5; and Dzamko et al., *op. cit*., p 405-13). It was also observed that overexpression of LRRK2, both wild-type and G2019S, in cortical cell cultures led to primary cortical neuron injury, as assessed by neurite shortening, and cell death, as assessed by DNA fragmentation (Lee et al., *op. cit*., p 998-1000). Thus LRRK2 inhibitors can be assessed for their ability to reduce neuron injury and cell death in such cortical cell cultures.

### Example C

### In vivo Activities

Pharmacokinetics of compounds as described herein (compounds of Formula I, e.g., compounds of the above Examples) can be assessed in animal models. For example, half-life and bioavailability can be assessed after intraperitoneal injection of compound into mice, and the phosphorylation of ser910 and ser935 can be assessed in tissues, such as kidney or brain tissue, where brain tissue can be used to assess whether compound can efficiently cross the blood-brain barrier (Deng et al., *op. cit*., p 204). In a herpes simplex virus (HSV) amplicon-based mouse model, it was shown that HSV amplicon mediated delivery of LRRK2 G2019S mutant induced a loss of tyrosine hydroxylase-positive neurons as compared to control mice expressing wild-type LRRK2. LRRK2 inhibitors can be assessed in this model for their ability to provide neuroprotection, i.e. attenuation of the loss of tyrosine hydroxylase-positive neurons (Lee et al., *op. cit*., p 999).

## Claims

1. A compound having a structure according to Formula I: 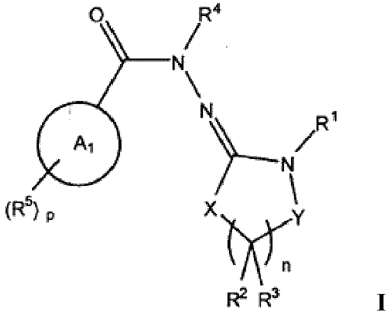 or a salt thereof,
wherein:
A₁ is selected from the group consisting of pyridine, pyrimidine, pyridazine, pyrazine, triazine, pyrrole, pyrazole, imidazole, triazole, tetrazole, oxazole, isoxazole, oxadiazole, thiazole, isothiazole, thiadiazole, furan, and thiophene;
X is -O-, -S-, -CR⁶R⁷-, or -NR⁸-;
Y is -C(O)- or -CH₂-;
R¹ is alkyl, cycloalkyl, heterocycloalkyl, aryl optionally substituted with one or more R⁹, or heteroaryl optionally substituted with one or more R¹⁰, wherein said alkyl is optionally substituted with one or more R¹¹; and wherein said cycloalkyl and said heterocycloalkyl are optionally substituted with one or more R¹²;
R² and R³ at each occurrence are independently selected from the group consisting of hydrogen, halogen, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₃₋₆ cycloalkyl, and 3-6 membered heterocycloalkyl; or
R² and R³ combined form =O or =NR¹⁵; or
one set of R² and R³ attached to the same carbon can combine with the carbon to which they are attached to form a carbocyclic ring or heterocyclic ring, wherein each ring is optionally substituted with one or more substituents selected from the group consisting of hydrogen, halogen, alkyl, -CN, -NO₂, =O, -OR¹³, -SR¹³, -NR¹⁴R¹⁵, =NR¹⁵, -C(O)R¹⁶, -S(O)ₘR¹⁶, cycloalkyl, and heterocycloalkyl, wherein cycloalkyl, and heterocycloalkyl are optionally substituted with one or more R¹² and wherein said alkyl is optionally substituted with one or more substituents selected from the group consisting of halogen, -CN, -NO₂, =O, -OR¹³, -SR¹³,-NR¹⁴R¹⁵, =NR¹⁵, -C(O)R¹⁶, -S(O)ₘR¹⁶, cycloalkyl, and heterocycloalkyl;
R⁴ is hydrogen or alkyl;
R⁵ at each occurrence is independently selected from the group consisting of halogen, C₁₋₆alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₁₋₆ alkylamino, C₁₋₆ dialkylamino, and N-linked-heterocycloalkyl
R⁶ and R⁷ are independently selected from the group consisting of hydrogen, halogen, alkyl, -CN, -NO₂, -OR¹³, -SR¹³, -NR¹⁴R¹⁵, -C(O)R¹⁶, -S(O)ₘR¹⁶, cycloalkyl, and heterocycloalkyl, Wherein said alkyl is optionally substituted with one or more substituents selected from the group consisting of halogen, -CN, -NO₂, =O, -OR¹³, -SR¹³,-NR¹⁴R¹⁵, =NR¹⁵, - C(O)R¹⁶, -S(O)ₘR¹⁶, cycloalkyl and heterocycloalkyl, and wherein said cycloalkyl and heterocycloalkyl are optionally substituted with one or more R¹¹;
R⁸ is selected from the group consisting of hydrogen and alkyl, wherein said alkyl is optionally substituted with one or more substituents selected from the group consisting of halogen, -CN, -NO₂, =O, -OR¹³, -SR¹³, -NR¹⁴R¹⁵, =NR¹⁵, -C(O)R¹⁶, -S(O)ₘR¹⁶, and N-linked heterocycloalkyl;
R⁹ and R¹⁰ at each occurrence are independently selected from the group consisting of halogen, alkyl, -CN, -NO₂, -OR¹⁷, -SR¹⁷, -NR¹⁸R¹⁹, -C(O)R²⁰, -S(O)ₘR²⁰, -C(O)OR¹⁷, - OC(O)R²⁰, -C(O)NR¹⁸R¹⁹, -NR¹⁸C(O)R²⁰, -S(O)₂NR¹⁸R¹⁹, -NR¹⁸S(O)₂R²⁰, cycloalkyl and heterocycloalkyl, wherein said alkyl is optionally substituted with one or more halogen, cycloalkyl, -CN, -NO₂, =O, -OR¹⁷, -SR¹⁷, -NR¹⁸R¹⁹, =NR¹⁹, -C(O)R²⁰, -S(O)ₘR²⁰, -C(O)OR¹⁷, - OC(O)R²⁰, -C(O)NR¹⁸R¹⁹, -NR¹⁸C(O)R²⁰, -S(O)₂NR¹⁸R¹⁹, -NR¹⁸S(O)₂R²⁰, cycloalkyl and heterocycloalkyl, and wherein said cycloalkyl and heterocycloalkyl are optionally substituted with one or more R¹²;
R¹¹ at each occurrence is independently selected from the group consisting of halogen, - CN, -NO₂, =O, -OR¹⁷, -SR¹⁷, -NR¹⁸R¹⁹, =NR¹⁹, -C(O)R²⁰, -S(O)mR²⁰, -C(O)OR¹⁷, -OC(O)R²⁰, - C(O)NR¹⁸R¹⁹, -NR¹⁸C(O)R²⁰, -S(O)₂NR¹⁸R¹⁹, -NR¹⁸S(O)₂R²⁰, cycloalkyl optionally substituted with one or more R¹², heterocycloalkyl optionally substituted with one or more R¹², aryl optionally substituted with one or more R⁹, or heteroaryl optionally substituted with one or more R¹⁰;
R¹² at each occurrence is independently selected from the group consisting of halogen, alkyl, haloalkyl, -OH, =O, alkoxy, haloalkoxy, -SH, =S, -S(O)_{q}- alkyl, -S(O)_{q}-haloalkyl, -NH₂, alkylamino, dialkylamino, and N-linked-heterocycloalkyl;
R¹³, R¹⁴, R¹⁵, R¹⁷, R¹⁸, and R¹⁹ at each occurrence are independently selected from the group consisting of hydrogen and alkyl wherein said alkyl is optionally substituted with one or more substituents selected from the group consisting of halogen, -OH, =O, alkoxy, haloalkoxy, - SH, =S, -S(O)_{q}-alkyl, -S(O)_{q}-haloalkyl, -NH₂, alkylamino,
dialkylamino, and N-linked-heterocycloalkyl;
R¹⁶ and R²⁰ at each occurrence are independently alkyl optionally substituted with one or more substituents selected from the group consisting of halogen, -OH, =O, alkoxy, haloalkoxy, - SH, =S, -S(O)_{q}-alkyl, -S(O)_{q}-haloalkyl, -NH₂, alkylamino, dialkylamino, and N-linked-heterocycloalkyl;
m is 1 or 2;
n is 1 or 2;
p is 1 or 2; and
q is 0, 1, or 2.

2. The compound according to claim 1 wherein
A₁ is selected from the group consisting of: 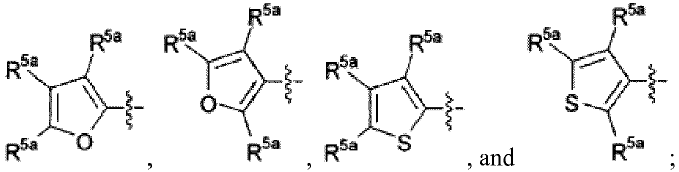 and
R^{5a} at each occurrence is independently selected from the group consisting of hydrogen, halogen, C₁₋₆alkyl, C₁₋₆haloalkyl, C₂₋₆ alkenyl, C₂₋₆haloalkenyl, C₂₋₆alkynyl, C₂₋₆haloalkynyl, C₁₋₆alkoxy, C₁₋₆haloalkoxy, C₁₋₆alkylthio, C₁₋₆haloalkylthio, C₁₋₆alkylsulfinyl, C₁₋₆haloalkylsulfinyl, C₁₋₆alkylsulfonyl, C₁₋₆haloalkylsulfonyl, C₁₋₆alkylamino, C₁₋₆dialkylamino, and N-linked-heterocycloalkyl.

3. The compound according to claim 2 wherein R¹ is -(CH₂)₀₋₁-phenyl, wherein phenyl is optionally substituted with 1 or 2 substituents independently selected from the group consisting of halogen, -CN, C₁₋₆alkyl, C₁₋₆haloalkyl, C₁₋₆alkoxy, C₁₋₆haloalkoxy, C₁₋₆alkylsulfonyl, C₁₋₆haloalkylsulfonyl, C₁₋₆alkylamino, C₁₋₆dialkylamino, and N-linked-heterocycloalkyl.

4. The compound according to any one of claims 1 to 3 wherein R² and R³ are independently selected from the group consisting of hydrogen, halogen, C₁₋₆alkyl, C₁₋₆haloalkyl, C₂₋₆alkenyl, C₂₋₆haloalkenyl, C₂₋₆alkynyl, C₂₋₆haloalkynyl, C₁₋₆alkoxy, C₁₋₆haloalkoxy, C₃₋₆cycloalkyl, and 3-6 membered heterocycloalkyl; or R² and R³ attached to the same carbon combine with the carbon to which they are attached to form C₃₋₆cycloalkyl or 3-6 membered heterocycloalkyl, and optionally wherein R² and R³ are independently selected from the group consisting of hydrogen, halogen, and C₁₋₆alkyl.

5. The compound according to claim 4 wherein
R⁴ is hydrogen;
n is 1;
X is S; and
Y is -C(O)-.

6. A compound having a structure according to Formula Ia: 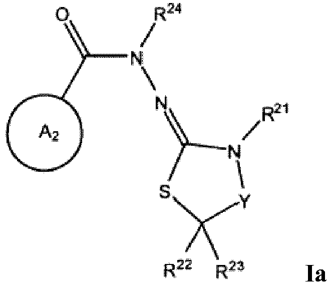 or a salt thereof,
wherein:
A₂ is selected from the group consisting of 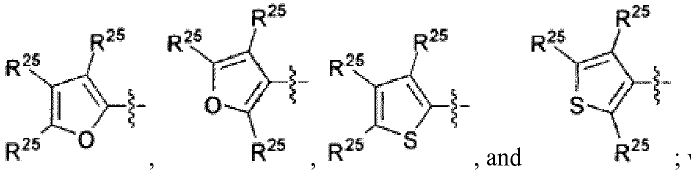 wherein 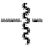 indicates the point of attachment of ring A₂ to the carbonyl carbon of the Formula Ia core structure;
Y is -C(O)- or -CH₂-;
R²¹ is -(CH₂)₀₋₁-phenyl, wherein phenyl is optionally substituted with 1 or 2 substituents independently selected from the group consisting of halogen, C₁₋₆alkyl, C₁₋₆haloalkyl, C₁₋₆alkoxy, and C₁₋₆haloalkoxy;
R²² and R²³ are independently hydrogen or C₁₋₃alkyl;
R²⁴ is hydrogen or C₁₋₃alkyl; and
one R²⁵ is selected from the group consisting of halogen, halogen, C₁₋₆alkyl, C₁₋₆haloalkyl, C₁₋₆alkoxy, and C₁₋₆haloalkoxy, and the other two R²⁵ are hydrogen.

7. The compound according to claim 6 wherein A₂ is

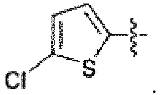

8. The compound according to claim 7 wherein R²¹ is -(CH₂)₀₋₁-phenyl, wherein phenyl is optionally substituted with 1 or 2 substituents independently selected from the group consisting of fluoro, methoxy, and trifluoromethyl; and R²⁴ is hydrogen, and optionally
wherein R²¹ is phenyl optionally substituted with 1 or 2 fluoro.

9. The compound according to claim 6 wherein Y is -C(O)-.

10. The compound according to claim 9 wherein A₂ is

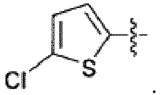

11. The compound according to claim 10 wherein R²¹ is -(CH₂)₀₋₁-phenyl, wherein phenyl is optionally substituted with 1 or 2 substituents independently selected from the group consisting of fluoro, methoxy, and tnfluoromethyl; and R⁴ is hydrogen, and optionally wherein R²¹ is phenyl optionally substituted with 1 or 2 fluoro.

12. The compound of claim 1 wherein the compound is selected from the group consisting of:
5-chloro-N'-(4-oxo-3-phenylthiazolidin-2-ylidene)thiophene-2-carbohydrazide,
5-chloro-N'-(3-(2,4-difluorophenyl)-4-oxothiazolidin-2-ylidene)thiophene-2-carbohydrazide,
5-chloro-N'-(3-(2-fluorophenyl)-4-oxothiazolidin-2-ylidene)thiophene-2-carbohydrazide,
5-chloro-N'-(3-(4-fluorophenyl)-4-oxothiazolidin-2-ylidene)thiophene-2-carbohydrazide,
5-chloro-N'-(3-(3-fluorophenyl)-4-oxothiazolidin-2-ylidene)thiophene-2-carbohydrazide,
5-chloro-N'-(5-methyl-4-oxo-3-phenylthiazolidin-2-ylidene)thiophene-2-carbohydrazide,
N'-(3-benzyl-4-oxothiazolidin-2-ylidene)-5-chlorothiophene-2-carbohydrazide,
5-chloro-N'-(3-phenylthiazolidin-2-ylidene)thiophene-2-carbohydrazide,
5-chloro-N'-(4-oxo-3-(3-(trifluoromethyl)phenyl)thiazolidin-2-ylidene)thiophene-2-carbohydrazide,
5-chloro-N'-(5-isopropyl-4-oxo-3-phenylthiazolidin-2-ylidene)thiophene-2-carbohydrazide,
5-chloro-N'-(5,5-dimethyl-4-oxo-3-phenylthiazolidin-2-ylidene)thiophene-2-carbohydrazide, and
5-chloro-N'-(3-(2-methoxyphenyl)-4-oxothiazolidin-2-ylidene)thiophene-2-carbohydrazide;
or a salt thereof.

13. A pharmaceutical composition comprising a compound according to any one of claims 1 to 12 and a pharmaceutically acceptable carrier.

14. A compound according to any one of claims 1 to 12 for use in a method of treating a neurodegenerative disease.

15. The compound for use in a method of treating a neurodegenerative disease according to claim 14, wherein:
(a) the disease is an alpha-synucleinopathy; or
(b) the disease is a member selected from the group consisting of Parkinson's disease, Parkinson disease with dementia, Parkinson's disease at risk syndrome, dementia with Lewy bodies, Lewy body variant of Alzheimer's disease, combined Parkinson's disease and Alzheimer's disease, multiple system atrophy, striatonigral degeneration, olivopontocerebellar atrophy, and Shy-Drager syndrome; or.
(c) the disease is Parkinson's disease.

## Patentansprüche

1. Verbindung mit einer Struktur der Formel I: 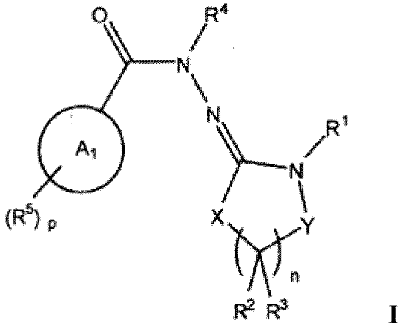 oder ein Salz davon,
worin:
A₁ ausgewählt ist aus der Gruppe bestehend aus Pyridin, Pyrimidin, Pyridazin, Pyrazin, Triazin, Pyrrol, Pyrazol, Imidazol, Triazol, Tetrazol, Oxazol, Isoxazol, Oxadiazol, Thiazol, Isothiazol, Thiadiazol, Furan und Thiophen;
X -O-, -S-, -CR⁶R⁷- oder -NR⁸- ist;
Y -C(O)- oder -CH₂- ist;
R¹ Alkyl, Cycloalkyl, Heterocycloalkyl, Aryl, gegebenenfalls mit einem oder mehreren R⁹ substituiert, oder Heteroaryl, gegebenenfalls mit einem oder mehreren R¹⁰ substituiert, ist, wobei das Alkyl gegebenenfalls mit einem oder mehreren R¹¹ substituiert ist; und wobei das Cycloalkyl und das Heterocycloalkyl gegebenenfalls mit einem oder mehreren R¹² substituiert sind;
R² und R³ bei jedem Auftreten unabhängig voneinander ausgewählt sind aus der Gruppe bestehend aus Wasserstoff, Halogen, C₁₋₆-Alkyl, C₁₋₆-Halogenalkyl, C₂₋₆-Alkenyl, C₂₋₆-Halogenalkenyl, C₂₋₆-Alkinyl, C₂₋₆-Halogenalkinyl, C₁₋₆-Alkoxy, C₁₋₆-Halogenalkoxy, C₃₋₆-Cycloalkyl und 3- bis 6-gliedrigem Heterocycloalkyl;
oder
R² und R³ zusammen =O oder =NR¹⁵ bilden; oder
ein Satz aus R² und R³, der an denselben Kohlenstoff gebunden ist, sich mit dem Kohlenstoff vereinigen kann, an den sie gebunden sind, um einen carbozyklischen Ring oder einen heterozyklischen Ring zu bilden, wobei jeder Ring gegebenenfalls mit einem oder mehreren Substituenten substituiert ist, die ausgewählt sind aus der Gruppe bestehend aus Wasserstoff, Halogen, Alkyl, -CN, -NO₂, =O, -OR¹³,-SR¹³, -NR¹⁴R¹⁵, =NR¹⁵, -C(O)R¹⁶, -S(O)ₘR¹⁶, Cycloalkyl und Heterocycloalkyl, wobei Cycloalkyl und Heterocycloalkyl gegebenenfalls mit einem oder mehreren R¹² substituiert sind und wobei das Alkyl gegebenenfalls mit einem oder mehreren Substituenten substituiert ist, die ausgewählt sind aus der Gruppe bestehend aus Halogen, - CN, -NO₂, =O, -OR¹³, -SR¹³, -NR¹⁴R¹⁵, =NR¹⁵, -C(O)R¹⁶, -S(O)ₘR¹⁶, Cycloalkyl und Heterocycloalkyl;
R⁴ Wasserstoff oder Alkyl ist;
R⁵ bei jedem Vorkommen unabhängig ausgewählt ist aus der Gruppe bestehend aus Halogen, C₁₋₆-Alkyl, C₁₋₆-Halogenalkyl, C₂₋₆-Alkenyl, C₂₋₆-Halogenalkenyl, C₂₋₆-Alkinyl, C₂₋₆-Halogenalkinyl, C₁₋₆-Alkoxy, C₁₋₆-Halogenalkoxy, C₁₋₆-Alkylthio, C₁₋₆-Halogenalkylthio, C₁₋₆-Alkylsulfinyl, C₁₋₆-Halogenalkylsulfinyl, C₁₋₆-Alkylsulfonyl, C₁₋₆-Halogenalkylsulfonyl, C₁₋₆-Alkylamino, C₁₋₆-Dialkylamino und N-gebundenem Heterocycloalkyl;
R⁶ und R⁷ unabhängig voneinander ausgewählt sind aus der Gruppe bestehend aus Wasserstoff, Halogen, Alkyl, -CN, -NO₂, -OR¹³, -SR¹³, -NR¹⁴R¹⁵, -C(O)R¹⁶,-S(O)ₘR¹⁶, Cycloalkyl und Heterocycloalkyl, wobei das Alkyl gegebenenfalls mit einem oder mehreren Substituenten substituiert ist, die ausgewählt sind aus der Gruppe bestehend aus Halogen, -CN, -NO₂, =O, -OR¹³, -SR¹³, -NR¹⁴R¹⁵, =NR¹⁵, - C(O)R¹⁶, -S(O)ₘR¹⁶, Cycloalkyl und Heterocycloalkyl, und wobei das Cycloalkyl und Heterocycloalkyl gegebenenfalls mit einem oder mehreren R¹¹ substituiert sind;
R⁸ ausgewählt ist aus der Gruppe bestehend aus Wasserstoff und Alkyl, wobei das Alkyl gegebenenfalls mit einem oder mehreren Substituenten substituiert ist, die ausgewählt sind aus der Gruppe bestehend aus Halogen, -CN, -NO₂, =O, -OR¹³, - SR¹³, -NR¹⁴R¹⁵, =NR¹⁵, -C(O)R¹⁶ und N-gebundenem Heterocycloalkyl;
R⁹ und R¹⁰ bei jedem Vorkommen unabhängig voneinander ausgewählt sind aus der Gruppe bestehend aus Halogen, Alkyl, -CN, -NO₂, -OR¹⁷, -SR¹⁷, -NR¹⁸R¹⁹, - C(O)R²⁰, -S(O)ₘR²⁰, -C(O)OR¹⁷, -OC(O)R²⁰, -C(O)NR¹⁸R¹⁹, -NR¹⁸C(O)R²⁰, - S(O)₂NR¹⁸R¹⁹, -NR¹⁸S(O)₂R²⁰, Cycloalkyl und Heterocycloalkyl, wobei das Alkyl gegebenenfalls mit einem oder mehreren aus Halogen, Cycloalkyl, -CN, -NO₂, =O, - OR¹⁷, -SR¹⁷, -NR¹⁸R¹⁹, =NR¹⁹, -C(O)R²⁰, -S(O)ₘR²⁰, -C(O)OR¹⁷, -OC(O)R²⁰, - C(O)NR¹⁸R¹⁹, -NR¹⁸C(O)R²⁰, -S(O)₂NR¹⁸R¹⁹, -NR¹⁸S(O)₂R²⁰, Cycloalkyl und Heterocycloalkyl substituiert ist und wobei das Cycloalkyl und Heterocycloalkyl gegebenenfalls mit einem oder mehreren R¹² substituiert sind;
R¹¹ bei jedem Vorkommen unabhängig ausgewählt ist aus der Gruppe bestehend aus Halogen, -CN, -NO₂, =O, -OR¹⁷, -SR¹⁷, -NR¹⁸R¹⁹, =NR¹⁹, -C(O)R²⁰, - S(O)ₘR²⁰, -C(O)OR¹⁷, -OC(O)R²⁰, -C(O)NR¹⁸R¹⁹, -NR¹⁸C(O)R²⁰, -S(O)₂NR¹⁸R¹⁹, - NR¹⁸S(O)₂R²⁰, Cycloalkyl, gegebenenfalls mit einem oder mehreren R¹² substituiert, Heterocycloalkyl, gegebenenfalls mit einem oder mehreren R¹² substituiert, Aryl, gegebenenfalls mit einem oder mehreren R⁹ substituiert, oder Heteroaryl, gegebenenfalls mit einem oder mehreren R¹⁰ substituiert;
R¹² bei jedem Vorkommen unabhängig ausgewählt ist aus der Gruppe bestehend aus Halogen, Alkyl, Halogenalkyl, -OH, =O, Alkoxy, Halogenalkoxy, -SH, =S, - S(O)_{q}-Alkyl, -S(O)_{q}-Halogenalkyl, -NH₂, Alkylamino, Dialkylamino und N-gebundenem Heterocycloalkyl;
R¹³, R¹⁴, R¹⁵, R¹⁷, R¹⁸ und R¹⁹ bei jedem Vorkommen unabhängig voneinander ausgewählt sind aus der Gruppe bestehend aus Wasserstoff und Alkyl, wobei das Alkyl gegebenenfalls mit einem oder mehreren Substituenten substituiert ist, die ausgewählt sind aus der Gruppe bestehend aus Halogen, -OH, =O, Alkoxy, Halogenalkoxy, -SH, =S, -S(O)_{q}-Alkyl, -S(O)_{q}-Halogenalkyl, -NH₂, Alkylamino, Dialkylamino und N-gebundenem Heterocycloalkyl;
R¹⁶ und R²⁰ bei jedem Vorkommen unabhängig Alkyl sind, gegebenenfalls substituiert mit einem oder mehreren Substituenten, die ausgewählt sind aus der Gruppe bestehend aus Halogen, -OH, =O, Alkoxy, Halogenalkoxy, -SH, =S, -S(O)_{q}-Alkyl,-S(O)_{q}-Halogenalkyl, -NH₂, Alkylamino, Dialkylamino und N-gebundenem Heterocycloalkyl;
m = 1 oder 2 ist;
n = 1 oder 2 ist;
p = 1 oder 2 ist; und
q = 0, 1 oder 2 ist.

2. Verbindung nach Anspruch 1, wobei
A₁ ausgewählt ist aus der Gruppe bestehend aus: 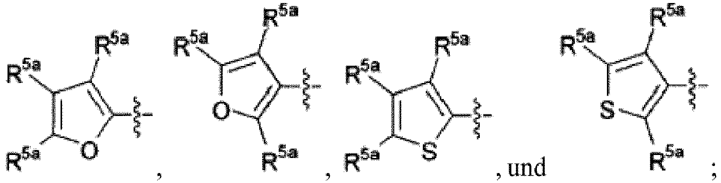 und
R^{5a} bei jedem Vorkommen unabhängig ausgewählt ist aus der Gruppe bestehend aus Wasserstoff, Halogen, C₁₋₆-Alkyl, C₁₋₆-Halogenalkyl, C₂₋₆-Alkenyl, C₂₋₆-Halogenalkenyl, C₂₋₆-Alkinyl, C₂₋₆-Halogenalkinyl, C₁₋₆-Alkoxy, C₁₋₆-Halogenalkoxy, C₁₋₆-Alkylthio, C₁₋₆-Halogenalkylthio, C₁₋₆-Alkylsulfinyl, C₁₋₆-Halogenalkylsulfinyl, C₁₋₆-Alkylsulfonyl, C₁₋₆-Halogenalkylsulfonyl, C₁₋₆-Alkylamino, C₁₋₆-Dialkylamino und N-gebundenem Heterocycloalkyl.

3. Verbindung nach Anspruch 2, wobei R¹ -(CH₂)₀₋₁-Phenyl ist, wobei Phenyl gegebenenfalls mit 1 oder 2 Substituenten substituiert ist, die unabhängig voneinander ausgewählt sind aus der Gruppe bestehend aus Halogen, -CN, C₁₋₆-Alkyl, C₁₋₆-Halogenalkyl, C₁₋₆-Alkoxy, C₁₋₆-Halogenalkoxy, C₁₋₆-Alkylsulfonyl, C₁₋₆-Halogenalkylsulfonyl, C₁₋₆-Alkylamino, C₁₋₆-Dialkylamino und N-gebundenem Heterocycloalkyl.

4. Verbindung nach einem der Ansprüche 1 bis 3, wobei R² und R³ unabhängig voneinander ausgewählt sind aus der Gruppe bestehend aus Wasserstoff, Halogen, C₁₋₆-Alkyl, C₁₋₆-Halogenalkyl, C₂₋₆-Alkenyl, C₂₋₆-Halogenalkenyl, C₂₋₆-Alkinyl, C₂₋₆-Halogenalkinyl, C₁₋₆-Alkoxy, C₁₋₆-Halogenalkoxy, C₃₋₆-Cycloalkyl und 3- bis 6-gliedrigem Heterocycloalkyl; oder R² und R³, die an denselben Kohlenstoff gebunden ist, sich mit dem Kohlenstoff vereinigen, an den sie gebunden sind, um ein C₃₋₆-Cycloalkyl oder 3- bis 6-gliedriges Heterocycloalkyl zu bilden, und wobei R² und R³ gegebenenfalls unabhängig voneinander ausgewählt sind aus der Gruppe bestehend aus Wasserstoff, Halogen und C₁₋₆-Alkyl.

5. Verbindung nach Anspruch 4, wobei
R⁴ Wasserstoff ist;
n 1 ist;
X S ist; und
Y -C(O)- ist.

6. Verbindung mit einer Struktur der Formel Ia: 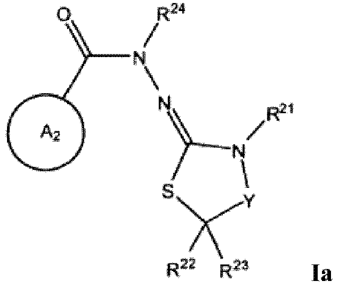 oder ein Salz davon,
worin:
A₂ ausgewählt ist aus der Gruppe bestehend aus 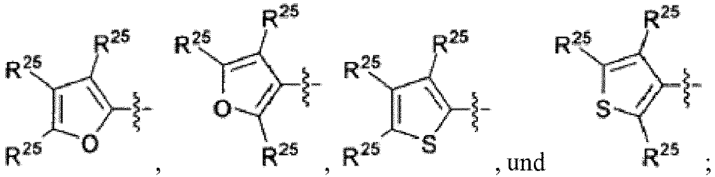 worin 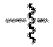 den Anbindungspunkt von Ring A₂ an den Carbonyl-Kohlenstoff der Kernstruktur der Formel Ia anzeigt;
Y -C(O)- oder -CH₂- ist;
R²¹ -(CH₂)₀₋₁-Phenyl ist, wobei Phenyl gegebenenfalls mit 1 oder 2 Substituenten substituiert ist, die unabhängig ausgewählt sind aus der Gruppe bestehend aus Halogen, C₁₋₆-Alkyl, C₁₋₆-Halogenalkyl, C₁₋₆-Alkoxy und C₁₋₆-Halogenalkoxy,
R²² und R²³ unabhängig voneinander Wasserstoff oder C₁₋₃-Alkyl sind;
R²⁴ Wasserstoff oder C₁₋₃-Alkyl ist; und
ein R²⁵ ausgewählt ist aus der Gruppe bestehend aus Halogen, Halogen, C₁₋₆-Alkyl, C₁₋₆-Halogenalkyl, C₁₋₆-Alkoxy und C₁₋₆-Halogenalkoxy, und die anderen beiden R²⁵ Wasserstoff sind.

7. Verbindung nach Anspruch 6, wobei A₂ 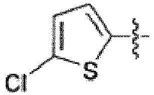 ist.

8. Verbindung nach Anspruch 7, worin R²¹ -(CH₂)₀₋₁-Phenyl ist, wobei Phenyl gegebenenfalls mit 1 oder 2 Substituenten substituiert ist, die unabhängig voneinander ausgewählt sind aus der Gruppe bestehend aus Fluor, Methoxy und Trifluormethyl; und R²⁴ Wasserstoff ist und wobei gegebenenfalls R²¹ Phenyl ist, das gegebenenfalls mit 1 oder 2 Fluor substituiert ist.

9. Verbindung nach Anspruch 6, wobei Y -C(O)- ist.

10. Verbindung nach Anspruch 9, wobei A₂ 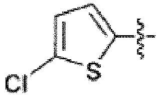 ist.

11. Verbindung nach Anspruch 10, wobei R²¹-(CH₂)₀₋₁-Phenyl ist, wobei Phenyl gegebenenfalls mit 1 oder 2 Substituenten substituiert ist, die unabhängig voneinander ausgewählt sind aus der Gruppe bestehend aus Fluor, Methoxy und Trifluormethyl; und R⁴ Wasserstoff ist und wobei gegebenenfalls R²¹ Phenyl ist, das gegebenenfalls mit 1 oder 2 Fluor substituiert ist.

12. Verbindung nach Anspruch 1, wobei die Verbindung ausgewählt ist aus der Gruppe bestehend aus:
5-Chlor-N'-(4-oxo-3-phenylthiazolidin-2-yliden)thiophen-2-carbohydrazid,
5-Chlor-N'-(3-(2,4-difluorphenyl)-4-oxothiazolidin-2-yliden)thiophen-2-carbohydrazid,
5-Chlor-N'-(3-(2-fluorphenyl)-4-oxothiazolidin-2-yliden)thiophen-2-carbohydrazid,
5-Chlor-N'-(3-(4-fluorphenyl)-4-oxothiazolidin-2-yliden)thiophen-2-carbohydrazid,
5-Chlor-N'-(3-(3-fluorphenyl)-4-oxothiazolidin-2-yliden)thiophen-2-carbohydrazid,
5-Chlor-N'-(5-methyl-4-oxo-3-phenylthiazolidin-2-yliden)thiophen-2-carbohydrazid,
N'-(3-Benzyl-4-oxothiazolidin-2-yliden)-5-Chlorthiophen-2-carbohydrazid,
5-Chlor-N'-(3-phenylthiazolidin-2-yliden)thiophen-2-carbohydrazid,
5-Chlor-N'-(4-oxo-3-(3-(trifluormethyl)phenyl)thiazolidin-2-yliden)thiophen-2-carbohy-drazid,
5-Chlor-N'-(5-isopropyl-4-oxo-3-phenylthiazolidin-2-yliden)thiophen-2-carbohydrazid,
5-Chlor-N'-(5,5-dimethyl-4-oxo-3-phenylthiazolidin-2-yliden)thiophen-2-carbohydrazid und
5-Ch lor-N'-(3-(2-methoxyphenyl)-4-oxothiazolidin-2-yliden)thiophen-2-carbohydrazid; oder einem Salz davon.

13. Pharmazeutische Zusammensetzung, umfassend eine Verbindung nach einem der Ansprüche 1 bis 12 und einen pharmazeutisch annehmbaren Träger.

14. Verbindung nach einem der Ansprüche 1 bis 12 zur Verwendung in einem Verfahren zur Behandlung einer neurodegenerativen Erkrankung.

15. Verbindung zur Verwendung in einem Verfahren zur Behandlung einer neurodegenerativen Erkrankung nach Anspruch 14, wobei:
(a) die Erkrankung eine α-Synukleinopathie ist; oder
(b) die Erkrankung eine aus der Gruppe bestehend aus Parkinson-Krankheit, Parkinson-Krankheit mit Demenz, Parkinson-Krankheit-Risiko-Syndrom, Demenz mit Lewy-Körpern, Lewy-Körper-Variante der Alzheimer-Krankheit, Kombination aus Parkinson-Krankheit und Alzheimer-Krankheit, Multisystematrophie, striatonigraler Degeneration, Olivo-ponto-cerebellärer Atrophie und Shy-Drager-Syndrom ausgewählte ist; oder
(c) die Erkrankung Parkinson-Krankheit ist.

## Revendications

1. Composé ayant une structure conforme à la formule I : 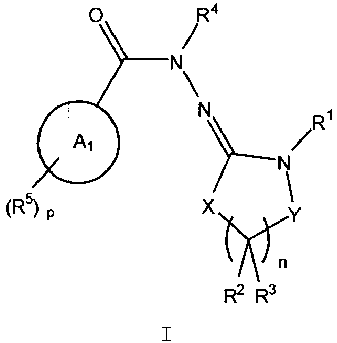 ou un sel de celui-ci,
formule dans laquelle :
A₁ est choisi dans l'ensemble constitué par la pyridine, la pyrimidine, la pyridazine, la pyrazine, la triazine, le pyrrole, le pyrazole, l'imidazole, le triazole, le tétrazole, l'oxazole, l'isoxazole, l'oxadiazole, le thiazole, l'isothiazole, le thiadiazole, le furane, et le thiophène ;
X est -0-, -S-, -CR⁶R⁷- ou -NR⁸- ;
Y est -C(O)- ou -CH₂- ;
R¹ est un alkyle, cycloalkyle, hétérocycloalkyle, aryle éventuellement substitué par un ou plusieurs R⁹, ou hétéroaryle éventuellement substitué par un ou plusieurs R¹⁰, où ledit alkyle est éventuellement substitué par un ou plusieurs R¹¹ ; et où ledit cycloalkyle et ledit hétérocycloalkyle sont éventuellement substitués par un ou plusieurs R¹² ;
R² et R³, à chaque occurrence, sont indépendamment choisis dans l'ensemble constitué par l'hydrogène, les halogènes, alkyle en C₁ à C₆, halogénoalkyle en C₁ à C₆, alcényle en C₂ à C₆, halogénoalcényle en C₂ à C₆, alcynyle en C₂ à C₆, halogénoalcynyle en C₂ à C₆, alcoxy en C₁ à C₆, halogénoalcoxy en C₁ à C₆, cycloalkyle en C₃ à C₆, et hétérocycloalkyle à 3 à 6 chaînons ; ou bien
R² et R³ combinés forment =0 ou =NR¹⁵ ; ou bien
un jeu de R² et R³ rattachés au même carbone peut se combiner au carbone auquel ils sont rattachés pour former un carbocycle ou un hétérocycle, où chaque cycle est éventuellement substitué par un ou plusieurs substituants choisis dans l'ensemble constitué par l'hydrogène, les halogènes, alkyle, -CN, -NO₂, =O, -OR¹³, -SR¹³, -NR¹⁴R¹⁵, =NR¹⁵, -C(O)R¹⁶, -S(O)ₘR¹⁶, cycloalkyle, et hétérocycloalkyle, où les cycloalkyle et hétérocycloalkyle sont éventuellement substitués par un ou plusieurs R¹² et où ledit alkyle est éventuellement substitué par un ou plusieurs substituants choisis dans l'ensemble constitué par les halogènes, -CN, -NO₂, =0, -OR¹³, -SR¹³, -NR¹⁴R¹⁵, =NR¹⁵, -C(O)R¹⁶, -S(O)R¹⁶, cycloalkyle, et hétérocycloalkyle ;
R⁴ est l'hydrogène ou un alkyle ;
R⁵, à chaque occurrence, est indépendamment choisi dans l'ensemble constitué par les halogènes, alkyle en C₁ à C₆, halogénoalkyle en C₁ à C₆, alcényle en C₂ à C₆, halogénoalcényle en C₂ à C₆, alcynyle en C₂ à C₆, halogénoalcynyle en C₂ à C₆, alcoxy en C₁ à C₆, halogénoalcoxy en C₁ à C₆, alkylthio en C₁ à C₆, halogénoalkylthio en C₁ à C₆, alkylsulfinyle en C₁ à C₆, halogénoalkylsulfinyle en C₁ à C₆, alkylsulfonyle en C₁ à C₆, halogénoalkylsulfonyle en C₁ à C₆, alkylamino en C₁ à C₆, dialkylamino en C₁ à C₆, et hétérocycloalkyle lié à N ;
R⁶ et R⁷ sont indépendamment choisis dans l'ensemble constitué par l'hydrogène, les halogènes, alkyle, -CN, -NO₂, -OR¹³, -SR¹³, -NR¹⁴R¹⁵, -C(O)R¹⁶, -S(O)ₘR¹⁶, cycloalkyle, et hétérocycloalkyle, où ledit alkyle est éventuellement substitué par un ou plusieurs substituants choisis dans l'ensemble constitué par les halogènes, -CN, -NO₂, =0, -OR¹³, -SR¹³, -NR¹⁴R¹⁵, =NR¹⁵, -C(O)R¹⁶, -S(O)ₘR¹⁶, cycloalkyle et hétérocycloalkyle, et où lesdits cycloalkyle et hétérocycloalkyle sont éventuellement substitués par un ou plusieurs R¹¹ ;
R⁸ est choisi dans l'ensemble constitué par l'hydrogène et alkyle, où ledit alkyle est éventuellement substitué par un ou plusieurs substituants choisis dans l'ensemble constitué par les halogènes, -CN, -NO₂, =0, -OR¹³, -SR¹³, -NR¹⁴R¹⁵, =NR¹⁵, -C(O)R¹⁶, et hétérocycloalkyle lié à N ;
R⁹ et R¹⁰, à chaque occurrence, sont indépendamment choisis dans l'ensemble constitué par les halogènes, alkyle, -CN, -NO₂, -OR¹⁷, -SR¹⁷, -NR¹⁸R¹⁹, -C(O)R²⁰, -S(O)ᵣR²⁰, -C(O)OR¹⁷, -OC(O)R²⁰, -C(O)NR¹⁸R¹⁹, -NR¹⁸C(O)R²⁰, -S(O)₂NR¹⁸R¹⁹, -NR¹⁸S(O)₂R²⁰, cycloalkyle et hétérocycloalkyle, où ledit alkyle est éventuellement substitué par un ou plusieurs halogènes, cycloalkyle, -CN, -NO₂, =0, -OR¹⁷, -SR¹⁷, -NR¹⁸R¹⁹, =NR¹⁹, -C(O)R²⁰, -S(O)ₘR²⁰, -C(O)OR¹⁷, -OC(O)R²⁰, -C(O)NR¹⁸R¹⁹, -NR¹⁸C(O)R²⁰, -S(O)₂NR¹⁸R¹⁹, -NR¹⁸S(O)₂R²⁰, cycloalkyle et hétérocycloalkyle, et où lesdits cycloalkyle et hétérocycloalkyle sont éventuellement substitués par un ou plusieurs R¹² ;
R¹¹, à chaque occurrence, est indépendamment choisi dans l'ensemble constitué par les halogènes, -CN, -NO₂, =0, -OR¹⁷, -SR¹⁷, -NR¹⁸R¹⁹, =NR¹⁹, -C(O)R²⁰, -S(O)ₘR²⁰, -C(O)OR¹⁷, -OC(O)R²⁰, -C(O)NR¹⁸R¹⁹, -NR¹⁸C(O)R²⁰, -S(O)₂NR¹⁸R¹⁹, -NR¹⁸S(O)₂R²⁰, cycloalkyle éventuellement substitué par un ou plusieurs R¹², hétérocycloalkyle éventuellement substitué par un ou plusieurs R¹², aryle éventuellement substitué par un ou plusieurs R⁹, et hétéroaryle éventuellement substitué par un ou plusieurs R¹⁰ ;
R¹², à chaque occurrence, est indépendamment choisi dans l'ensemble constitué par les halogènes, alkyle, halogénoalkyle, -OH, =0, alcoxy, halogénoalcoxy, -SH, =S, -S(O)_{q}-alkyle, -S(O)_{q}-halogénoalkyle, -NH₂, alkylamino, dialkylamino, et hétérocycloalkyle lié à N ;
R¹³, R¹⁴, R¹⁵, R¹⁷, R¹⁸ et R¹⁹, à chaque occurrence, sont indépendamment choisis dans l'ensemble constitué par l'hydrogène et alkyle, où ledit alkyle est éventuellement substitué par un ou plusieurs substituants choisis dans l'ensemble constitué par les halogènes, -OH, =0, alcoxy, halogénoalcoxy, -SH, =S, -S(O)_{q}-alkyle, -S(O)_{q}-halogénoalkyle, -NH₂, alkylamino, dialkylamino, et hétérocycloalkyle lié à N ;
R¹⁶ et R²⁰, à chaque occurrence, sont indépendamment un alkyle éventuellement substitué par un ou plusieurs substituants choisis dans l'ensemble constitué par les halogènes, -OH, =0, alcoxy, halogénoalcoxy, -SH, =S, -S(O)_{q}-alkyle, -S(O)_{q}-halogénoalkyle, -NH₂, alkylamino, dialkylamino, et hétérocycloalkyle lié à N ;
m vaut 1 ou 2 ;
n vaut 1 ou 2 ;
p vaut 1 ou 2 ; et
q vaut 0, 1 ou 2.

2. Composé selon la revendication 1, dans lequel
A₁ est choisi dans l'ensemble constitué par : 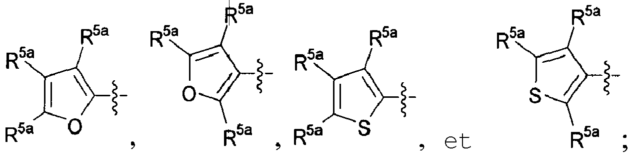 R^{5a}, à chaque occurrence, est indépendamment choisi dans l'ensemble constitué par l'hydrogène, les halogènes, alkyle en C₁ à C₆, halogénoalkyle en C₁ à C₆, alcényle en C₂ à C₆, halogénoalcényle en C₂ à C₆, alcynyle en C₂ à C₆, halogénoalcynyle en C₂ à C₆, alcoxy en C₁ à C₆, halogénoalcoxy en C₁ à C₆, alkylthio en C₁ à C₆, halogénoalkylthio en C₁ à C₆, alkylsulfinyle en C₁ à C₆, halogénoalkylsulfinyle en C₁ à C₆, alkylsulfonyle en C₁ à C₆, halogénoalkylsulfonyle en C₁ à C₆, alkylamino en C₁ à C₆, dialkylamino en C₁ à C₆, et hétérocycloalkyle lié à N.

3. Composé selon la revendication 2, dans lequel R¹ est -(CH₂)₀₋₁-phényle, où le phényle est éventuellement substitué par 1 ou 2 substituants indépendamment choisis dans l'ensemble constitué par les halogènes, -CN, alkyle en C₁ à C₆, halogénoalkyle en C₁ à C₆, alcoxy en C₁ à C₆, halogénoalcoxy en C₁ à C₆, alkylsulfonyle en C₁ à C₆, halogénoalkylsulfonyle en C₁ à C₆, alkylamino en C₁ à C₆, dialkylamino en C₁ à C₆, et hétérocycloalkyle lié à N.

4. Composé selon l'une quelconque des revendications 1 à 3, dans lequel R² et R³ sont indépendamment choisis dans l'ensemble constitué par l'hydrogène, les halogènes, alkyle en C₁ à C₆, halogénoalkyle en C₁ à C₆, alcényle en C₂ à C₆, halogénoalcényle en C₂ à C₆, alcynyle en C₂ à C₆, halogénoalcynyle en C₂ à C₆, alcoxy en C₁ à C₆, halogénoalcoxy en C₁ à C₆, cycloalkyle en C₃ à C₆, et hétérocycloalkyle à 3 à 6 chaînons ; ou bien R² et R³ rattachés au même carbone se combinent avec le carbone auquel ils sont rattachés pour former un cycloalkyle en C₃ à C₆ ou un hétérocycloalkyle à 3 à 6 chaînons, et éventuellement dans lequel R² et R³ sont indépendamment choisis dans l'ensemble constitué par l'hydrogène, les halogènes, et alkyle en C₁ à C₆.

5. Composé selon la revendication 4, dans lequel
R⁴ est l'hydrogène ;
n vaut 1 ;
X est S ; et
Y est -C(O)-.

6. Composé ayant une structure conforme à la formule Ia : 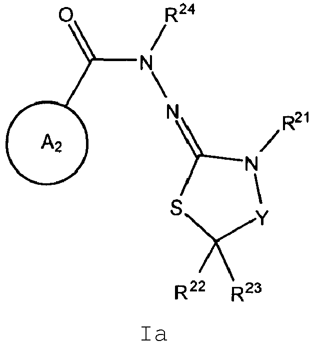 ou un sel de celui-ci,
formule dans laquelle :
A₂ est choisi dans l'ensemble constitué par 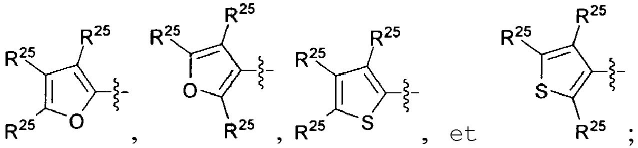
où 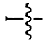 indique le point de rattachement du cycle A₂ au carbone de carbonyle de la structure de coeur de la formule Ia ;
Y est -C(O)- ou -CH₂- ;
R²¹ est un -(CH₂)₀₋₁-phényle, où le phényle est éventuellement substitué par 1 ou 2 substituants indépendamment choisis dans l'ensemble constitué par les halogènes, alkyle en C₁ à C₆, halogénoalkyle en C₁ à C₆, alcoxy en C₁ à C₆, et halogénoalcoxy en C₁ à C₆ ;
R²² et R²³ sont indépendamment l'hydrogène ou un alkyle en C₁ à C₃ ;
R²⁴ est l'hydrogène ou un alkyle en C₁ à C₃ ; et
un R²⁵ est choisi dans l'ensemble constitué par les halogènes, les halogènes, alkyle en C₁ à C₆, halogénoalkyle en C₁ à C₆, alcoxy en C₁ à C₆, et halogénoalcoxy en C₁ à C₆, et les deux autres R²⁵ sont des hydrogènes.

7. Composé selon la revendication 6, dans lequel A₂ est

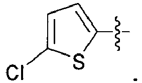

8. Composé selon la revendication 7, dans lequel R²¹ est un -(CH₂)₀₋₁-phényle, où le phényle est éventuellement substitué par 1 ou 2 substituants indépendamment choisis dans l'ensemble constitué par fluoro, méthoxy, et trifluorométhyle ; et R²⁴ est l'hydrogène, et éventuellement dans lequel R²¹ est un phényle éventuellement substitué par 1 ou 2 fluoro.

9. Composé selon la revendication 6, dans lequel Y est -C(O)-.

10. Composé selon la revendication 9, dans lequel A₂ est

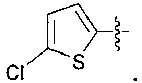

11. Composé selon la revendication 10, dans lequel R²¹ est un -(CH₂)₀₋₁-phényle, où le phényle est éventuellement substitué par 1 ou 2 substituants indépendamment choisis dans l'ensemble constitué par fluoro, méthoxy, et trifluorométhyle ; et R⁴ est l'hydrogène, et éventuellement dans lequel R²¹ est un phényle éventuellement substitué par 1 ou 2 fluoro.

12. Composé selon la revendication 1, lequel composé est choisi dans l'ensemble constitué par les suivants :
5-chloro-N'-(4-oxo-3-phénylthiazolidin-2-ylidène)thiophène-2-carbohydrazide,
5-chloro-N'-(3-(2,4-difluorophényl)-4-oxothiazolidin-2-ylidène)thiophène-2-carbohydrazide,
5-chloro-N'-(3-(2-fluorophényl)-4-oxothiazolidin-2-ylidène)thiophène-2-carbohydrazide,
5-chloro-N'-(3-(4-fluorophényl)-4-oxothiazolidin-2-ylidène)thiophène-2-carbohydrazide,
5-chloro-N'-(3-(3-fluorophényl)-4-oxothiazolidin-2-ylidène)thiophène-2-carbohydrazide,
5-chloro-N'-(5-méthyl-4-oxo-3-phénylthiazolidin-2-ylidène)thiophène-2-carbohydrazide,
N'-(3-benzyl-4-oxothiazolidin-2-ylidène)-5-chlorothiophène-2-carbohydrazide,
5-chloro-N'-(3-phénylthiazolidin-2-ylidène)thiophène-2-carbohydrazide,
5-chloro-N'-(4-oxo-3-(3-(trifluorométhyl)phényl)thiazolidin-2-ylidène)thiophène-2-carbohydrazide,
5-chloro-N'-(5-isopropyl-4-oxo-3-phénylthiazolidin-2-ylidène)thiophène-2-carbohydrazide,
5-chloro-N'-(5,5-diméthyl-4-oxo-3-phénylthiazolidin-2-ylidène)thiophène-2-carbohydrazide, et
5-chloro-N'-(3-(2-méthoxyphényl)-4-oxothiazolidin-2-ylidène)thiophène-2-carbohydrazide ;
ou un sel d'un tel composé.

13. Composition pharmaceutique comprenant un composé selon l'une quelconque des revendications 1 à 12 et un véhicule pharmaceutiquement acceptable.

14. Composé selon l'une quelconque des revendications 1 à 12, pour une utilisation dans une méthode de traitement d'une maladie neurodégénérative.

15. Composé pour utilisation dans une méthode de traitement d'une maladie neurodégénérative selon la revendication 14, dans lequel :
(a) la maladie est une alpha-synucléinopathie ; ou
(b) la maladie est un membre choisi dans l'ensemble constitué par la maladie de Parkinson, la maladie de Parkinson avec démence, le syndrome de risque associé à la maladie de Parkinson, la démence à corps de Lewy, la variante à corps de Lewy de la maladie d'Alzheimer, la maladie de Parkinson combinée à la maladie d'Alzheimer, l'atrophie multisystémique, la dégénérescence striatonigrique, l'atrophie olivo-ponto-cérébelleuse, et le syndrome de Shy-Drager ; ou
(c) la maladie est la maladie de Parkinson.
